# EUROPEAN PATENT APPLICATION

(11) **EP 4 851 154 A2**
(43) Date of publication of application: **09.12.2026**
(21) Application number: 26205273.1
(22) Date of filing: 06.02.2019
(51) Int. Cl.: C07D 405/14

(54) **FAP INHIBITOR**

(30) Priority: 06.02.2018 EP 18155420; 06.02.2018 EP 18155419; 10.10.2018 EP 18199641
(62) Document number(s) of the earlier application(s) in accordance with Art. 76 EPC: 19703101.6 / 3 749 663
(71) Applicant: Universität Heidelberg, 69117 Heidelberg (DE)
(72) Inventor: HABERKORN, Uwe, 68723 Schwetzingen (DE); LOKTEV, Anastasia, 69124 Heidelberg (DE); LINDNER, Thomas, 68723 Schwetzingen (DE); MIER, Walter, 64625 Bensheim (DE); GIESEL, Frederik, 69115 Heidelberg (DE); KRATOCHWIL, Clemens, 69493 Hirschberg a.d.B. (DE)
(74) Representative: Zwicker, Jörk

(57) **Abstract**

The present invention relates to a compound, a pharmaceutical composition comprising or consisting of said compound, a kit comprising or consisting of said compound or pharmaceutical composition and use of the compound or pharmaceutical composition in the diagnosis or treatment of a disease characterized by overexpression of fibroblast activation protein (FAP).

## Description

The present invention relates to a compound, a pharmaceutical composition comprising or consisting of said compound, a kit comprising or consisting of said compound or pharmaceutical composition and use of the compound or pharmaceutical composition in the diagnosis or treatment of a disease characterized by overexpression of fibroblast activation protein (FAP).

### Background of the Invention

Tumor growth and spread are not only determined by the cancer cells, but also by the non-malignant constituents of the malignant lesion, which are subsumed under the term stroma. The stroma may represent over 90% of the tumor mass in tumors with desmoplastic reaction such as breast, colon and pancreatic carcinoma. Especially a subpopulation of fibroblasts called cancer-associated fibroblasts (CAFs) is known to be involved in tumor growth, migration and progression. Therefore, these cells represent an attractive target for diagnosis and anti-tumor therapy.

A distinguishing feature of CAFs is the expression of seprase or fibroblast activation protein α (FAP-α), a type II membrane bound glycoprotein belonging to the dipeptidyl peptidase 4 (DPP4) family. FAP-α has both dipeptidyl peptidase and endopeptidase activity. The endopeptidase activity distinguishes FAP-α from the other members of the DPP4 family. Identified substrates for the endopeptidase activity so far are denatured Type I collagen, α1-antitrypsin and several neuropeptides. FAP-α has a role in normal developmental processes during embryogenesis and in tissue modelling. It is not or only at insignificant levels expressed on adult normal tissues. However, high expression occurs in wound healing, arthritis, artherosclerotic plaques, fibrosis and in more than 90% of epithelial carcinomas.

The appearance of FAP-α in CAFs in many epithelial tumors and the fact that overexpression is associated with a worse prognosis in cancer patients led to the hypothesis that FAP-α activity is involved in cancer development as well as in cancer cell migration and spread. Therefore, the targeting of this enzyme for imaging and endoradiotherapy can be considered as a promising strategy for the detection and treatment of malignant tumors. The present inventors developed a small molecule based on a FAP-α specific inhibitor and were able to show specific uptake, rapid internalization and successful imaging of tumors in animal models as well as in tumor patients. A comparison with the commonly used radiotracer ¹⁸F-fluorodeoxyglucose (¹⁸F-FDG) revealed a clear superiority of the new FAP-α ligand in patients with locally advanced lung adenocarcinoma. Thus, the present invention provides inter alia: (i) detection of smaller primary tumors and, thus the possibility of earlier diagnosis, (ii) the detection of smaller metastasis and, thus a better assessment of tumor stage, (iii) precise intra-operative guidance facilitating complete surgical removal of tumor tissue, (iv) better differentiation between inflammation and tumor tissue, (v) more precise staging of patients with tumors, (vi) better follow up of tumor lesions after antitumor therapy, (vii) the opportunity to use the molecules as theranostic agents for diagnosis and therapy. Furthermore, the molecules can be used for the diagnosis and treatment of non-malignant diseases such as chronic inflammation, atherosclerosis, fibrosis, tissue remodeling and keloid disorders.

### Summary of the Invention

In a first aspect, the present invention provides a compound of Formula (I) 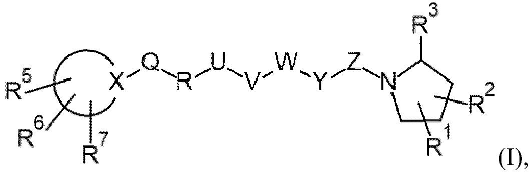 wherein
Q, R, U, V, W, Y, Z are individually present or absent under the proviso that at least three of Q, R, U, V, W, Y, Z are present;
Q, R, U, V, W, Y, Z are independently selected form the group consisting of O, CH₂, NR⁴, C=O, C=S, C=NR⁴, HCR⁴ and R⁴CR⁴, with the proviso that two Os are not directly adjacent to each other;
R¹ and R² are independently selected from the group consisting of -H, -OH, halo, C₁₋₆-alkyl, - O-C₁₋₆-alkyl, S-C₁₋₆-alkyl;
R³ is selected from the group consisting of -H , -CN , -B(OH)₂, -C(O) -alkyl, -C(O) -aryl-, - C=C-C(O) -aryl, -C=C-S(O)₂-aryl, -CO₂H , -SO₃H , -SO₂NH₂,-PO₃H₂, and 5-tetrazolyl;
R⁴ is selected from the group consisting of -H, -C₁₋₆-alkyl, -O-C₁₋₆-alkyl, -S-C₁₋₆-alkyl, aryl, and -C₁₋₆-aralkyl, each of said -C₁₋₆-alkyl being optionally substituted with from 1 to 3 substituents selected from -OH, oxo, halo and optionally connected to Q, R, U, V, W, Y or Z; R⁵ is selected from the group consisting of -H, halo and C₁₋₆-alkyl;
R⁶, and R⁷ are independently selected from the group consisting of -H, 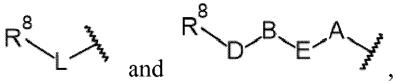 under the proviso that R⁶ and R⁷ are not at the same time H,
wherein L is a linker,
wherein D, A, E, and B are individually present or absent, preferably wherein at least A, E, and B are present, wherein when present:
   D is a linker;
   A is selected from the group consisting of NR⁴, O, S, and CH₂;
   E is selected from the group consisting of
      C₁₋₆-alkyl, 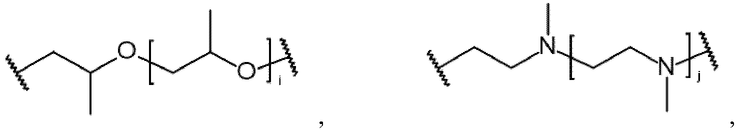 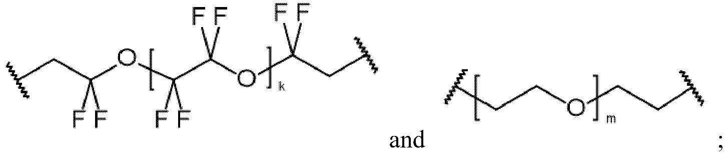
   wherein i is 1, 2, or 3;
   wherein j is 1, 2, or 3;
   wherein k is 1, 2, or 3;
   wherein m is 1, 2, or 3;
   A and E together form a group selected from a cycloalkyl, heterocycloalkyl, aryl and heteroaryl, wherein A and E can be mono-, bi- and multicyclic, preferably monocyclic. Each A and E being optionally substituted by 1 to 4 residues from the group consisting of -H, -C₁₋₆-alkyl, -O-C₁₋₆-alkyl, -S-C₁₋₆-alkyl, alkenyl, heteroalkenyl, cycloalkenyl, cycloheteroalkenyl, alkynyl, aryl, and -C₁₋₆-aralkyl, each of said -C₁₋₆-alkyl being optionally substituted with from 1 to 3 substituents selected from -OH, oxo, halo; and optionally connected to A, B, D, E or 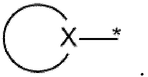
   B is selected from the group consisting of S, NR⁴, NR⁴-O, NR⁴-C₁₋₆-alkyl, NR⁴-C₁₋₆-alkyl-NR⁴, and a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein NR⁴-C₁₋₆-alkyl-NR⁴ and the N-containing heterocycle is substituted with 1 to 3 substituents selected from the group consisting of C₁₋₆-alkyl, aryl, C₁₋₆-aralkyl; and;
   R⁸ is selected from the group consisting of radioactive moiety, chelating agent, fluorescent dye, a contrast agent and combinations thereof; 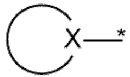
   is a 1-naphtyl moiety or a 5 to 10- membered N-containing aromatic or non-aromatic mono- or bicyclic heterocycle, wherein there are 2 ring atoms between the N atom and X; said heterocycle optionally further comprising 1, 2 or 3 heteroatoms selected from O, N and S; and X is a C atom;
   or a pharmaceutically acceptable tautomer, racemate, hydrate, solvate, or salt thereof.

In a second aspect, the present invention relates to a pharmaceutical composition comprising or consisting of at least one compound of the first aspect, and, optionally, a pharmaceutically acceptable carrier and/or excipient.

In a third aspect, the present invention relates to the compound of the first aspect or the pharmaceutical composition of the second aspect for use in the diagnosis or treatment of a disease characterized by overexpression of fibroblast activation protein (FAP) in an animal or a human subject.

In a fourth aspect, the present invention relates to a kit comprising or consisting of the compound of the first aspect or the pharmaceutical composition of the second aspect and instructions for the diagnosis or treatment of a disease.

### List of Figures

In the following, the content of the figures comprised in this specification is described. In this context please also refer to the detailed description of the invention above and/or below.

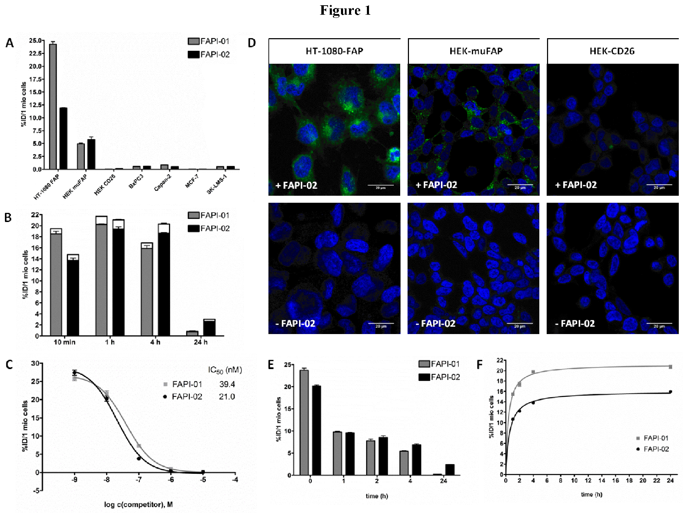

**Figure 1****: In vitro characterization of ¹²⁵I-FAPI-01 and ¹⁷⁷Lu-FAPI-02.** A. Binding of radiolabeled FAPI-01 and FAPI-02 to different human cancer cell lines as well as cell lines transfected with human FAP-α (HT-1080-FAP), murine FAP-α (HEK-muFAP) and human CD26 (HEK-CD26) after 60 min of incubation. **B.** Internalization of radiolabeled FAPI-01 and FAPI-02 into HT-1080-FAP cells after incubation for 10 min to 24 h. The internalized proportion is shown in grey and black, respectively; the extracellularly bound fraction is indicated by the white bars. **C.** Competitive binding of radiolabeled FAPI-01 and FAPI-02 to HT-1080-FAP cells after adding increasing concentrations of unlabeled FAPI-01 and Lu-FAPI-02. **D.** Internalization of FAPI-02 into FAP-α positive and negative cell lines. Blue: DAPI; green: FAPI-02-Atto488. **E+F.** Efflux kinetics of FAPI-01 and FAPI-02 after 1 h incubation of HT-1080-FAP cells with radiolabeled compounds followed by incubation with compound-free medium for 1 to 24 h. All values are given as percentage of total applied dose normalized to 1 million cells (%ID/1 mio cells).

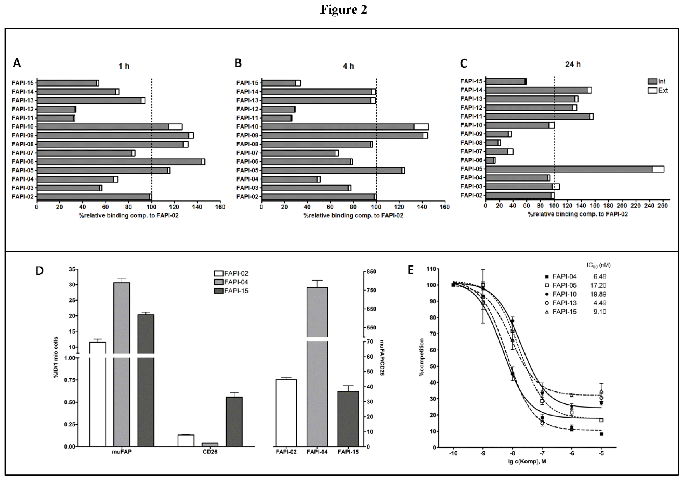

**Figure 2****: Binding specificity and relative internalization rates of FAPI derivatives. A-C.** Binding and internalization rates of FAPI-03 to FAPI-15 in relation to FAPI-02 (defined as 100%). Internalization rates after 1, 4 and 24 hrs of incubation are depicted in grey; the extracellular bound fraction is represented by the white bars. **D.** Binding of selected FAPI derivatives to HEK cells expressing murine FAP-α and human CD26 after 60 min of incubation. *Right side:* Ratio of muFAP to CD26 binding. **E.** Competitive binding of selected FAPI derivatives to HT-1080-FAP cells after adding increasing concentrations of unlabeled compound.

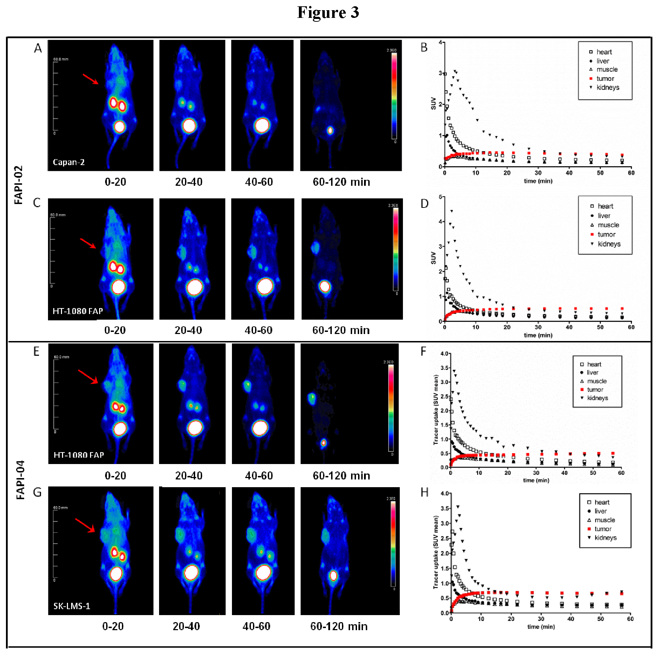

**Figure 3****: Imaging of FAPI-02 and -04 in mice bearing human FAP-positive (HT-1080-FAP) and negative (Capan-2, SK-LMS-1) tumor xenografts. A+C, E+G.** Small animal PET imaging was performed after intravenous administration of 4 nmol ⁶⁸Ga-FAPI-02 and -04 (10 MBq resp.) at indicated times. The radiotracer gets rapidly enriched within the tumor (indicated by the red arrow) while not accumulating in non-cancerous tissue. Furthermore, a rapid elimination via the kidneys and bladder is seen. **B+D, F+H.** Quantification of the PET images demonstrates a solid clearance of ⁶⁸Ga-FAPI-02 and -04 from the cardiovascular system and a constant uptake into the tumor.

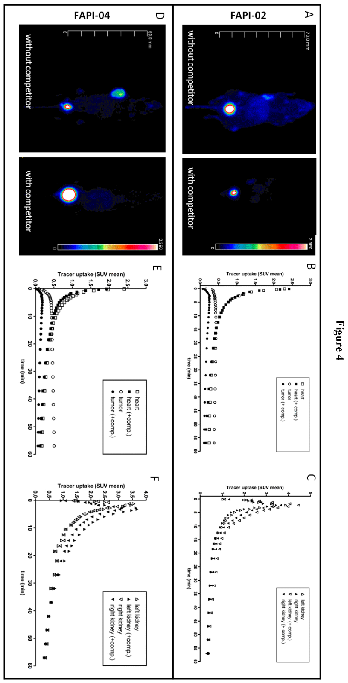

**Figure 4****: Blocking experiments for analysis of binding specificity in vivo A+D.** Blocking of ⁶⁸Ga-FAPI-02 and -04 tumor accumulation by co-administration of 30 nmol unlabeled compound in HT-1080-FAP tumor bearing mice. **B+C, E+F.** Time-activity curves of ⁶⁸Ga-FAPI-02 and -04 in selected organs after intravenous administration with and without unlabeled compound as a competitor.

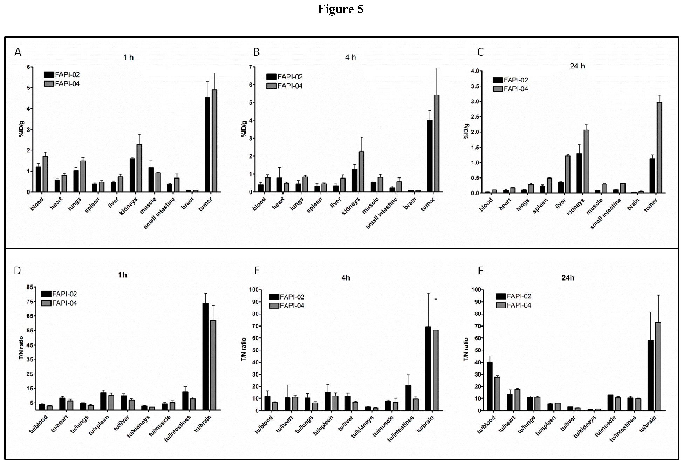

**Figure 5****: Organ distribution of ¹⁷⁷Lu-FAPI-02 and -04 in HT-1080-FAP tumor bearing nude mice**
   **A-C.** Biodistribution of ¹⁷⁷Lu-FAPI-02 and -04 was measured ex vivo at indicated times after intravenous administration of 1 MBq to mice bearing human FAP-positive HT-1080 tumor xenografts; n=3 for each time point. The values stated are expressed as percentage of injected dose per gram of tissue (% ID/g). The radiotracers are shown to accumulate within the FAP-expressing tumor, showing the highest enrichment after 1 h for FAPI-02 (4.5 %ID/g) and 2 h for FAPI-04 (5.4 %ID/g). **D-F.** Tumor-to-normal tissue ratios of ¹⁷⁷Lu-FAPI-02 and -04 1, 4 and 24 hrs after intravenous administration.

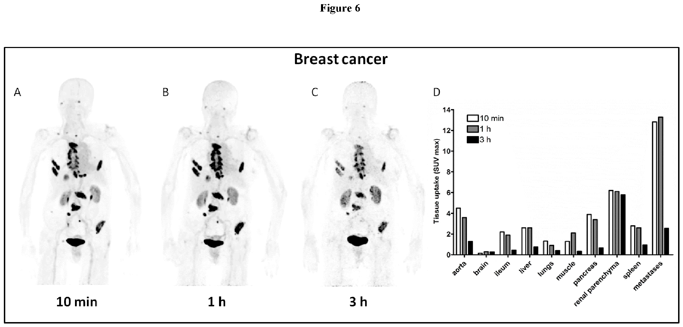

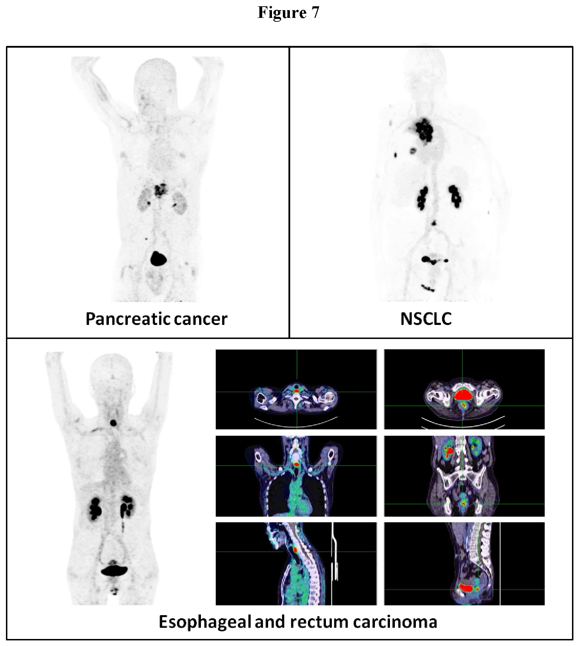

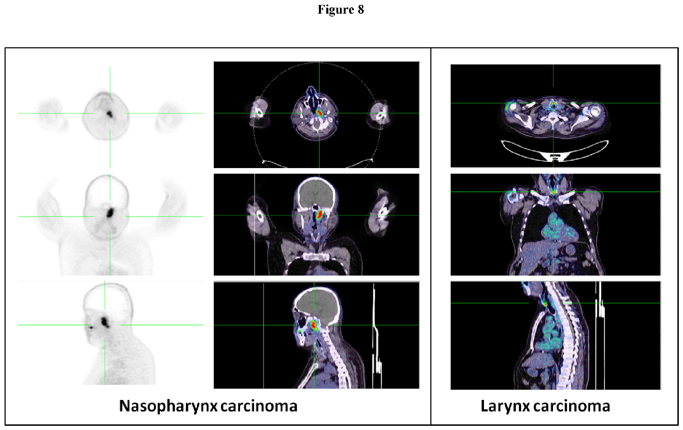

**Figures 6-9****: PET/CT imaging of FAPI-02 in cancer patients**
   **6A-C.** Maximum intensity projections (MIP) of PET/CT scans in a patient suffering from metastasized breast cancer. **D.** Maximum tissue uptake of ⁶⁸Ga-FAPI-02 10 min, 1 h and 3 h after intravenous administration to a patient with metastasized breast cancer.
   **7.** MIP of PET/CT scans in patients with pancreatic cancer, non-small cell lung cancer (NSCLC) and esophageal and rectum carcinoma 1 h after administration of ⁶⁸Ga-FAPI-02. **8.** MIP of PET/CT scans in patients with nasopharynx and larynx carcinoma 1 h after administration of ⁶⁸Ga-FAPI-02.
   **9A+B.** Whole-body PET/CT imaging (MIP) 1 h after administration of ¹⁸F-FDG and ⁶⁸Ga-FAPI-02 to a patient with locally advanced lung adenocarcinoma. **C+D.** Transaxial view of lung adenocarcinoma patient 1 h after administration of ¹⁸F-FDG and ⁶⁸Ga-FAPI-02. FAPI-02 is selectively accumulated in FAP-α expressing tissue and shows significantly higher uptake in the malignant lesions compared to ¹⁸F-FDG.

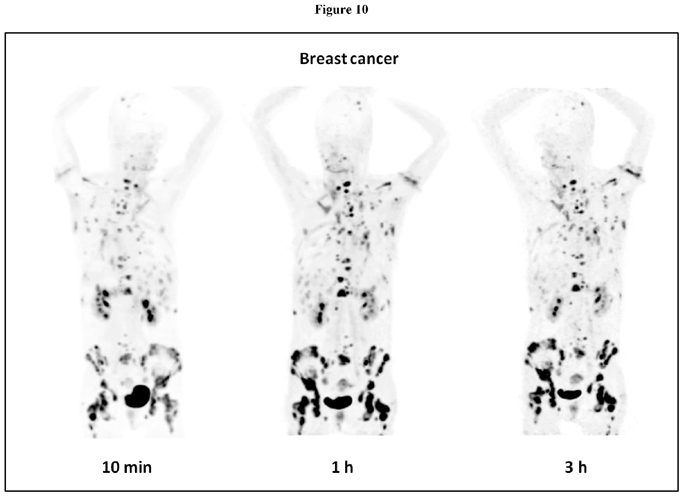

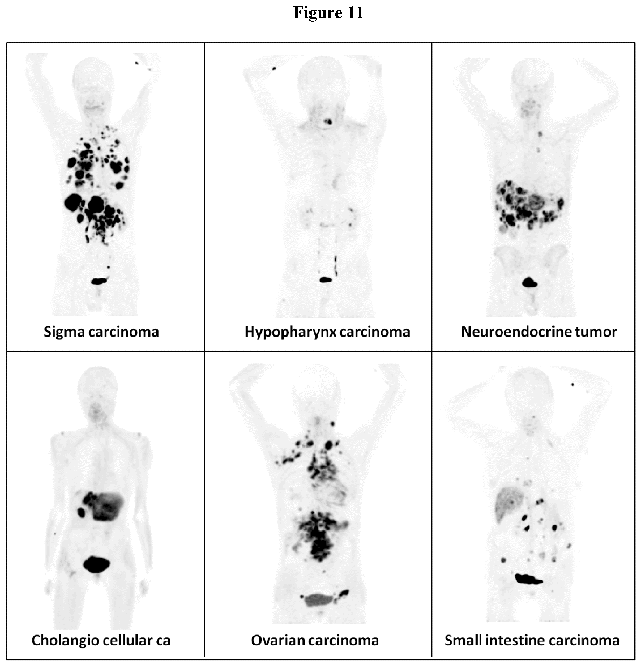

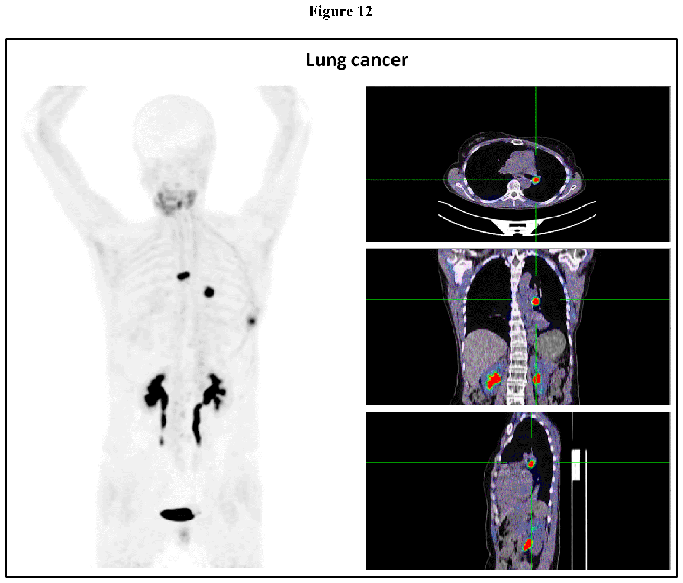

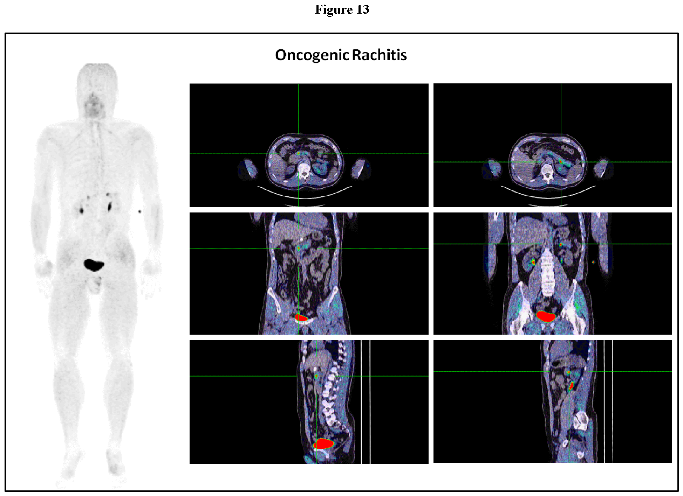

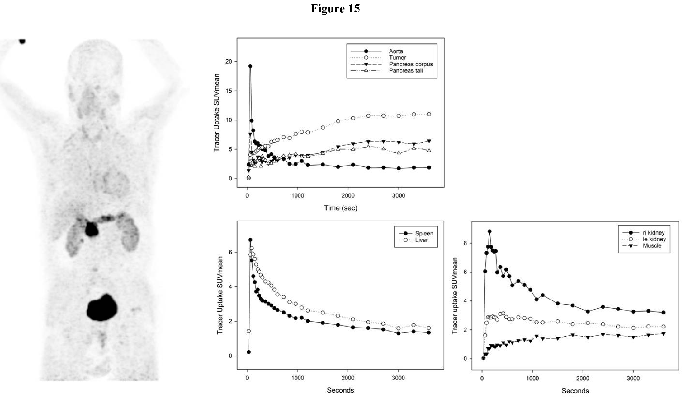

**Figures 10-16****: PET/CT imaging of FAPI-04 in cancer patients**
   **10** Maximum intensity projections (MIP) of PET/CT scans in a patient suffering from metastasized breast cancer 10 min, 1 and 3 hrs after administration of ⁶⁸Ga-FAPI-04.
   **11** MIP of PET/CT scans in patients with sigma carcinoma, hypopharynx carcinoma, neuroendocrine tumors, cholangio, ovarial and small intestine carcinoma 1 h after administration of ⁶⁸Ga-FAPI-04.
   **12** MIP of PET/CT scans in a patient with lung cancer 1 h after administration of ⁶⁸Ga-FAPI-04.
   **13** MIP of PET/CT scans in a patient with oncogenic rachitis 1 h after administration of ⁶⁸Ga-FAPI-04.
   **14** Comparative imaging of one patient with metastasized prostate cancer. MIP of PET/CT scans 1 h after application of radiolabeled DOTATOC, PSMA and FAPI-04.
   **15** Maximum intensity projection (MIP) and time-activity curves of a dynamic ⁶⁸Ga-FAPI-04 PET/CT scan in a pancreatic cancer patient.
   16 Relative binding rates of Lu-177 labeled FAPI derivatives compared to FAPI-04 (set to 100%) after incubation for 1, 4 and 24 h on FAP-expressing HT-1080 cells; n=3.
**Figure 17****:** Competitive binding of selected FAPI derivatives to HT-1080-FAP cells after adding increasing concentrations of unlabeled compound (10⁻¹⁰ to 10⁻⁵ M, incubation for 60 min, n=3).
**Figure 18****:** Binding of FAPI derivatives to HEK cells expressing murine FAP and human CD26 after 60 min of incubation, n=3. Values are expressed as percentage of applied dose (%ID) per 1 mio cells.
**Figure 19****:** Biodistribution of selected FAPI derivatives in HT-1080-FAP xenotransplants 1, 4 and 24 h after intravenous administration of the radiotracers, n=3. Values are expressed as percentage of injected dose per gram of tissue (%ID/g).
**Figure 20****:** Tumor-to-blood ratio of selected FAPI derivatives in HT-1080-FAP xenotransplants 1, 4 and 24 h after intravenous administration of the radiotracers, n=3.
**Figure 21****:** PET imaging of Ga-68 labeled FAPI-21 and FAPI-46 in HT-1080-FAP tumor bearing mice; n=1.
**Figure 22****:** Maximum standardized uptake values (SUV) of selected FAPI derivatives in HT-1080-FAP tumor bearing mice; n=1.
**Figure 23****:** Maximum (SUV max, Fig. 23 A) and mean (SUV mean, Fig. 23 B) standardized uptake values of Ga-68 labeled FAPI-02 and FAPI-04 in cancer patients; n=25.
**Figure 24****:** Intra-individual comparison of 6 patients with 6 different tumor entities undergoing FDG-PET and FAPI-PET imaging within <9 days.
**Figure 25****:** PET/CT imaging of Ga-68 labeled FAPI-04 in patients with peritonitis carcinomatosa (A), myocarditis (B) and hip joint arthrosis (C) 1 h p.i.
**Figure 26****:** PET/CT imaging of Ga-68 labeled FAPI-21 in cancer patients 1 h p.i.
**Figure 27****:** PET/CT imaging of Ga-68 labeled FAPI-46 1 h p.i. and intratherapeutical imaging of Sm-153 labeled FAPI-46 30 min p.i. in cancer patients.
**Figure 28****:** Intratherapeutical imaging of Sm-153 labeled FAPI-46 up to 20 h p.i.

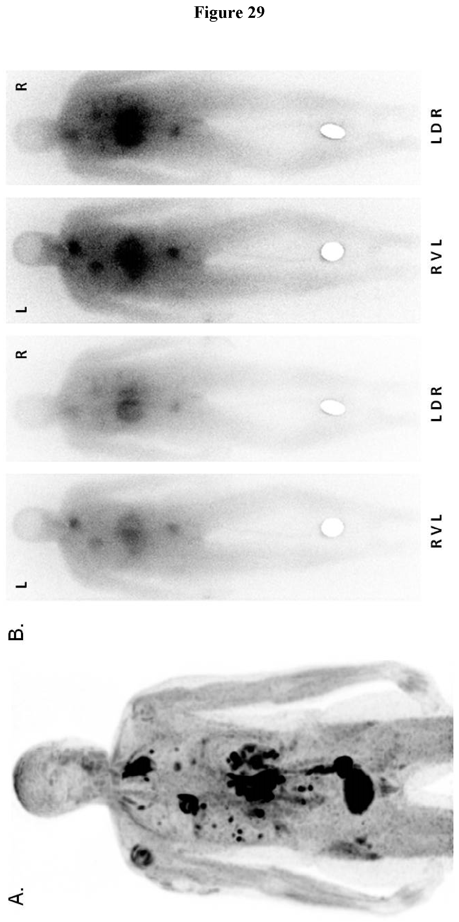

**Figure 29****:** A. Maximum intensity projection (MIP) 1 h after intravenous administration of ⁶⁸Ga-FAPI-46 to a patient with metastasized colorectal carcinoma. B. Imaging of Bremsstrahlung 2 h after therapeutic treatment with ⁹⁰Y-FAPI-46 of the same patient.
**Figure 30****:** PET/CT imaging of Ga-68 labeled FAPI-46 1 h p.i. in lung cancer patients with idiopathic lung fibrosis. A, B. Maximum tracer uptake into tumor tissue is significantly higher than into non-exacerbated fibrotic lesions. C. Maximum tracer uptake into tumor tissue is slightly lower than into exacerbated fibrotic tissue.
**Figure 31****:** A. Binding of Tc-99m labeled FAPI-19 to HT-1080-FAP cells, n=3. B. Competitive binding of Tc-99m labeled FAPI-19 to HT-1080-FAP cells after adding increasing concentrations of unlabeled compound (10⁻¹⁰ to 10⁻⁵ M, incubation for 60 min, n=3). C. Scintigraphy of Tc-99m labeled FAPI-19 in HT-1080-FAP xenotransplants, n=1.
**Figure 32****:** A. Binding of Tc-99m labeled FAPI-34 to HT-1080-FAP cells, n=3. B. Scintigraphy of Tc-99m labeled FAPI-34 in HT-1080-FAP xenotransplants, n=1.
**Figure 33****:** Scintigraphy of Tc-99m labeled FAPI-34 in one patient with metastasized pancreas carcinoma.
**Figure 34****:** A. Binding of Pb-203 labeled FAPI derivatives to HT-1080-FAP cells, n=3. B. Efflux kinetics of Pb-203 labeled FAPI derivatives after incubation of HT-1080-FAP cells with radiolabeled compound for 60 min and consequent incubation with nonradioactive medium for 1 to 24 hours, n=3. C. Competitive binding of Pb-203 labeled FAPIs to HT-1080-FAP cells after adding increasing concentrations of unlabeled compound (10⁻¹⁰ to 10⁻⁵ M, incubation for 60 min, n=3).
**Figure 35****:** Scintigraphy of Pb-203 labeled FAPI-04 and FAPI-46 in HT-1080-FAP xenotransplants, n=1.
**Figure 36****:** Biodistribution of Pb-203 labeled FAPI-04 and FAPI-46 in HT-1080-FAP xenotransplants 1, 4, 6 and 24 h after intravenous administration of the radiotracers, n=3. Values are expressed as percentage of injected dose per gram of tissue (%ID/g).
**Figure 37****:** A. Binding of Cu-64 labeled FAPI-42 and FAPI-52 to HT-1080-FAP cells, n=3. B. Competitive binding of Cu-64 labeled FAPI-42 and FAPI-52 to HT-1080-FAP cells after adding increasing concentrations of unlabeled compound (10⁻¹⁰ to 10⁻⁵ M, incubation for 60 min, n=3). C. Efflux kinetics of Cu-64 labeled FAPI-42 and FAPI-52 after incubation of HT-1080-FAP cells with radiolabeled compound for 60 min and consequent incubation with nonradioactive medium for 1 to 24 hours, n=3.
**Figure 38****:** PET imaging of Cu-64 labeled FAPI-42 and FAPI-52 in HT-1080-FAP tumor bearing mice; n=1.
**Figure 39****:** PET imaging of AlF-18 labeled FAPI-42 and FAPI-52 in HT-1080-FAP tumor bearing mice; n=1.
**Figure 40****:** a. Small animal PET imaging of 68Ga-labeled FAPI-02 in U87MG tumor bearing nude mice up to 140 min after intravenous administration of the radiotracer. The tumor is indicated by the red arrow. b. Biodistribution of 177Lu-labeled FAPI-02 and FAPI-04 in U87MG tumor bearing nude mice 1, 4 and 24 h after intravenous administration of the radiotracers; n=3.
**Figure 41****:** Tumor-to-organ ratios of 177Lu-labeled FAPI-02 and -04 in U87MG tumor bearing mice 1, 4 and 24 h after intravenous administration.
**Figure 42****:** Maximum intensity projection (MIP) of PET/CT scans in a glioblastoma patient 10 min, 1 and 3 h after administration of 68Ga-FAPI-02.
**Figure 43****:** Exemplary images (contrast enhanced T1 weighted MRI, FAPI-PET and fused images of both modalities) of IDH wt glioblastomas, IDH-mutant gliomas WHO grade II and IDH-mutant glioblastomas.
**Figure 44****:** Absolute SUVmax values of all 18 gliomas.

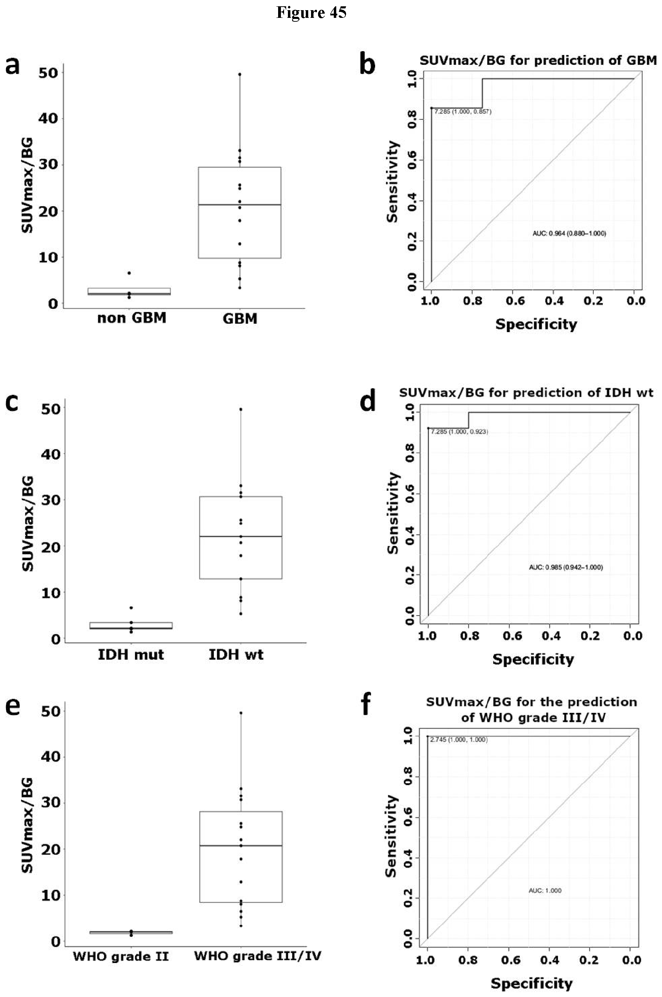

**Figure 45****:** Statistical Analysis of SUVmax/BG values. Boxplots of SUVmax/BG values and corresponding ROC curves in GBM versus non-GBM (a, b), IDH-mutant versus IDH wildtype gliomas (c, d) and gliomas grade II versus gliomas grade III/IV (e, f).
**Figure 46****:** Dose-dependent inhibition of enzymatic FAP activity by FAPI-04 and Talabostat. In contrast to Talabostat, a potent DPP4 inhibitor with marginal FAP activity, FAPI-04 demonstrates robust, dose-dependent FAP inhibition.

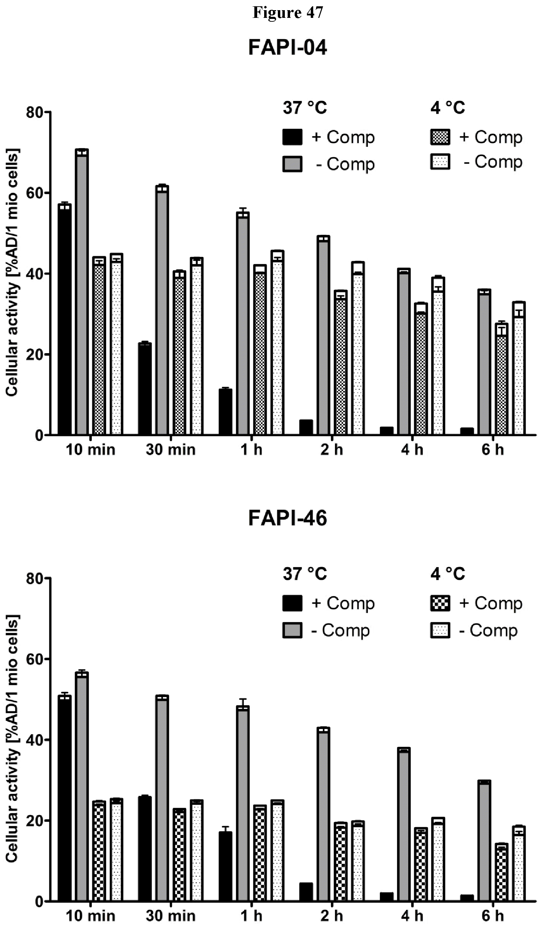

**Figure 47****:** Reuptake of ¹⁷⁷Lu-labeled FAPI-04 and FAPI-46 in HT-1080-FAP cells. Following incubation of the cells with the radiotracers for 60 min at 37°C, the compounds are removed and non-radioactive medium with (+ Comp.) and without unlabeled compound (-Comp.) added and incubated for 10 min to 6 h. Already within the first ten minutes of incubation, renewed uptake of the unlabeled FAPI derivatives occurs, displacing parts of the radiolabeled fraction, which results in significantly lower radioactivity values as compared to pure medium without competitor. After 6 h of incubation, almost complete displacement of the radiolabeled FAPIs has occurred. These findings indicate a continuous reuptake of intact FAP molecules back to the cell membrane upon initial internalization, allowing renewed binding and internalization of FAP ligands.
**Figure 48****:** Organ distribution of ¹⁷⁷Lu-labeled FAPI-04 after single and multiple injection in HT-1080-FAP tumor bearing nude mice. Administration of two equal doses of ¹⁷⁷Lu-FAPI-04 at intervals of 4 h results in increased overall organ activities, including the tumor, measured 8 and 24 h after the first injection. In contrast, administration of three doses (higher initial dose, lower subsequent doses) reveals no change in the overall organ activities.

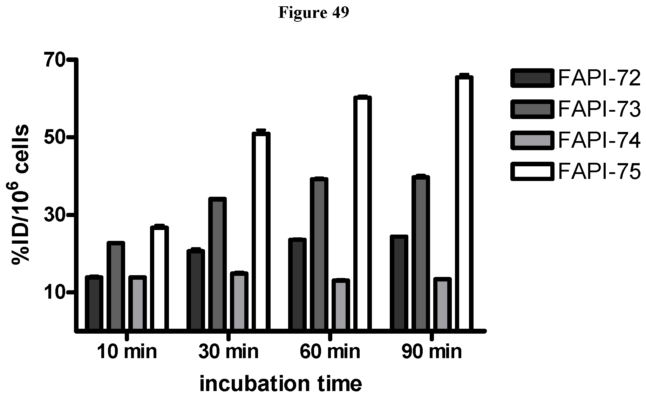

**Figure 49****:** Binding of F-18-FAPI derivatives to HT1080 cells expressing human FAP after 10, 30, 60 and 90 min of incubation, n=3. Values are expressed as percentage of applied dose (%ID) per 1 mio cells.
**Figure 50****:** PET imaging of AlF-18 labeled FAPI-74 and FAPI-52 in HT-1080-FAP tumor bearing mice; n=1.
**Figure 51****:** Biodistribution of FAPI-75 in HT-1080-FAP xenotransplants 1, 4 and 24 h after intravenous administration of the radiotracer, n=3. Values are expressed as percentage of injected dose per gram of tissue (%ID/g).
**Figure 52****:** PET imaging of patient with non-small cell lung cancer: Robust accumulation of F18-labeled FAPI-74 in multiple metastases
**Figure 53****:** Time activity curves of the heart region (SUVmean) for FAPI-04 and -46 as illustration of the fast blood pool clearance.

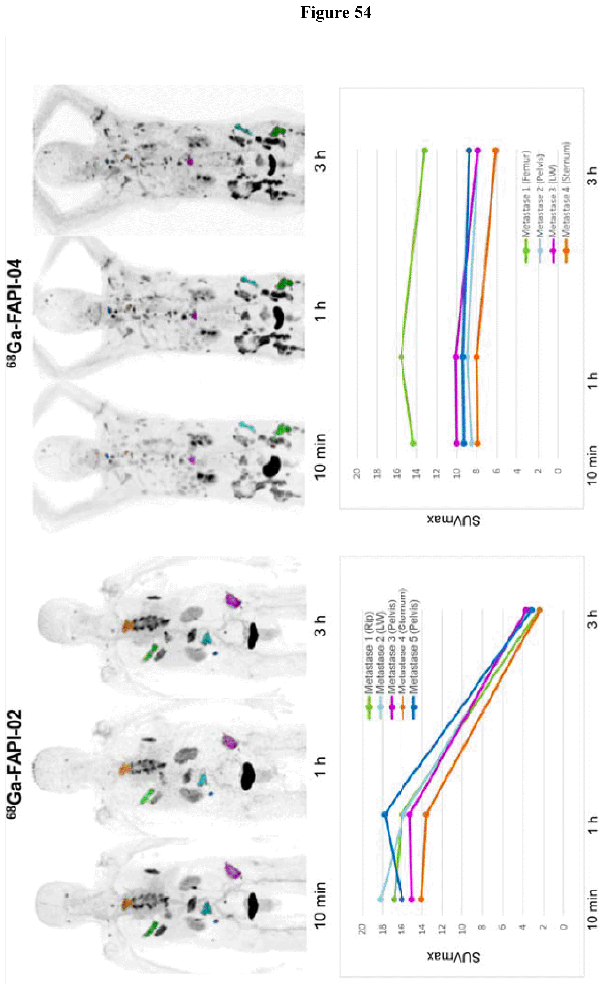

**Figure 54****:** FAPI-02 and FAPI-04 at the different imaging time-point (10 min, 1h and 3h p.i.) in two patients with metastasized breast cancer. Rapid tumor targeting and fast blood clearance is followed by a long plateau phase without relevant change in image contrast (top). In comparison to FAPI-02 the ligand FAPI-04 is characterized by a prolonged tumor retention time (bottom).

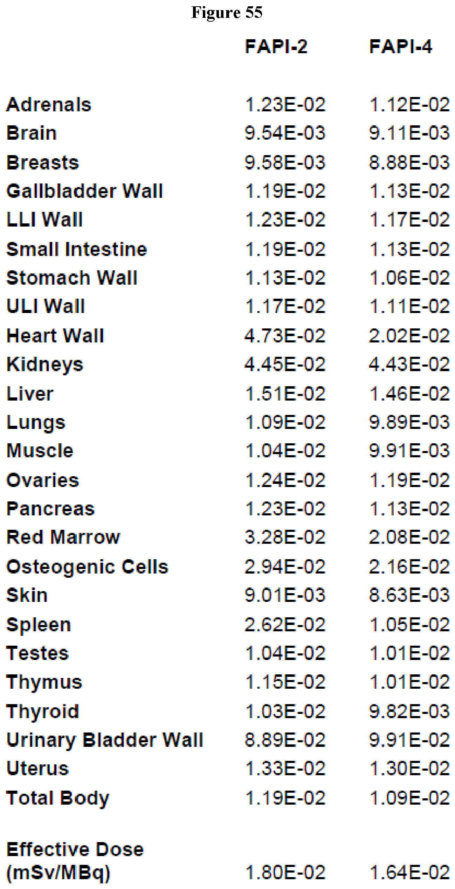

Figure 55: The effective dose of FAPI-02 was 1.80E-02 mSv/MBq calculated with OLINDA (1.82E-02 with IDAC1 / ICRP60, 1.79E-02 with IDAC2 / ICRP103). The effective dose for FAPI-04 PET/CT was 1.64E-02 mSv/MBq calculated with OLINDA (1.66E-02 with IDAC1 / ICRP60, 1.35E-02 with IDAC2 / ICRP103). If the delayed scan at 3h p.i. is omitted in clinical practice, the routine activity for an FAPI-exam could be reduced to 200 MBq ⁶⁸Ga; consecutively the radiation dose of such a FAPI-PET/CT scan would be 3-4 mSv.

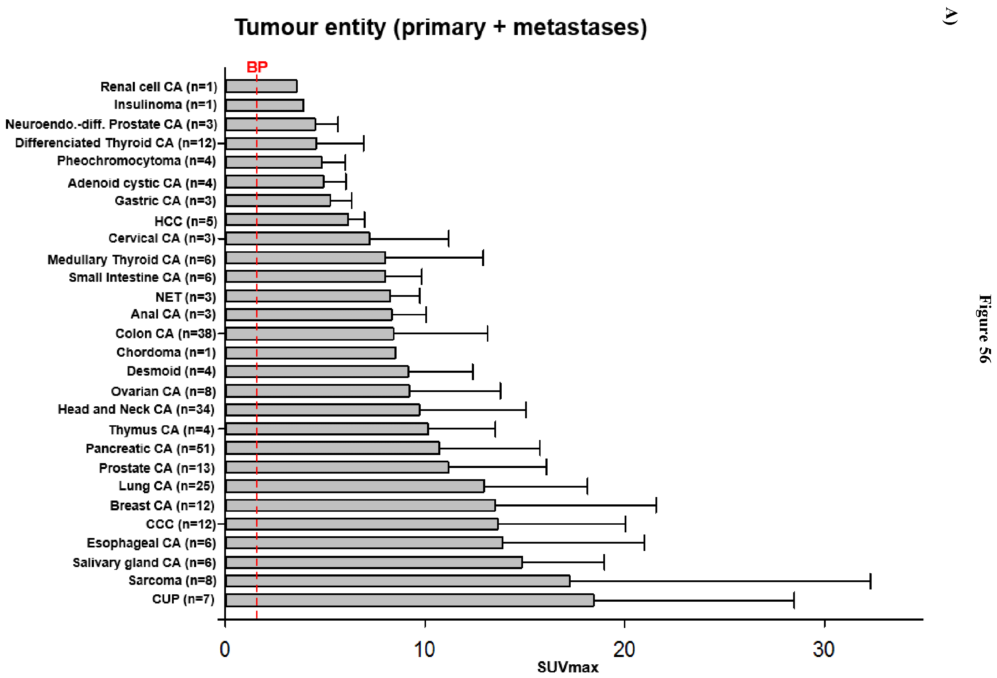

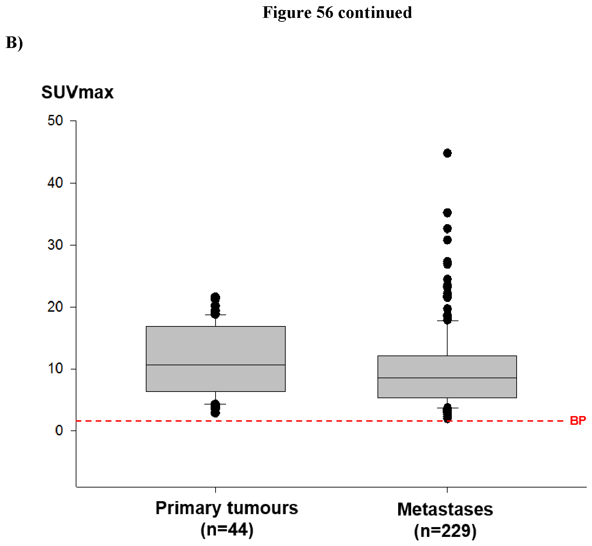

Figure 56: A) ⁶⁸Ga-FAPI-04 after 1h post injection in different tumor entities in PET/CT. The highest average SUVmax (>12) were found in sarcoma, esophageal, breast, cholangiocellular carcinoma and lung cancer. The lowest FAPI uptake (average SUVmax <6) was observed in renal cell, differentiated thyroid, adenoid-cystic, gastric carcinoma and pheochromocytoma. The average SUVmax of hepatocellular carcinoma, colorectal carcinoma, head-neck-cancer, ovarial carcinoma, pancreatic carcinoma was intermediate (SUV 6<x<12). Within all tumor entities a high inter-individual variation was observed. Due to low background activity (SUV 2), the tumor-to-background ratios are > 2-fold in the intermediate and > 4-fold in the high intensity uptake group. B) Primary tumour entities presented similar SUV-uptake compared tumour entities using FAPI-04

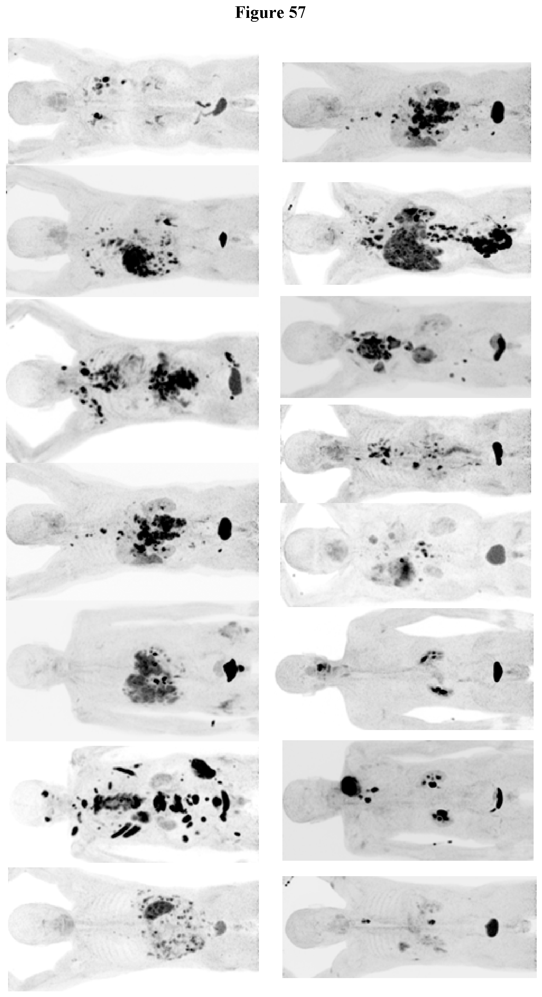

**Figure 57****:** Exemplary PET images of different tumor entities that have been used for the quantifications shown in figure 56 A-B.

### Detailed Descriptions of the Invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland).

Throughout this specification and the claims, which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages, different aspects of the invention are defined in more detail. Each aspect so defined may be combined with any other aspect or aspects unless clearly indicated to the contrary. In particular, any feature indicated as being optional, preferred or advantageous may be combined with any other feature or features indicated as being optional, preferred or advantageous.

Several documents are cited throughout the text of this specification. Each of the documents cited herein (including all patents, patent applications, scientific publications, manufacturer's specifications, instructions etc.), whether supra or infra, is hereby incorporated by reference in its entirety. Nothing herein is to be construed as an admission that the invention is not entitled to antedate such disclosure by virtue of prior invention. Some of the documents cited herein are characterized as being *"incorporated by reference".* In the event of a conflict between the definitions or teachings of such incorporated references and definitions or teachings recited in the present specification, the text of the present specification takes precedence.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

In the following, some definitions of terms frequently used in this specification are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

In the following definitions of the terms: alkyl, heteroalkyl, cycloalkyl, heterocycloalkyl, aryl, aralkyl, heteroaryl, heteroaralkyl, alkenyl and alkynyl are provided. These terms will in each instance of its use in the remainder of the specification have the respectively defined meaning and preferred meanings.

The term "alkyl" refers to a saturated straight or branched carbon chain. Preferably, the chain comprises from 1 to 10 carbon atoms, i.e. 1, 2, 3, 4, 5, 6, 7, 8, 9 or 10 e.g. methyl, ethyl methyl, ethyl, propyl, iso-propyl, butyl, iso-butyl, *tert-butyl,* pentyl, hexyl, pentyl, or octyl. Alkyl groups are optionally substituted.

The term "heteroalkyl" refers to a saturated straight or branched carbon chain. Preferably, the chain comprises from 1 to 9 carbon atoms, i.e. 1, 2, 3, 4, 5, 6, 7, 8, 9 e.g. methyl, ethyl, propyl, iso-propyl, butyl, iso-butyl, *sec-butyl, tert-butyl,* pentyl, hexyl, pentyl, octyl, which is interrupted one or more times, e.g. 1, 2, 3, 4, 5, with the same or different heteroatoms. Preferably the heteroatoms are selected from O, S, and N, e.g. -O-CH₃, -S-CH₃, -CH₂-O-CH₃, -CH₂-O-C₂H₅, -CH₂-S-CH₃, -CH₂-S-C₂H₅, -C₂H₄-O-CH₃, -C₂H₄-O-C₂H₃, -C₂H₄-S-CH₃, - C₂H₄-S-C₂H₅ etc. Heteroalkyl groups are optionally substituted.

The terms "cycloalkyl" and "heterocycloalkyl", by themselves or in combination with other terms, represent, unless otherwise stated, cyclic versions of "alkyl" and "heteroalkyl", respectively, with preferably 3, 4, 5, 6, 7, 8, 9 or 10 atoms forming a ring, e.g. cyclopropyl, cyclobutyl, cyclopentyl, cyclohexyl, cycloheptyl, cyclooctyl etc. The terms "cycloalkyl" and "heterocycloalkyl" are also meant to include bicyclic, tricyclic and polycyclic versions thereof. The term "heterocycloalkyl" preferably refers to a saturated ring having five of which at least one member is a N, O or S atom and which optionally contains one additional O or one additional N; a saturated ring having six members of which at least one member is a N, O or S atom and which optionally contains one additional O or one additional N or two additional N atoms; or a saturated bicyclic ring having nine or ten members of which at least one member is a N, O or S atom and which optionally contains one, two or three additional N atoms. "Cycloalkyl" and "heterocycloalkyl" groups are optionally substituted. Additionally, for heterocycloalkyl, a heteroatom can occupy the position at which the heterocycle is attached to the remainder of the molecule. Examples of cycloalkyl include cyclopropyl, cyclobutyl, cyclopentyl, cyclohexyl, 1-cyclohexenyl, 3-cyclohexenyl, cycloheptyl, spiro[3,3]heptyl, spiro[3,4]octyl, spiro[4,3]octyl, spiro[3,5]nonyl, spiro[5,3]nonyl, spiro[3,6]decyl, spiro[6,3]decyl, spiro[4,5]decyl, spiro[5,4]decyl, bicyclo[2.2.1]heptyl, bicyclo[2.2.2]octyl, adamantyl, and the like. Examples of heterocycloalkyl include 1-(1,2,5,6-tetrahydropyridyl), 1-piperidinyl, 2-piperidinyl, 3-piperidinyl, 4-morpholinyl, 3-morpholinyl, 1,8 diazo-spiro-[4,5] decyl, 1,7 diazo-spiro-[4,5] decyl, 1,6 diazo-spiro-[4,5] decyl, 2,8 diazo-spiro[4,5] decyl, 2,7 diazo-spiro[4,5] decyl, 2,6 diazo-spiro[4,5] decyl, 1,8 diazo-spiro-[5,4] decyl, 1,7 diazo-spiro-[5,4] decyl, 2,8 diazo-spiro-[5,4] decyl, 2,7 diazo-spiro[5,4] decyl, 3,8 diazo-spiro[5,4] decyl, 3,7 diazo-spiro[5,4] decyl, 1-azo-7,11-dioxo-spiro[5,5] undecyl, 1,4-diazabicyclo[2.2.2]oct-2-yl, tetrahydrofuran-2-yl, tetrahydrofuran-3-yl, tetrahydrothien-2-yl, tetrahydrothien-3-yl, 1-piperazinyl, 2-piperazinyl, and the like.

The term "aryl" preferably refers to an aromatic monocyclic ring containing 6 carbon atoms, an aromatic bicyclic ring system containing 10 carbon atoms or an aromatic tricyclic ring system containing 14 carbon atoms. Examples are phenyl, naphtyl or anthracenyl. The aryl group is optionally substituted.

The term "aralkyl" refers to an alkyl moiety, which is substituted by aryl, wherein alkyl and aryl have the meaning as outlined above. An example is the benzyl radical. Preferably, in this context the alkyl chain comprises from 1 to 8 carbon atoms, i.e. 1, 2, 3, 4, 5, 6, 7, or 8, e.g. methyl, ethyl methyl, ethyl, propyl, iso-propyl, butyl, iso-butyl, sec-butenyl, *tert-butyl,* pentyl, hexyl, pentyl, octyl. The aralkyl group is optionally substituted at the alkyl and/or aryl part of the group.

The term "heteroaryl" preferably refers to a five or six-membered aromatic monocyclic ring wherein at least one of the carbon atoms are replaced by 1, 2, 3, or 4 (for the five membered ring) or 1, 2, 3, 4, or 5 (for the six membered ring) of the same or different heteroatoms, preferably selected from O, N and S; an aromatic bicyclic ring system wherein 1, 2, 3, 4, 5, or 6 carbon atoms of the 8, 9, 10, 11 or 12 carbon atoms have been replaced with the same or different heteroatoms, preferably selected from O, N and S; or an aromatic tricyclic ring system wherein 1, 2, 3, 4, 5, or 6 carbon atoms of the 13, 14, 15, or 16 carbon atoms have been replaced with the same or different heteroatoms, preferably selected from O, N and S. Examples are oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3,-thiadiazolyl, 1,2,5-thiadiazolyl, pyridinyl, pyrimidinyl, pyrazinyl, 1,2,3-triazinyl, 1,2,4-triazinyl, 1,3,5-triazinyl, 1-benzofuranyl, 2-benzofuranyl, indoyl, isoindoyl, benzothiophenyl, 2-benzothiophenyl, 1H-indazolyl, benzimidazolyl, benzoxazolyl, indoxazinyl, 2,1-benzosoxazoyl, benzothiazolyl, 1,2-benzisothiazolyl, 2,1-benzisothiazolyl, benzotriazolyl, quinolinyl, isoquinolinyl, quinoxalinyl, quinazolinyl, quinolinyl, 1,2,3-benzotriazinyl, or 1,2,4-benzotriazinyl.

The term "heteroaralkyl" refers to an alkyl moiety, which is substituted by heteroaryl, wherein alkyl and heteroaryl have the meaning as outlined above. An example is the 2-alklypyridinyl, 3-alkylpyridinyl, or 2-methylpyridinyl. Preferably, in this context the alkyl chain comprises from 1 to 8 carbon atoms, i.e. 1, 2, 3, 4, 5, 6, 7, or 8, e.g. methyl, ethyl methyl, ethyl, propyl, iso-propyl, butyl, iso-butyl, sec-butenyl, *tert-butyl,* pentyl, hexyl, pentyl, octyl. The heteroaralkyl group is optionally substituted at the alkyl and/or heteroaryl part of the group.

The terms "alkenyl" and "cycloalkenyl" refer to olefinic unsaturated carbon atoms containing chains or rings with one or more double bonds. Examples are propenyl and cyclohexenyl. Preferably, the alkenyl chain comprises from 2 to 8 carbon atoms, i.e. 2, 3, 4, 5, 6, 7, or 8, e.g. ethenyl, 1-propenyl, 2-propenyl, iso-propenyl, 1-butenyl, 2-butenyl, 3-butenyl, iso-butenyl, sec-butenyl, 1-pentenyl, 2-pentenyl, 3-pentenyl, 4-pentenyl, hexenyl, pentenyl, octenyl. Preferably the cycloalkenyl ring comprises from 3 to 8 carbon atoms, i.e. 3, 4, 5, 6, 7, or 8, e.g. 1-cyclopropenyl, 2-cyclopropenyl, 1-cyclobutenyl, 2-cylcobutenyl, 1-cyclopentenyl, 2-cyclopentenyl, 3-cyclopentenyl, cyclohexenyl, cyclopentenyl, cyclooctenyl.

The term "alkynyl" refers to unsaturated carbon atoms containing chains or rings with one or more triple bonds. An example is the propargyl radical. Preferably, the alkynyl chain comprises from 2 to 8 carbon atoms, i.e. 2, 3, 4, 5, 6, 7, or 8, e.g. ethynyl, 1-propynyl, 2-propynyl, 1-butynyl, 2-butynyl, 3-butynyl, 1-pentynyl, 2-pentynyl, 3-pentynyl, 4-pentynyl, hexynyl, pentynyl, octynyl.

In one embodiment, carbon atoms or hydrogen atoms in alkyl, heteroalkyl, cycloalkyl, aryl, aralkyl, alkenyl, cycloalkenyl, alkynyl radicals may be substituted independently from each other with one or more elements selected from the group consisting of O, S, N or with groups containing one or more elements selected from the group consisting of O, S, N.

Embodiments include alkoxy, cycloalkoxy, arykoxy, aralkoxy, alkenyloxy, cycloalkenyloxy, alkynyloxy, alkylthio, cycloalkylthio, arylthio, aralkylthio, alkenylthio, cycloalkenylthio, alkynylthio, alkylamino, cycloalkylamino, arylamino, aralkylamino, alkenylamino, cycloalkenylamino, alkynylamino radicals.

Other embodiments include hydroxyalkyl, hydroxycycloalkyl, hydroxyaryl, hydroxyaralkyl, hydroxyalkenyl, hydroxycycloalkenyl, hydroxyalinyl, mercaptoalkyl, mercaptocycloalkyk, mercaptoaryl, mercaptoaralkyl, mercaptoalkenyl, mercaptocycloalkenyl, mercaptoalkynyl, aminoalkyl, aminocycloalkyl, aminoaryl, aminoaralkyl, aminoalkenyl, aminocycloalkenyl, aminoalkynyl radicals.

In another embodiment, hydrogen atoms in alkyl, heteroalkyl, cycloalkyl, aryl, aralkyl, alkenyl, cycloalkenyl, alkynyl radicals may be substituted independently from each other with one or more halogen atoms. One radical is the trifluoromethyl radical.

If two or more radicals or two or more residues can be selected independently from each other, then the term "independently" means that the radicals or the residues may be the same or may be different.

As used herein a wording defining the limits of a range of length such as, e. g., "from 1 to 6" means any integer from 1 to 6, i. e. 1, 2, 3, 4, 5 and 6. In other words, any range defined by two integers explicitly mentioned is meant to comprise and disclose any integer defining said limits and any integer comprised in said range.

The term "halo" as used herein refers to a halogen residue selected from the group consisting of F, Br, I and Cl. Preferably, the halogen is F.

The term "linker" as used herein refers to any chemically suitable linker. Preferably, linker are not or only slowly cleaved under physiological conditions. Thus, it is preferred that the linker does not comprise recognition sequences for proteases or recognition structures for other degrading enzymes. Since it is preferred that the compounds of the invention are administered systemically to allow broad access to all compartments of the body and subsequently enrichment of the compounds of the invention wherever in the body the tumor is located, it is preferred that the linker is chosen in such that it is not or only slowly cleaved in blood. The cleavage is considered slowly, if less than 50% of the linkers are cleaved 2 h after administration of the compound to a human patient. Suitable linkers, for example, comprises or consists of optionally substituted alkyl, heteroalkyl, cycloalkyl, cycloheteroalkyl, aryl, heteroaryl, aralkyl, heteroaralyl, alkenyl, heteroalkenyl, cycloalkenyl, cycloheteroalkenyl, alkynyl, sulfonyl, amines, ethers, thioethers phosphines, phosphoramidates, carboxamides, esters, imidoesters, amidines, thioesters, sulfonamides, 3-thiopyrrolidine-2,5-dion, carbamates, ureas, guanidines, thioureas, disulfides, oximes, hydrazines, hydrazides, hydrazones, diaza bonds, triazoles, triazolines, tetrazines, platinum complexes and amino acids, or combinations thereof. Preferably, the linker comprises or consists of 1,4-piperazine, 1,3-propane and a phenolic ether or combinations thereof.

The expression "optionally substituted" refers to a group in which one, two, three or more hydrogen atoms may have been replaced independently of each other by the respective substituents.

As used herein, the term "amino acid" refers to any organic acid containing one or more amino substituents, e.g. α-, β- or γ-amino, derivatives of aliphatic carboxylic acids. In the polypeptide notation used herein, e.g. Xaa5, i.e. Xaa1Xaa2Xaa3Xaa4Xaa5, wherein Xaa1 to Xaa5 are each and independently selected from amino acids as defined, the left hand direction is the amino terminal direction and the right hand direction is the carboxy terminal direction, in accordance with standard usage and convention.

The term "conventional amino acid" refers to the twenty naturally occurring amino acids, and encompasses all stereomeric isoforms, i.e. D,L-, D- and L-amino acids thereof. These conventional amino acids can herein also be referred to by their conventional three- letter or one-letter abbreviations and their abbreviations follow conventional usage (see, for example, Immunology-A Synthesis, 2nd Edition, E. S. Golub and D. R. Gren, Eds., Sinauer Associates, Sunderland Mass. (1991)).

The term "non-conventional amino acid" refers to unnatural amino acids or chemical amino acid analogues, e.g. α,α-disubstituted amino acids, N-alkyl amino acids, homo-amino acids, dehydroamino acids, aromatic amino acids (other than phenylalanine, tyrosine and tryptophan), and ortho-, meta- or para-aminobenzoic acid. Non-conventional amino acids also include compounds which have an amine and carboxyl functional group separated in a 1,3 or larger substitution pattern, such as β-alanine, γ-amino butyric acid, Freidinger lactam, the bicyclic dipeptide (BTD) , amino-methyl benzoic acid and others well known in the art. Statine-like isosteres, hydroxyethylene isosteres, reduced amide bond isosteres, thioamide isosteres, urea isosteres, carbamate isosteres, thioether isosteres, vinyl isosteres and other amide bond isosteres known to the art may also be used. The use of analogues or non-conventional amino acids may improve the stability and biological half-life of the added peptide since they are more resistant to breakdown under physiological conditions. The person skilled in the art will be aware of similar types of substitution which may be made. A non-limiting list of non-conventional amino acids which may be used as suitable building blocks for a peptide and their standard abbreviations (in brackets) is as follows: α-aminobutyric acid (Abu), L-N-methylalanine (Nmala), α-amino-α-methylbutyrate (Mgabu), L-N-methylarginine (Nmarg), aminocyclopropane (Cpro), L-N-methylasparagine (Nmasn), carboxylate L-N-methylaspartic acid (Nmasp), aniinoisobutyric acid (Aib), L-N-methylcysteine (Nmcys), aminonorbornyl (Norb), L-N-methylglutamine (Nmgln), carboxylate L-N-methylglutamic acid (Nmglu), cyclohexylalanine (Chexa), L-N-methylhistidine (Nmhis), cyclopentylalanine (Cpen), L-N-methylisolleucine (Nmile), L-N-methylleucine (Nmleu), L-N-methyllysine (Nmlys), L-N-methylmethionine (Nmmet), L-N-methylnorleucine (Nmnle), L-N-methylnorvaline (Nmnva), L-N-methylornithine (Nmorn), L-N-methylphenylalanine (Nmphe), L-N-methylproline (Nmpro), L-N-methylserine (Nmser), L-N-methylthreonine (Nmthr), L-N-methyltryptophan (Nmtrp), D-ornithine (Dorn), L-N-methyltyrosine (Nmtyr), L-N-methylvaline (Nmval), L-N-methylethylglycine (Nmetg), L-N-methyl-t-butylglycine (Nmtbug), L-norleucine (NIe), L-norvaline (Nva), α-methyl-aminoisobutyrate (Maib), α-methyl-γ-aminobutyrate (Mgabu), D-α-methylalanine (Dmala), α-methylcyclohexylalanine (Mchexa), D-α-methylarginine (Dmarg), α-methylcylcopentylalanine (Mcpen), D-α-methylasparagine (Dmasn), α-methyl-α-napthylalanine (Manap), D-α-methylaspartate (Dmasp), α-methylpenicillamine (Mpen), D-α-methylcysteine (Dmcys), N-(4-aminobutyl)glycine (NgIu), D-α-methylglutamine (Dmgln), N-(2-aminoethyl)glycine (Naeg), D-α-methylhistidine (Dmhis), N-(3 -aminopropyl)glycine (Norn), D-α-methylisoleucine (Dmile), N-amino-α-methylbutyrate (Nmaabu), D-α-methylleucine (Dmleu), α-napthylalanine (Anap), D-α-methyllysine (Dmlys), N-benzylglycine (Nphe), D-α-methylmethionine (Dmmet), N-(2-carbamylethyl)glycine (NgIn), D-α-methylornithine (Dmorn), N-(carbamylmethyl)glycine (Nasn), D-α-methylphenylalanine (Dmphe), N-(2-carboxyethyl)glycine (NgIu), D-α-methylproline (Dmpro), N-(carboxymethyl)glycine (Nasp), D-α-methylserine (Dmser), N-cyclobutylglycine (Ncbut), D-α-methylthreonine (Dmthr), N-cycloheptylglycine (Nchep), D-α-methyltryptophan (Dmtrp), N-cyclohexylglycine (Nchex), D-α-methyltyrosine (Dmty), N-cyclodecylglycine (Ncdec), D-α-methylvaline (Dmval), N-cylcododecylglycine (Ncdod), D-N-methylalanine (Dnmala), N-cyclooctylglycine (Ncoct), D-N-methylarginine (Dnmarg), N-cyclopropylglycine (Ncpro), D-N-methylasparagine (Dnmasn), N-cycloundecylglycine (Ncund), D-N-methylaspartate (Dnmasp), N-(2,2-diphenylethyl)glycine (Nbhm), D-N-methylcysteine (Dnmcys), N-(3,3-diphenylpropyl)glycine (Nbhe), D-N-methylglutamine (Dnmgln), N-(3 - guanidinopropyl)glycine (Narg), D-N-methylglutamate (Dnmglu), N-( 1 - hydroxyethyl)glycine (Ntbx), D-N-methylhistidine (Dnmhis), N-(hydroxyethyl))glycine (Nser), D-N-methylisoleucine (Dnmile), N-(imidazolylethyl))glycine (Nhis), D-N-methylleucine (Dnmleu), N-(3 -indolylyethyl)glycine (Nhtrp), D-N-methyllysine (Dnnilys), N-methyl-γ-aminobutyrate (Nmgabu), N-methylcyclohexylalanine (Nmchexa), D-N-methylmethionine (Dnmmet), D-N-methylornithine (Dnmorn), N-methylcyclopentylalanine (Nmcpen), N-methylglycine (NaIa), D-N-methylphenylalanine (Dnmphe), N-methylaminoisobutyrate (Nmaib), D-N-methylproline (Dnmpro), N-( 1 -methylpropyl)glycine (Nile), D-N-methylserine (Dnmser), N-(2-methylpropyl)glycine (Nleu), D-N-methylthreonine (Dnmthr), D-N-methyltryptophan (Dnmtrp), N-(1-methylethyl)glycine (Nval), D-N-methyltyrosine (Dnmtyr), N-methyla-napthylalanine (Nmanap), D-N-methylvaline (Dnmval), N-methylpenicillamine (Nmpen), γ-aminobutyric acid (Gabu), N-(p-hydroxyphenyl)glycine (Nhtyr), L-/-butylglycine (Tbug), N-(thiomethyl)glycine (Ncys), L-ethylglycine (Etg), penicillamine (Pen), L-homophenylalanine (Hphe), L-α-methylalanine (Mala), L-α-methylarginine (Marg), L-α-methylasparagine (Masn), L-α-methylaspartate (Masp), L-α-methyl-t-butylglycine (Mtbug), L-α-methyleysteine (Mcys), L-methylethylglycine (Metg), L-α-methylglutamine (MgIn), L-α-methylglutamate (MgIu), L-α-methylhistidine (Mhis), L-α-methylhomophenylalanine (Mhphe), L-α-methylisoleucine (Mile), N-(2-methylthioethyl)glycine (Nmet), L-α-methylleucine (Mleu), L-α-methyllysine (Mlys), L-α-methylmethionine (Mmet), L-α-methylnorleucine (MnIe), L-α-methylnorvaline (Mnva), L-α-methylornithine (Morn), L-α-methylphenylalanine (Mphe), L-α-methylproline (Mpro), L-α-methylserine (Mser), L-α-methylthreonine (Mthr), L-α-methyltryptophan (Mtrp), L-α-methyltyrosine (Mtyr), L-α-methylvaline (Mval), L-N-methylhomophenylalanine (Nmhphe), N-(N-(2,2-diphenylethyl)carbamylmethyl)glycine (Nnbhm), N-(N-(3 ,3 -diphenylpropyl)-carbamylmethyl)glycine (Nnbhe), 1-carboxy-1-(2,2-diphenyl-ethylamino)cyclopropane (Nmbc), L-O-methyl serine (Omser), L-O-methyl homoserine (Omhser).

The term "N-containing aromatic or non-aromatic mono or bicyclic heterocycle" as used herein refers to a cyclic saturated or unsaturated hydrocarbon compound which contains at least one nitrogen atom as constituent of the cyclic chain.

The term "radioactive moiety" as used herein refers to a molecular assembly which carries a radioactive nuclide. The nuclide is bound either by covalent or coordinate bonds which remain stable under physiological conditions. Examples are [¹³¹I]-3-iodobenzoic acid or ⁶⁸Ga-DOTA.

A "fluorescent isotope" as used herein emits electromagnetic radiation after excitation by electromagnetic radiation of a shorter wavelength.

A "radioisotope" as used herein is a radioactive isotope of an element (included by the term "radionuclide") emitting α-, β-, and/or γ-radioation.

The term "radioactive drug" is used in the context of the present invention to refer to a biologic active compound which is modified by a radioisotope. Especially intercalating substances can be used to deliver the radioactivity to direct proximity of DNA (e.g. a ¹³¹I-carrying derivative of Hoechst-33258).

The term "chelating agent" or "chelate" are used interchangeably in the context of the present invention and refer to a molecule, often an organic one, and often a Lewis base, having two or more unshared electron pairs available for donation to a metal ion. The metal ion is usually coordinated by two or more electron pairs to the chelating agent. The terms, "bidentate chelating agent", "tridentate chelating agent, and "tetradentate chelating agent" refer to chelating agents having, respectively, two, three, and four electron pairs readily available for simultaneous donation to a metal ion coordinated by the chelating agent. Usually, the electron pairs of a chelating agent forms coordinate bonds with a single metal ion; however, in certain examples, a chelating agent may form coordinate bonds with more than one metal ion, with a variety of binding modes being possible.

The term "fluorescent dye" is used in the context of the present invention to refer to a compound that emits visible or infrared light after excitation by electromagnetic radiation of a shorter and suitable wavelength. It is understood by the skilled person, that each fluorescent dye has a predetermined excitation wavelength.

The term "contrast agent" is used in the context of the present invention to refer to a compound which increases the contrast of structures or fluids in medical imaging. The enhancement is achieved by absorbing electromagnetic radiation or altering electromagnetic fields.

The term "paramagnetic" as used herein refers to paramagnetism induced by unpaired electrons in a medium. A paramagnetic substance induces a magnetic field if an external magnetic field is applied. Unlike diamagnetism the direction of the induced field is the same as the external field and unlike ferromagnetism the field is not maintained in absence of an external field.

The term "nanoparticle" as used herein refers to particles preferably of spheric shape, with diameters of sizes between 1 and 100 nanometers. Depending on the composition, nanoparticles can possess magnetical, optical or physico-chemical qualities that can be assessed. Additionally surface modification is achievable for many types of nanoparticles.

The term "pharmaceutically acceptable salt" refers to a salt of the compound of the present invention. Suitable pharmaceutically acceptable salts of the compound of the present invention include acid addition salts which may, for example, be formed by mixing a solution of choline or derivative thereof with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound of the invention carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include but are not limited to: acetate, adipate, alginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, subacetate, succinate, tannate, tartrate, teoclate, tosylate, triethiodide, undecanoate, valerate, and the like (see, for example, Berge, S. M., et al, "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In addition to salt forms, the present invention provides compounds which are in a prodrug form. Prodrugs of the compounds described herein are those compounds that readily undergo chemical changes under physiological conditions to provide a compound of formula (I). A prodrug is an active or inactive compound that is modified chemically through *in vivo* physiological action, such as hydrolysis, metabolism and the like, into a compound of this invention following administration of the prodrug to a patient. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme. The suitability and techniques involved in making and using prodrugs are well known by those skilled in the art. For a general discussion of prodrugs involving esters see Svensson and Tunek Drug Metabolism Reviews 16.5 (1988) and Bundgaard Design of Prodrugs, Elsevier (1985). Examples of a masked carboxylate anion include a variety of esters, such as alkyl (for example, methyl, ethyl), cycloalkyl (for example, cyclohexyl), aralkyl (for example, benzyl, p-methoxybenzyl), and alkylcarbonyloxyalkyl (for example, pivaloyloxymethyl). Amines have been masked as arylcarbonyloxymethyl substituted derivatives which are cleaved by esterases in vivo releasing the free drug and formaldehyde (Bungaard J. Med. Chem. 2503 (1989)). Also, drugs containing an acidic NH group, such as imidazole, imide, indole and the like, have been masked with N-acyloxymethyl groups (Bundgaard Design of Prodrugs, Elsevier (1985)). Hydroxyl groups have been masked as esters and ethers. EP 0 039 051 (Sloan and Little, Apr. 11, 1981) discloses Mannich-base hydroxamic acid prodrugs, their preparation and use.

Compounds according to the invention can be synthesized according to one or more of the following methods. It should be noted that the general procedures are shown as it relates to preparation of compounds having unspecified stereochemistry. However, such procedures are generally applicable to those compounds of a specific stereochemistry, e.g., where the stereochemistry about a group is (S) or (R). In addition, the compounds having one stereochemistry (e.g., (R)) can often be utilized to produce those having opposite stereochemistry (i.e., (S)) using well-known methods, for example, by inversion.

Certain compounds of the present invention can exist in unsolvated forms as well as in solvated forms, including hydrated forms. In general, the solvated forms are equivalent to unsolvated forms and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, geometric isomers and individual isomers are all intended to be encompassed within the scope of the present invention.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

The term "pharmaceutical composition" as used in the present application refers to a substance and/or a combination of substances being used for the identification, prevention or treatment of a tissue status or disease. The pharmaceutical composition is formulated to be suitable for administration to a patient in order to prevent and/or treat disease. Further a pharmaceutical composition refers to the combination of an active agent with a carrier, inert or active, making the composition suitable for therapeutic use. Pharmaceutical compositions can be formulated for oral, parenteral, topical, inhalative, rectal, sublingual, transdermal, subcutaneous or vaginal application routes according to their chemical and physical properties. Pharmaceutical compositions comprise solid, semisolid, liquid, transdermal therapeutic systems (TTS). Solid compositions are selected from the group consisting of tablets, coated tablets, powder, granulate, pellets, capsules, effervescent tablets or transdermal therapeutic systems. Also comprised are liquid compositions, selected from the group consisting of solutions, syrups, infusions, extracts, solutions for intravenous application, solutions for infusion or solutions of the carrier systems of the present invention. Semisolid compositions that can be used in the context of the invention comprise emulsion, suspension, creams, lotions, gels, globules, buccal tablets and suppositories.

"Pharmaceutically acceptable" means approved by a regulatory agency of the Federal or a state government or listed in the U.S. Pharmacopeia or other generally recognized pharmacopeia for use in animals, and more particularly in humans.

The term "carrier", as used herein, refers to a diluent, adjuvant, excipient, or vehicle with which the therapeutic agent is administered. Such pharmaceutical carriers can be sterile liquids, such as saline solutions in water and oils, including those of petroleum, animal, vegetable or synthetic origin, such as peanut oil, soybean oil, mineral oil, sesame oil and the like. A saline solution is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. Examples of suitable pharmaceutical carriers are described in "Remington's Pharmaceutical Sciences" by E. W. Martin.

The term "fibroblast activation protein (FAP)" as used herein is also known under the term "seprase". Both terms can be used interchangeably herein. Fibroblast activation protein is a homodimeric integral protein with dipeptidyl peptidase IV (DPPIV)-like fold, featuring an alpha/beta-hydrolase domain and an eight-bladed beta-propeller domain.

### Embodiments

In the following different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a first aspect, the present invention provides a compound of Formula (I) 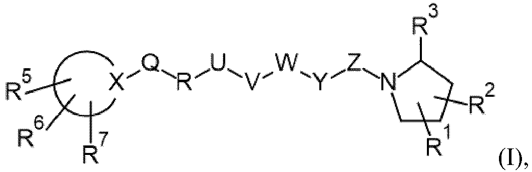 wherein
Q, R, U, V, W, Y, Z are individually present or absent under the proviso that at least three of Q, R, U, V, W, Y, Z are present;
Q, R, U, V, W, Y, Z are independently selected form the group consisting of O, CH₂, NR⁴, C=O, C=S, C=NR⁴, HCR⁴ and R⁴CR⁴, with the proviso that two Os are not directly adjacent to each other; preferably out of the six four groups are present of which two are C=O, one is CH₂ and one is NH; more preferably four groups are present of which two are C=O, one is CH₂ and one is NH; most preferably, V, W, Y and Z are present of which V and Z are C=O and W and Y are independently selected from CH₂ and NH;
R¹ and R² are independently selected from the group consisting of -H, -OH, halo, C₁₋₆-alkyl, - O-C₁₋₆-alkyl, S-C₁₋₆-alkyl;
R³ is selected from the group consisting of -H , -CN , -B(OH)₂, -C(O) -alkyl, -C(O) -aryl-, - C=C-C(O) -aryl, -C=C-S(O)₂-aryl, -CO₂H , -SO₃H , -SO₂NH₂,-PO₃H₂, and 5-tetrazolyl;
R⁴ is selected from the group consisting of -H, -C₁₋₆-alkyl, -O-C₁₋₆-alkyl, -S-C₁₋₆-alkyl, alkenyl, heteroalkenyl, cycloalkenyl, cycloheteroalkenyl, alkynyl, aryl, and -C₁₋₆-aralkyl, each of said - C₁₋₆-alkyl being optionally substituted with from 1 to 3 substituents selected from -OH, oxo, halo and optionally connected to Q, R, U, V, W, Y or Z;
R⁵ is selected from the group consisting of -H, halo and C₁₋₆-alkyl;
R⁶, and R⁷ are independently selected from the group consisting of -H, 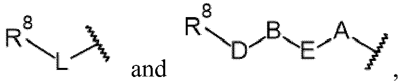 under the proviso that R⁶ and R⁷ are not at the same time H, preferably R⁶ is attached to the 7- or 8-quinolyl position and R⁷ is attached to the 5- or 6- quinolyl position; more preferably R⁶ is attached to the 7-quinolyl position and R⁷ is attached to the 6-quinolyl position,
wherein L is a linker,
wherein D, A, E, and B are individually present or absent, preferably wherein at least A, E,
and B are present, wherein when present:
   D is a linker;
   A is selected from the group consisting of NR⁴, O, S, and CH₂;
   E is selected from the group consisting of
      C₁₋₆-alkyl, 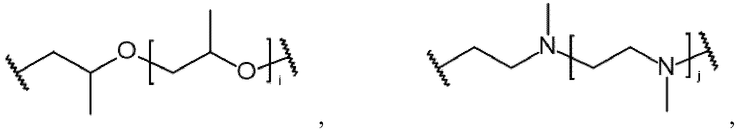 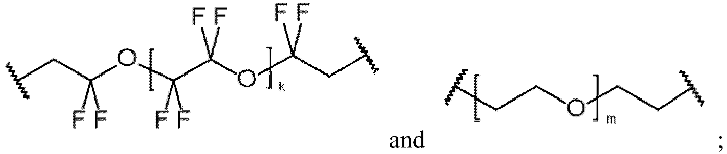
   wherein i is 1, 2, or 3;
   wherein j is 1, 2, or 3;
   wherein k is 1, 2, or 3;
   wherein m is 1, 2, or 3;
   more preferably, E is C₁₋₆-alkyl, most preferably, E is C3 or C4 alkyl;
   A and E together form a group selected from: a cycloalkyl, heterocycloalkyl, aryl and heteroaryl, preferably heterocycloalkyl, wherein A and E can be mono-, bi- and multicyclic, preferably monocyclic. Each A and E being optionally substituted with 1 to 4 substituents selected from -H, -C₁₋₆-alkyl, -O-C₁₋₆-alkyl, -S-C₁₋₆-alkyl, alkenyl, heteroalkenyl, cycloalkenyl, cycloheteroalkenyl, alkynyl, aryl, and -C₁₋₆-aralkyl, each of said -C₁₋₆-alkyl being optionally substituted with from 1 to 3 substituents selected from -OH, oxo, halo; and optionally connected to A, B, D, E or 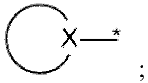
   B is selected from the group consisting of S, NR⁴, NR⁴-O, NR⁴-C₁₋₆-alkyl, NR⁴-C₁₋₆-alkyl-NR⁴, and a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein NR⁴-C₁₋₆-alkyl-NR⁴ and the N-containing heterocycle is substituted with 1 to 3 substituents selected the group consisting of C₁₋₆-alkyl, aryl, C₁₋₆-aralkyl; and
   R⁸ is selected from the group consisting of radioactive moiety, chelating agent, fluorescent dye, a contrast agent and combinations thereof; 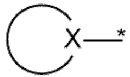
   is a 1-naphtyl moiety or a 5 to 10- membered N-containing aromatic or non-aromatic mono- or bicyclic heterocycle, wherein there are 2 ring atoms between the N atom and X; said heterocycle optionally further comprising 1, 2 or 3 heteroatoms selected from O, N and S; and X is a C atom;
   or a pharmaceutically acceptable tautomer, racemate, hydrate, solvate, or salt thereof. Preferably, C₁₋₆-alkyl is selected from the group consisting of methyl, ethyl, propyl, i-propyl, butyl, sec-butyl, tert-butyl, pentyl and hexyl.
In a preferred embodiment, A and E together form a group selected from the group consisting of a C₃, C₄, C₅, C₆, C₇ and C₈ monocyclic, preferably C₅ or C₆ monocyclic, or C₇, C₈, C₉, C₁₀, C₁₁ or C₁₂ bicyclic, preferably C₇, C₈, C₉ and C₁₀ bicyclic heterocycloalkyl, comprising 1, 2, 3, or 4, preferably 1 or 2 heteroatoms independently selected from the group consisting of N, O and S, preferably N and O, most preferably 1 or 2 N.

In a preferred embodiment of the first aspect of the present invention a compound of Formula (I) is provided: 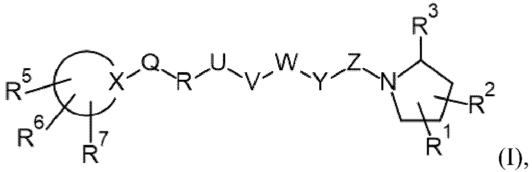 wherein
Q, R, U, V, W, Y, Z are individually present or absent under the proviso that at least three of Q, R, U, V, W, Y, Z are present;
Q, R, U, V, W, Y, Z are independently selected form the group consisting of O, CH₂, NR⁴, C=O, C=S, C=NR⁴, HCR⁴ and R⁴CR⁴, with the proviso that two Os are not directly adjacent to each other; preferably out of the six four groups are present of which two are C=O, one is CH₂ and one is NH; more preferably four groups are present of which two are C=O, one is CH₂ and one is NH; most preferably, V, W, Y and Z are present of which V and Z are C=O and W and Y are independently selected from CH₂ and NH;
R¹ and R² are independently selected from the group consisting of -H, -OH, halo, C₁₋₆-alkyl, - O-C₁₋₆-alkyl, S-C₁₋₆-alkyl;
R³ is selected from the group consisting of -H , -CN , -B(OH)₂, -C(O) -alkyl, -C(O) -aryl-, - C=C-C(O) -aryl, -C=C-S(O)₂-aryl, -CO₂H , -SO₃H , -SO₂NH₂,-PO₃H₂, and 5-tetrazolyl;
R⁴ is selected from the group consisting of -H, -C₁₋₆-alkyl, -O-C₁₋₆-alkyl, -S-C₁₋₆-alkyl, alkenyl, heteroalkenyl, cycloalkenyl, cycloheteroalkenyl, alkynyl, aryl, and -C₁₋₆-aralkyl, each of said - C₁₋₆-alkyl being optionally substituted with from 1 to 3 substituents selected from -OH, oxo, halo and optionally connected to Q, R, U, V, W, Y or Z;
R⁵ is selected from the group consisting of -H, halo and C₁₋₆-alkyl;
R⁶, and R⁷ are independently selected from the group consisting of -H, 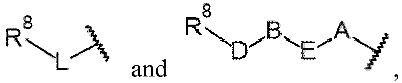 under the proviso that R⁶ and R⁷ are not at the same time H, preferably R⁶ is attached to the 7- or 8-quinolyl position and R⁷ is attached to the 5- or 6- quinolyl position; more preferably R⁶ is attached to the 7-quinolyl position and R⁷ is attached to the 6-quinolyl position,
wherein L is a linker,
wherein D, A, E, and B are individually present or absent, preferably wherein at least A, E, and B are present, wherein when present:
   D is a linker;
   A is selected from the group consisting of NR⁴, O, S, and CH₂;
   E is selected from the group consisting of
      C₁₋₆-alkyl, 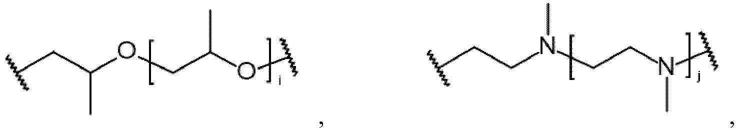 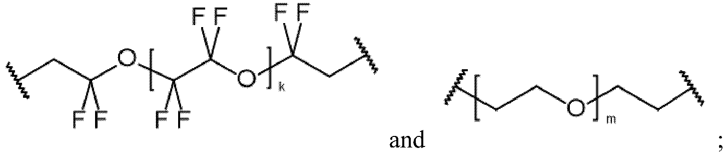
   wherein i is 1, 2, or 3;
   wherein j is 1, 2, or 3;
   wherein k is 1, 2, or 3;
   wherein m is 1, 2, or 3;
   more preferably, E is C₁₋₆-alkyl, most preferably, E is C3 or C4 alkyl;
   B is selected from the group consisting of S, NR⁴, NR⁴-O, NR⁴-C₁₋₆-alkyl, NR⁴-C₁₋₆-alkyl-NR⁴, and a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein NR⁴-C₁₋₆-alkyl-NR⁴ and the N-containing heterocycle is substituted with 1 to 3 substituents selected the group consisting of C₁₋₆-alkyl, aryl, C₁₋₆-aralkyl; and
   R⁸ is selected from the group consisting of radioactive moiety, chelating agent, fluorescent dye, a contrast agent and combinations thereof; 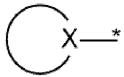
   is a 1-naphtyl moiety or a 5 to 10- membered N-containing aromatic or non-aromatic mono- or bicyclic heterocycle, wherein there are 2 ring atoms between the N atom and X; said heterocycle optionally further comprising 1, 2 or 3 heteroatoms selected from O, N and S; and X is a C atom;
   or a pharmaceutically acceptable tautomer, racemate, hydrate, solvate, or salt thereof. Preferably, C₁₋₆-alkyl is selected from the group consisting of methyl, ethyl, propyl, i-propyl, butyl, sec-butyl, tert-butyl, pentyl and hexyl.

In another preferred embodiment of the first aspect of the present invention A and E together form a group consisting of a C₃, C₄, C₅, C₆, C₇ and C₈ monocyclic, preferably C₅ or C₆ monocyclic, or C₇, C₈, C₉, C₁₀, C₁₁ or C₁₂ bicyclic, preferably C₇, C₈, C₉ and C₁₀ bicyclic heterocycloalkyl, preferably comprising 1, 2, 3, or 4, more preferably 1 or 2 heteroatoms independently selected from the group consisting of N, O and S, preferably N and O, most preferably 1 or 2 N. Preferred monocyclic heterocycloalkyls are selected from the group consisting of pyrrolidinyl, piperidinyl, imidazolidinyl, 1,2-diazacyclohexanyl, 1,3-diazacyclohexanyl, piperazinyl, 1-oxo-2-azacyclohexanyl, 1-oxo-3-azacyclohexanyl, or morpholinyl, preferably piperidinyl, piperazinyl, and pyrrolidinyl. Preferred bicyclic heterocycloalkyls are selected from the group consisting of bicyclo[2.2.1] 2,5-diazaheptanyl, 3,6-diazabicyclo[3.2.1]octanyl, 3,6-diazabicyclo[3.2.2]nonyl, octahydropyrrolo[2,3-b]pyrrolyl, octahydropyrrolo[3,2-b]pyrrolyl, octahydropyrrolo[3,4-b]pyrrolyl, octahydropyrrolo[3,4-c]pyrrolyl, 9-methyl-3,7,9-triazabicyclo[3.3.1]nonanyl.

The bond between the heterocycle formed by A and E and B on one hand and/or R⁶ or R⁷ on the other is preferably through the heteroatom, preferably through N.

Particularly, preferred examples of the heterocycle formed by A and E are selected from the group consisting of 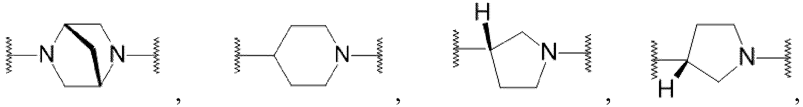 and

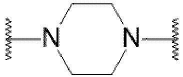

In a preferred embodiment of the first aspect of the present invention,
Q, R, U are CH₂ and are individually present or absent; preferably, Q and R are absent; V is CH₂, C=O, C=S or C=NR⁴; preferably, V is C=O;
W is NR⁴; preferably, W is NH;
Y is HCR⁴; preferably, Y is CH₂; and
Z is C=O, C=S or C=NR⁴, preferably, Z is C=O.

In a further preferred embodiment of the first aspect of the present invention,
Q, R, U are absent;
V is CH₂;
W is NH;
Y is CH₂; and
Z is C=O.

In a further preferred embodiment of the first aspect of the present invention,
R¹ and R² are independently selected from the group consisting of -H and halo; preferably, R¹ and R² are halo; more preferably, R¹ and R² are F;
R³ is selected from the group consisting of -H, -CN, and -B(OH)₂; preferably, R³ is -CN or - B(OH)₂; more preferably, R³ is -CN;
R⁴ is selected from the group consisting of -H and -C₁₋₆-alkyl, wherein the -C₁₋₆-alkyl is optionally substituted with from 1 to 3 substituents selected from -OH. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
Q, R, U are absent;
V is CH₂;
W is NH;
Y is CH₂;
Z is C=O;
R¹ and R² are independently selected from the group consisting of -H and halo; preferably, R¹ and R² are halo; more preferably, R¹ and R² are F;
R³ is selected from the group consisting of -H, -CN, and -B(OH)₂; preferably, R³ is -CN or - B(OH)₂; more preferably, R³ is -CN;
R⁴ is selected from the group consisting of -H and -C₁₋₆-alkyl, wherein the -C₁₋₆-alkyl is optionally substituted with from 1 to 3 substituents selected from -OH. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
Q, R, U are absent;
V is CH₂;
W is CH₂;
Y is NH;
Z is C=O;
R¹ and R² are independently selected from the group consisting of -H and halo; preferably, R¹ and R² are halo; more preferably, R¹ and R² are F;
R³ is selected from the group consisting of -H, -CN, and -B(OH)₂; preferably, R³ is -CN or - B(OH)₂; more preferably, R³ is -CN;
R⁴ is selected from the group consisting of -H and -C₁₋₆-alkyl, wherein the -C₁₋₆-alkyl is optionally substituted with from 1 to 3 substituents selected from -OH. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention, 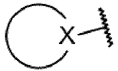 is selected from the group consisting of 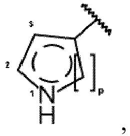 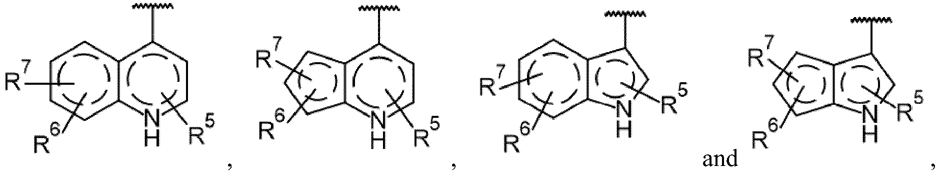 optionally further comprising 1 or 2 heteroatoms selected from O, N, and S.

In a further preferred embodiment of the first aspect of the present invention, 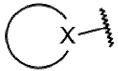 is 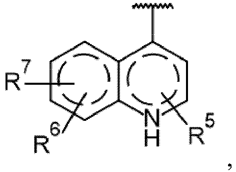 optionally further comprising 1 or 2 heteroatoms selected from O, N, and S.

In a further preferred embodiment of the first aspect of the present invention, 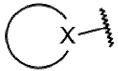 is selected from the group consisting of 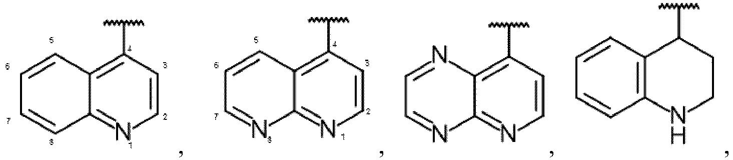 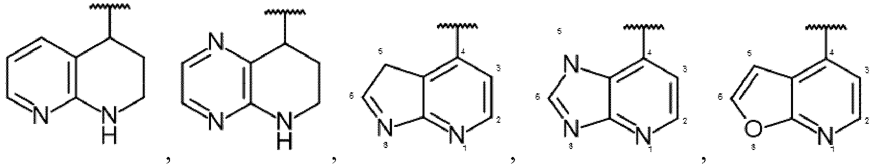 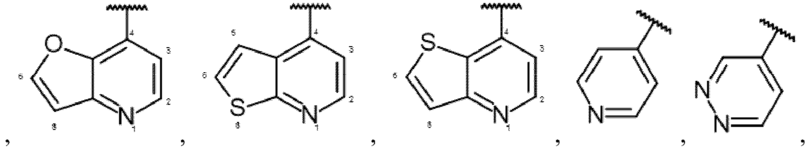 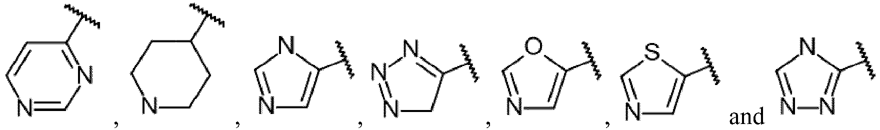 . R⁶, and R⁷ are independently selected from the group consisting of -H, 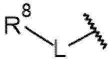 and 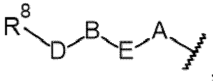 , under the proviso that R⁶ and R⁷ are not at the same time H and preferably R⁶ and R⁷ are attached on positions 5, 6 or 7.
In a preferred embodiment, 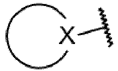 is selected from the group consisting of 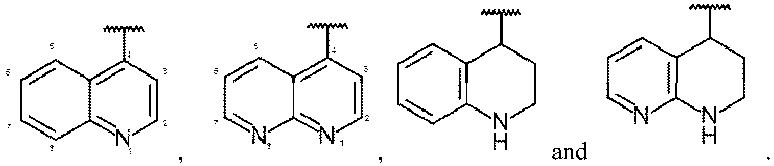 In another preferred embodiment 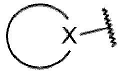 is

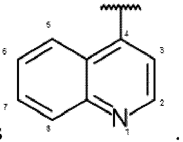

In a further preferred embodiment of the first aspect of the present invention, R⁵ and R⁶ are H;
R⁷ is 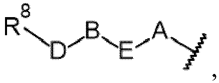 preferably R⁷ is attached to the 5- or 6-quinolyl position; more preferably R⁷ is attached to the 6-quinolyl position, wherein D is absent;
A is O, S, CH₂, NH, NCH₃;
E is C₁₋₆-alkyl or 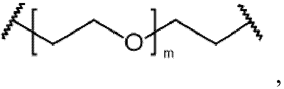 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
   or
A and E together form a group selected from: 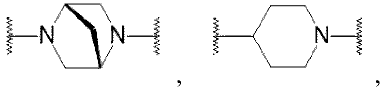 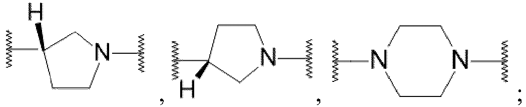
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 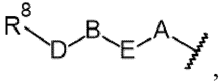 preferably R⁷ is attached to the 5- or 6-quinolyl position; more preferably R⁷ is attached to the 6-quinolyl position, wherein
D is absent;
A is O;
E is C₁₋₆-alkyl or 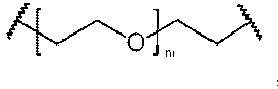 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 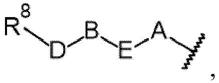 preferably R⁷ is attached to the 5- or 6-quinolyl position; more preferably R⁷ is attached to the 6-quinolyl position, wherein
D is absent;
A is S;
E is C₁₋₆-alkyl or 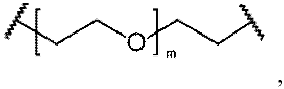 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 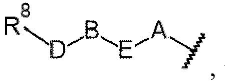 preferably R⁷ is attached to the 5- or 6-quinolyl position; more preferably R⁷ is attached to the 6-quinolyl position, wherein
D is absent;
A is CH₂;
E is C₁₋₆-alkyl or 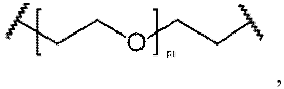 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR¹-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 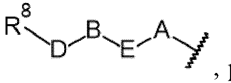 preferably R⁷ is attached to the 5- or 6-quinolyl position; more preferably R⁷ is attached to the 6-quinolyl position, wherein
D is absent;
A is NH;
E is C₁₋₆-alkyl or 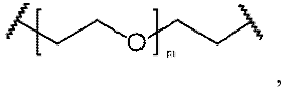 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 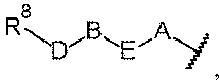 preferably R⁷ is attached to the 5- or 6-quinolyl position; more preferably R⁷ is attached to the 6-quinolyl position, wherein
D is an amino acid, preferably carrying a charged side chain;
A is O;
E is C₁₋₆-alkyl or 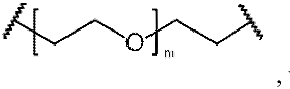 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 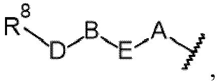 preferably R⁷ is attached to the 5- or 6-quinolyl position; more preferably R⁷ is attached to the 6-quinolyl position, wherein
D is an amino acid, preferably carrying a charged side chain;
A is S;
E is C₁₋₆-alkyl or 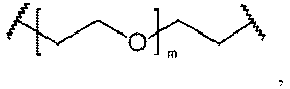 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 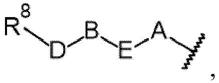 preferably R⁷ is attached to the 5- or 6-quinolyl position; more preferably R⁷ is attached to the 6-quinolyl position, wherein
D is an amino acid, preferably carrying a charged side chain;
A is CH₂;
E is C₁₋₆-alkyl or 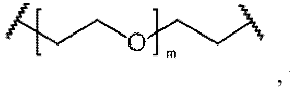 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 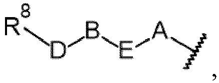 preferably R⁷ is attached to the 5- or 6-quinolyl position; more preferably R⁷ is attached to the 6-quinolyl position, wherein
D is an amino acid, preferably carrying a charged side chain;
A is NH;
E is C₁₋₆-alkyl or 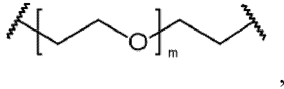 wherein m is 1, 2, or 3; Preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl. Preferably, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention,
R⁵ and R⁶ are H;
R⁷ is 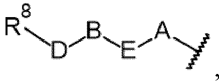 preferably R⁷ is attached to the 5- or 6-quinolyl position; more preferably R⁷ is attached to the 6-quinolyl position, wherein
D is absent;
A is O;
E is C₁₋₆-alkyl or 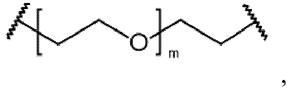 wherein m is 1, 2, or 3; Preferably, E is C₁₋₆-alkyl and C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is a 5- to 10-membered N-containing aromatic or non-aromatic mono- or bicyclic heterocycle, preferably further comprising 1 or 2 nitrogen atoms.

In a further preferred embodiment of the first aspect of the present invention,
R⁵ and R⁶ are H;
R⁷ is 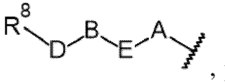 preferably R⁷ is attached to the 5- or 6-quinolyl position; more preferably R⁷ is attached to the 6-quinolyl position, wherein
D is absent;
A is O;
E is C3 or C4 alkyl; more preferably, E is propyl or butyl;
B is a 5- to 10-membered N-containing aromatic or non-aromatic mono- or bicyclic heterocycle, preferably further comprising 1 or 2 nitrogen atoms.

In a further preferred embodiment of the first aspect of the present invention,
the N-containing heterocycle comprised in B is an aromatic or non-aromatic monocyclic heterocycle: 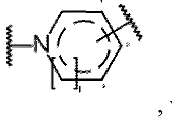 wherein
the heterocycle optionally further comprises 1 or 2 heteroatoms selected form O, N and S, optionally further comprises 1 nitrogen; 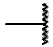
is attached to position 1, 2, or 3, preferably to position 2;
1 is 1 or 2.

In a further preferred embodiment of the first aspect of the present invention,
the N-containing heterocycle comprised in B is an aromatic or non-aromatic monocyclic heterocycle: 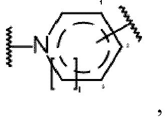 wherein
the heterocycle optionally further comprises 1 or 2 heteroatoms selected form O, N and S, optionally further comprises 1 nitrogen; 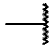
is attached to position 1, 2, or 3, preferably to position 2;
1 is 1 or 2;
wherein the N-containing heterocycle is substituted with a C₁₋₆-alkyl.

In a further preferred embodiment of the first aspect of the present invention, the N-containing heterocycle comprised in B is selected from the group consisting of: 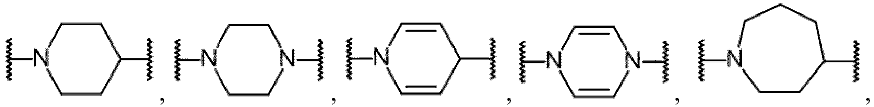 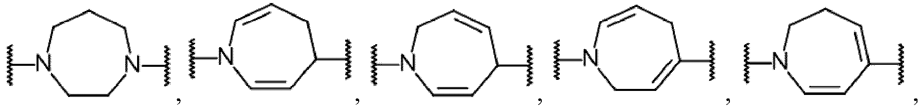 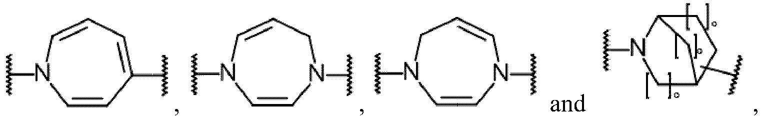
wherein the N-containing heterocycle is substituted with a C₁₋₆-alkyl
wherein if the N-containing heterocycle comprised in B is 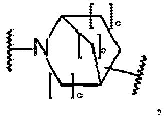 the heterocycle optionally further comprises 1 or 2 heteroatoms selected from O, N and S, optionally further comprises 1 nitrogen, optionally compromises one or more (e.g. amino acid derived) side chains; 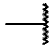
is attached to position 1, 2, or 3, preferably to position 2;
o is 1 or 2;
preferably, if the N-containing heterocycle comprised in B is 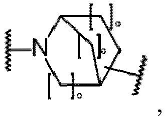 the N-containing heterocycle comprised in B is selected from the group consisting of 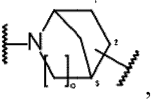 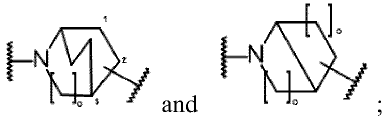 more preferably, if the N-containing heterocycle comprised in B is 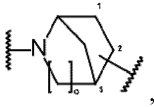 the N-containing heterocycle comprised in B is

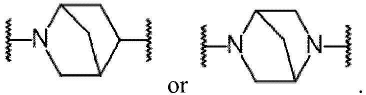

In a further preferred embodiment of the first aspect of the present invention, the N-containing heterocycle comprised in B is selected from the group consisting of: 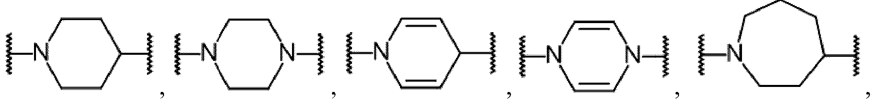 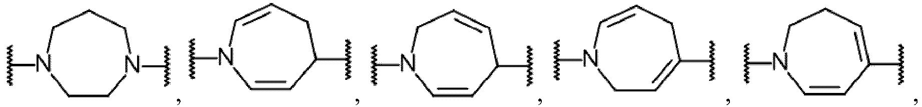 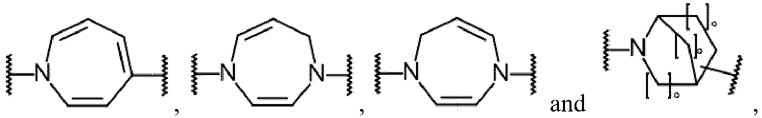
wherein if the N-containing heterocycle comprised in B is 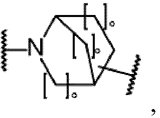 the heterocycle optionally further comprises 1 or 2 heteroatoms selected from O, N and S, optionally further comprises 1 nitrogen, optionally compromises one or more (e.g. amino acid derived) side chains; 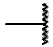
is attached to position 1, 2, or 3, preferably to position 2;
o is 1 or 2;
preferably, if the N-containing heterocycle comprised in B is 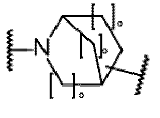 , the N-containing heterocycle comprised in B is selected from the group consisting of 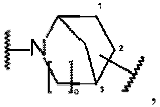 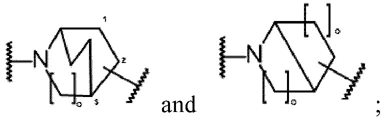 more preferably, if the N-containing heterocycle comprised in B is 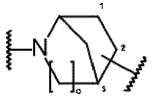 , the N-containing heterocycle comprised in B is

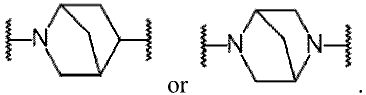

In a further preferred embodiment of the first aspect of the present invention, the N-containing heterocycle comprised in B is selected from the group consisting of:

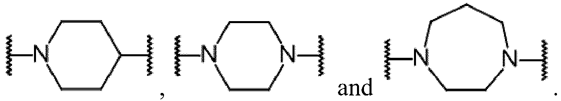

In a further preferred embodiment of the first aspect of the present invention, the N-containing heterocycle comprised in B is selected from the group consisting of: 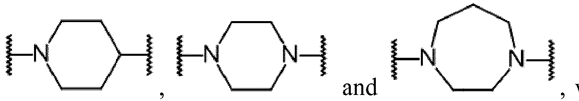 wherein B is substituted with a C₁₋₃ alkyl.

In a further preferred embodiment of the first aspect of the present invention,
R⁵ and R⁶ are H;
R⁷ is 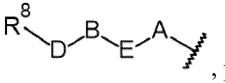 preferably R⁷ is attached to the 6-quinolyl position, wherein
D is absent;
A is O;
E is propyl or butyl;
B is is

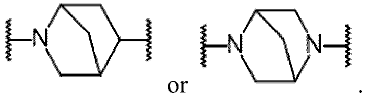

In a further preferred embodiment of the first aspect of the present invention,
Q, R, U are absent;
V is C=O;
W is NH;
Y is CH₂;
Z is C=O;
R¹ and R² are independently selected from the group consisting of -H and halo; preferably, R¹ and R² are independently selected from the group consisting of -H and F; more preferably, R¹ and R² are the same and are selected from the group consisting of -H and F;
R³ is -CN;
R⁵ and R⁶ are H;
R⁷ is 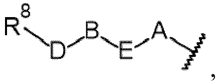 preferably R⁷ is attached to the 6-quinolyl position, wherein
D is absent;
A is O;
E is C₁₋₆-alkyl or 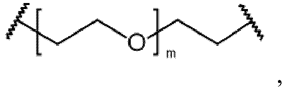 wherein m is 1, 2, or 3; preferably, E is C₁₋₆-alkyl; preferably, C₁₋₆-alkyl is selected from the group consisting of methyl, ethyl, propyl, i-propyl, butyl, sec-butyl, tert-butyl, pentyl and hexyl; more preferably, E is C₁₋₆-alkyl, most preferably, E is C3 or C4 alkyl;
B is NH-C₁₋₆-alkyl, 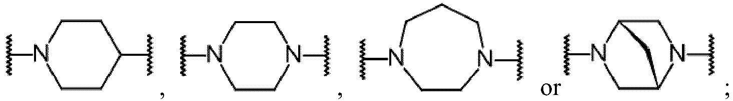 preferably, C₁₋₆-alkyl is selected from the group consisting of methyl, ethyl, propyl, i-propyl, butyl, sec-butyl, tert-butyl, pentyl and hexyl; preferably, B is 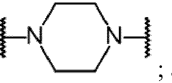 and 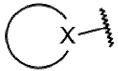
is

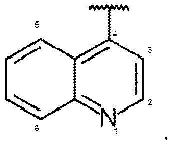

In a further preferred embodiment of the first aspect of the present invention,
Q, R, U are absent;
V is C=O;
W is NH;
Y is CH₂;
Z is C=O;
R¹ and R² are the same and are selected from the group consisting of -H and F;
R³ is -CN;
R⁵ and R⁶ are H;
R⁷ is 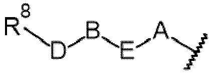 , preferably R⁷ is attached to the 6-quinolyl position, wherein D is absent;
A is O, S, CH₂, NH, NCH₃;
E is methyl, ethyl, propyl or butyl;
A and E together form a group selected from: 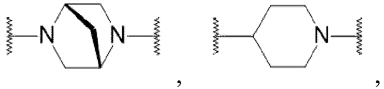 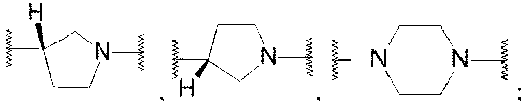
B is 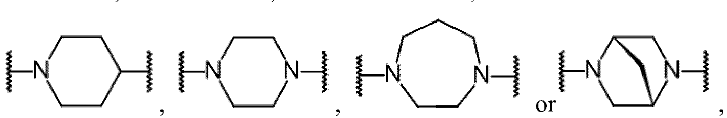 optionally B is substituted with a C₁₋₃ alkyl; preferably, B is 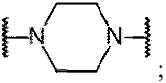 and 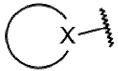
is

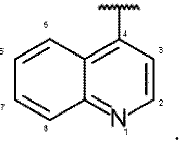

In a further preferred embodiment of the first aspect of the present invention,
Q, R, U are absent;
V is C=O;
W is NH;
Y is CH₂;
Z is C=O;
R¹ and R² are the same and are selected from the group consisting of -H and F;
R³ is -CN;
R⁵ and R⁶ are H;
R⁷ is 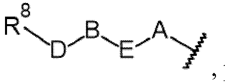 preferably R⁷ is attached to the 6-quinolyl position, wherein
D is absent;
A is O;
E is methyl, ethyl, propyl or butyl;
B is 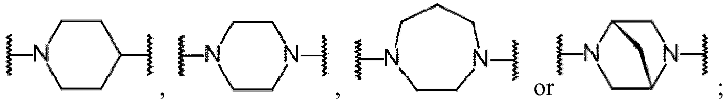 preferably, B is 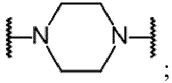 and 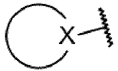
is

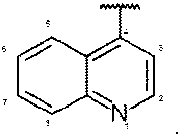

In a further preferred embodiment of the first aspect of the present invention,
Q, R, U are absent;
V is C=O;
W is NH;
Y is CH₂;
Z is C=O;
R¹ and R² are the same and are selected from the group consisting of -H and F;
R³ is -CN;
R⁵ and R⁶ are H;
R⁷ is 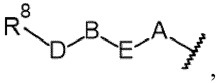 R⁷ is attached to the 6-quinolyl position, wherein
D is absent;
A is O;
E is methyl, ethyl, propyl or butyl;
B is 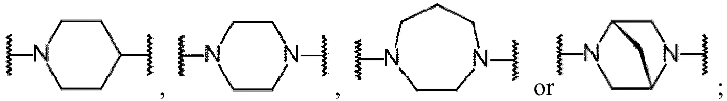 preferably, B is 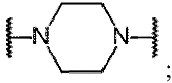 and 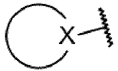
is

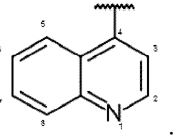

In a further preferred embodiment of the first aspect of the present invention, C₁₋₆-alkyl is selected from the group consisting of methyl, ethyl, propyl, i-propyl, butyl, sec-butyl, tert-butyl, pentyl and hexyl.

In a further preferred embodiment of the first aspect of the present invention, C₁₋₃-alkyl is selected from the group consisting of methyl, ethyl, propyl and i-propyl.

In a further preferred embodiment of the first aspect of the present invention, C₁₋₆-aralkyl is selected from the group consisting of benzyl, phenyl-ethyl, phenyl-propyl, and phenyl-butyl.

In a preferred embodiment of the first aspect of the present invention, the compound of the first aspect of the invention is selected from the compounds of table 1. More preferably, the compound of the first aspect of the invention is selected from the compounds of table 2. More preferably, the compound of the first aspect of the invention is selected from the group consisting of FAPI-02 and FAPI-04.

In a preferred embodiment of the first aspect of the present invention, the compound of the first aspect of the invention is selected from the compounds of table 1 and/or table 3. More preferably, the compound of the first aspect of the invention is selected from the compounds of table 2 and/or table 4. More preferably, the compound of the first aspect of the invention is selected from the group consisting of FAPI-02, FAPI-04, FAPI-46, FAPI-34, FAPI-42, FAPI-52, FAPI-69, FAPI-70, FAPI-71, FAPI-72 and FAPI-73.

**Table 1: Preferred compounds of the first aspect of the invention.**

| § fluorescent compounds; $ ^{99m}Tc-chelators; * Pb-chelators; R¹ and R² are located at the 4-pyrrolidine position; Q, R, U are absent; 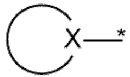 is 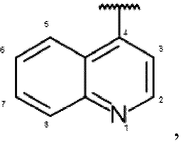 R⁵ is H; R⁶ is attached to the 7-quinolyl position; R⁷ is attached to the 6-quinolyl position; '-' indicates that R⁶ or R⁷ being H; '+' indicates R⁶ or R⁷ being 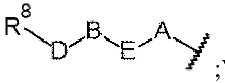 V is C=O; W is NH; Y is CH₂; Z is C=O; R³ is -CN; A is O (except FAPI-01: A is absent, R⁷ is attached to the 5-quinolyl position). | | | | | | | |
|---|---|---|---|---|---|---|---|
| **name** | **R¹/R²** | **R⁶** | **R⁷** | **R⁸** | **D** | **B** | **E** |
| **FAPI-01** | H/H | - | + | I | - | - | - |
| **FAPI-02** | H/H | - | + | 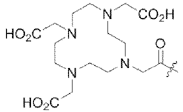 | - | 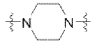 | *n*-C₃H₆ |
| **FAPI-02 atto 488^{§}** | H/H | - | + | 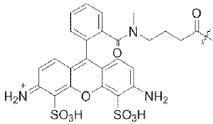 | - | 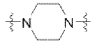 | *n*-C₃H₆ |
| **FAPI-02 dy800 CW^{§}** | H/H | - | + | 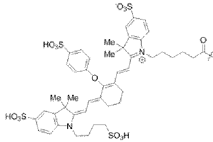 | - | 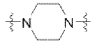 | *n*-C₃H₆ |
| **FAPI-03** | H/H | - | + | 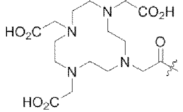 | - | 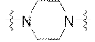 | n-C₄H₈ |
| **FAPI04** | F/F | - | + | 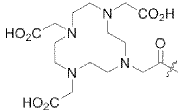 | - | 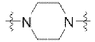 | *n*-C₃H₆ |

| **name** | **R¹/R²** | **R⁶** | **R⁷** | **R⁸** | **D** | **B** | **E** |
|---|---|---|---|---|---|---|---|
| **FAPI-04 atto 488^{§}** | **F/F** | **-** | **+** | 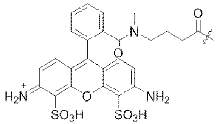 | **-** | 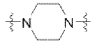 | *n*-C₃H₆ |
| **FAPI-05** | F/F | - | + | 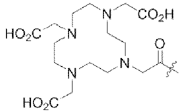 | - | 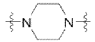 | n-C₄H₈ |
| **FAPI-06** | H/H | - | + | 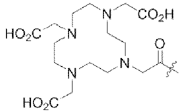 | - | 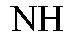 | *n*-C₃H₆ |
| **FAPI-07** | F/F | - | + | 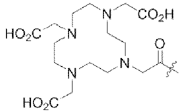 | - | 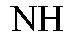 | *n*-C₃H₆ |
| **FAPI-08** | H/H | + | - | 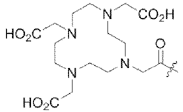 | - | 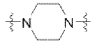 | *n*-C₃H₆ |
| **FAPI-09** | F/F | + | - | 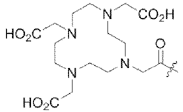 | - | 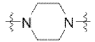 | *n*-C₃H₆ |
| **FAPI-10** | F/F | - | + | 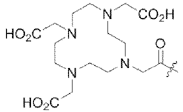 | 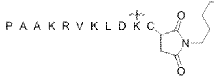 | 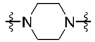 | *n*-C₃H₆ |
| **FAPI-11** | F/F | - | + | 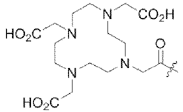 | - | 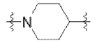 | CH₂ |
| **FAPI-12** | F/F | - | + | 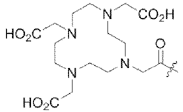 | - | 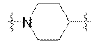 | n-C₂H₄ |
| **FAPI-13** | F/F | - | + | 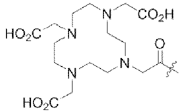 | - | 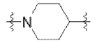 | *n*-C₃H₆ |
| **FAPI-14** | F/F | - | + | 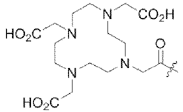 | - | 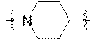 | n-C₄H₈ |
| **FAPI-15** | F/F | - | + | 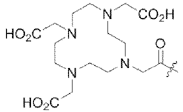 | - | 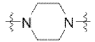 | (C₂H₄O) ₂C₂H₄ |
| **FAPI-16** | F/F | - | + | 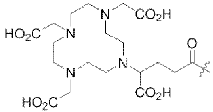 | - | 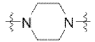 | *n*-C₃H₆ |
| **FAPI-17** | F/F | - | + | 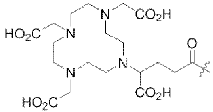 | 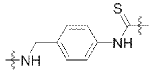 | 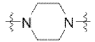 | *n*-C₃H₆ |
| **FAPI-18** | F/F | - | + | 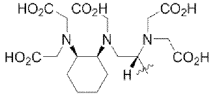 | 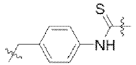 | 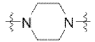 | *n*-C₃H₆ |
| **FAPI-19^{$}** | F/F | - | + | 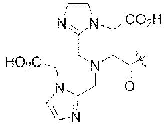 | - | 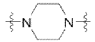 | *n*-C₃H₆ |
| **FAPI-20** | F/F | - | + | 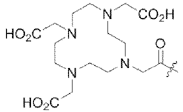 | - | 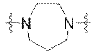 | *n*-C₃H₆ |
| **FAPI-21** | F/F | - | + | 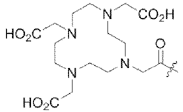 | - | 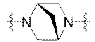 | *n*-C₃H₆ |
| **FAPI-22** | F/F | - | + | 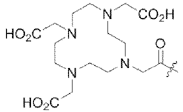 | - | 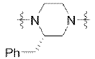 | *n*-C₃H₆ |
| **FAPI-23** | F/F | - | + | 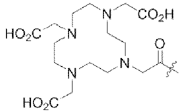 | - | 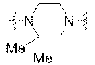 | *n*-C₃H₆ |
| **FAPI-24** | F/F | - | + | 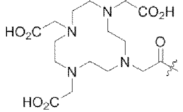 | - | 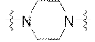 | (C₂H₄O) ₃C₂H₄ |
| **FAPI-25** | F/F | - | + | 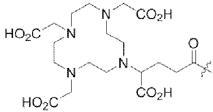 | 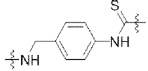 | 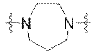 | *n*-C₃H₆ |
| **FAPI-26** | F/F | - | + | 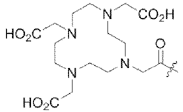 | - | 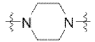 | (C₂H₄O) ₂C₂H₄ |
| **FAPI-27^{$}** | F/F | - | + | 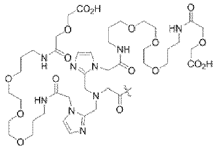 | - | 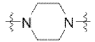 | *n*-C₃H₆ |
| **FAPI-28^{$}** | F/F | - | + | 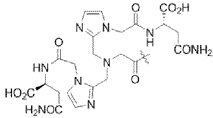 | - | 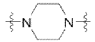 | *n*-C₃H₆ |
| **FAPI-29^{$}** | F/F | - | + | 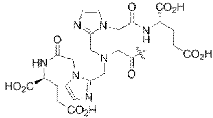 | - | 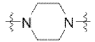 | *n*-C₃H₆ |
| **FAPI-30** | F/F | - | + | 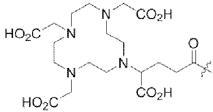 | - | 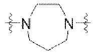 | *n*-C₃H₆ |
| **FAPI-31** | F/F | - | + | 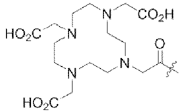 | - | 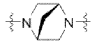 | *n*-C₃H₆ |
| **FAPI-32*** | F/F | - | + | 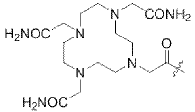 | - | 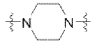 | *n*-C₃H₆ |
| **FAPI-33^{$}** | F/F | - | + | 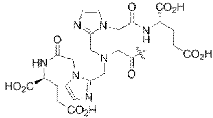 | - | 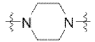 | (C₂H₄O) ₂C₂H₄ |
| **FAPI-34^{$}** | F/F | - | + | 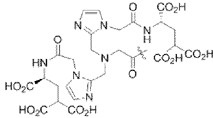 | - | 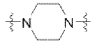 | *n*-C₃H₆ |
| **FAPI-35** | F/F | - | + | 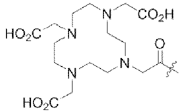 | - | 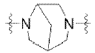 | *n*-C₃H₆ |
| **FAPI-36** | F/F | - | + | 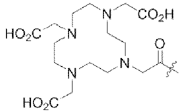 | - | 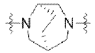 | *n*-C₃H₆ |
| **FAPI-37** | F/F | - | + | 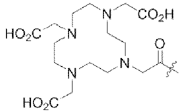 | 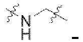 | 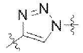 | *n*-C₃H₆ |
| **FAPI-38** | F/F | - | + | 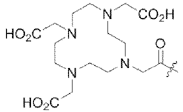 | D-Arg | 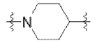 | *n*-C₃H₆ |

| **Table 2:** Compounds of special interest. Q, R, U, D are absent; R¹ and R² are located at the 4-pyrrolidine position; 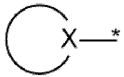 is 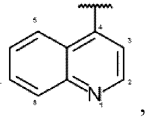 R⁵, R⁶ are H; R⁷ is attached to the 6-quinolyl position; V is C=O; W is NH; Y is CH₂; Z is C=O; R³ is -CN; B is 1,4-piperazine; E is 1,3-propane; A is O. | | | |
|---|---|---|---|
| **name** | **Purpose** | **R¹/R²** | **R⁸** |
| ⁶⁸Ga-FAPI-02 | PET | H/H | 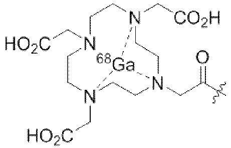 |
| ⁶⁸Ga-FAPI-04 | PET | F/F | 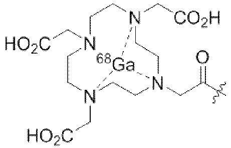 |
| Gd-FAPI-04 | MRI (contrast agent) | F/F | 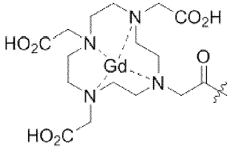 |
| ⁹⁰Y-FAPI-04 | radiotherapy (β⁻) | F/F | 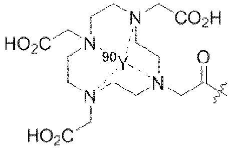 |
| ^{99m}Tc-FAPI-29 | SPECT | F/F | 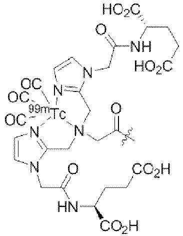 |
| 203Pb-FAPI-32 | SPECT | F/F | 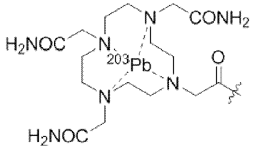 |
| ¹⁸F-FAPI-n.a. | PET | F/F | 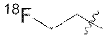 |
| ¹⁸⁸Re-FAPI-60 | radiotherapy (β⁻) | F/F | 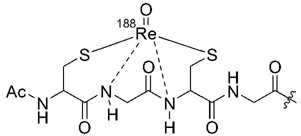 |
| Al¹⁸F-FAPI-42 | PET | F/F | 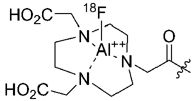 |
| ¹⁸F-FAPI-72 | PET | F/F | 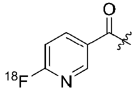 |

**Table 3: Further preferred compounds of the first aspect of the invention.**

| § fluorescent compounds; $ ^{99m}Tc-chelators; * precursors for ¹⁸F-labeling; Q, R, U are absent; R¹ and R² are located at the 4-pyrrolidine position; 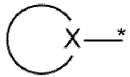 is 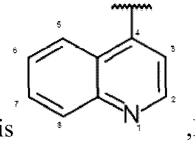 R⁵ and R⁶ are H; R⁷ is attached to the 6-quinolyl position and **is** 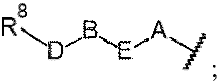 V is C=O; W is NH; Y is CH₂; Z is C=O; R³ is -CN. | | | | | | |
|---|---|---|---|---|---|---|
| **name** | **R¹/R²** | **R⁸** | **D** | **B** | **E** | **A** |
| **FAPI-39** | **F/F** | 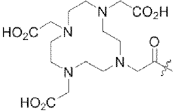 | - | 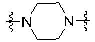 | *n*-C₃H₆ | CH₂ |
| **FAPI-40** | **F/F** | 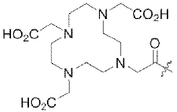 | - | 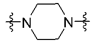 | *n*-C₃H₆ | S |
| **FAPI-41** | **F/F** | 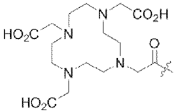 | - | 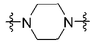 | *n*-C₃H₆ | NH |
| **FAPI-42** | **F/F** | 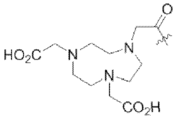 | - | 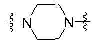 | *n*-C₃H₆ | O |
| **FAPI-43^{$}** | **F/F** | 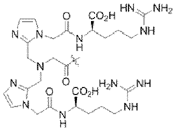 | - | 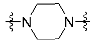 | *n*-C₃H₆ | O |
| **FAPI-44^{$}** | **F/F** | 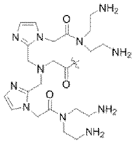 | - | 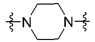 | *n*-C₃H₆ | O |
| **FAPI-45^{$}** | **F/F** | 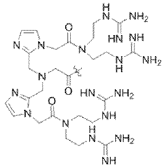 | - | 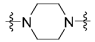 | *n*-C₃H₆ | O |

| **name** | **R¹/R²** | **R⁸** | **D** | **B** | **E** | **A** |
|---|---|---|---|---|---|---|
| **FAPI-46** | F/F | 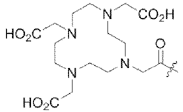 | - | 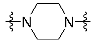 | *n*-C₃H₆ | NMe |
| **FAPI-47** | F/F | 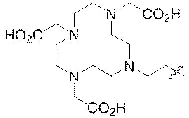 | - | 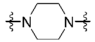 | *n*-C₃H₆ | O |
| **FAPI-48** | F/F | 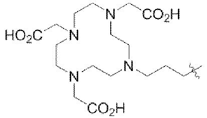 | - | 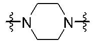 | *n*-C₃H₆ | O |
| **FAPI-49** | F/F | 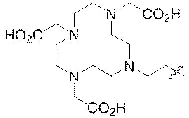 | - | 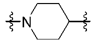 | *n*-C₃H₆ | O |
| **FAPI-50** | F/F | 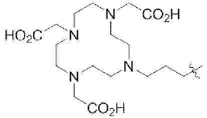 | - | 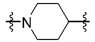 | *n*-C₃H₆ | O |
| **FAPI-51** | F/F | 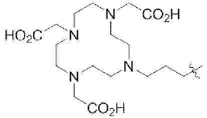 | - | 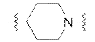 | *n*-C₃H₆ | O |
| **FAPI-52** | F/F | 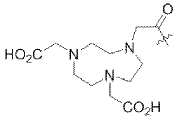 | - | 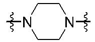 | *n*-C₃H₆ | NMe |
| **FAPI-53** | F/F | 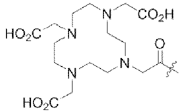 | - | 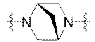 | *n*-C₃H₆ | NMe |
| **FAPI-54** | F/F | 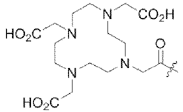 | - | 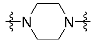 | 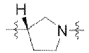 | |
| **FAPI-55** | F/F | 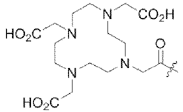 | - | 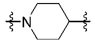 | *n*-C₃H₆ | NMe |
| **FAPI-56** | F/F | 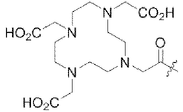 | - | 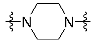 | 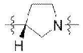 | |
| **FAPI-57** | F/F | 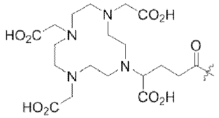 | - | 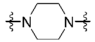 | 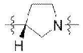 | |
| **FAPI-58** | F/F | 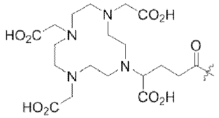 | - | 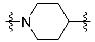 | *n*-C₃H₆ | NMe |
| **FAPI-59** | F/F | 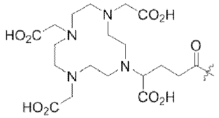 | - | 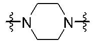 | *n*-C₃H₆ | NMe |
| **FAPI-60^{$}** | F/F | 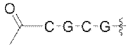 | - | 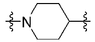 | *n*-C₃H₆ | O |
| **FAPI-61^{$}** | F/F | 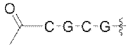 | - | 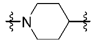 | *n*-C₃H₆ | O |
| **FAPI-62^{$}** | F/F | 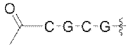 | - | 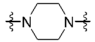 | *n*-C₃H₆ | NMe |
| **FAPI-63** | F/F | 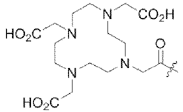 | - | 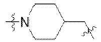 | 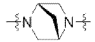 | |
| **FAPI-64** | F/F | 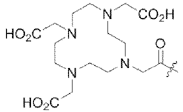 | - | 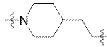 | 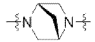 | |
| **FAPI-65** | F/F | 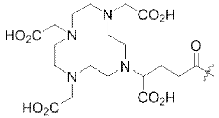 | - | 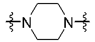 | 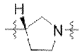 | |
| **FAPI-66** | F/F | 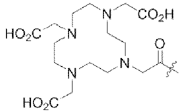 | - | 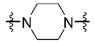 | 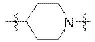 | |
| **FAPI-67** | F/F | 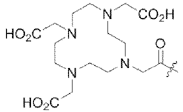 | - | 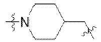 | 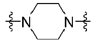 | |
| **FAPI-68** | F/F | 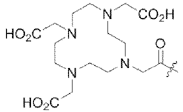 | - | 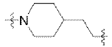 | 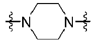 | |
| **FAPI-69^{$}** | F/F | 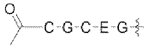 | - | 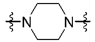 | *n*-C₃H₆ | NMe |
| **FAPI-70^{$}** | F/F | 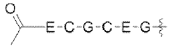 | - | 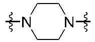 | *n*-C₃H₆ | NMe |
| **FAPI-71^{$}** | F/F | 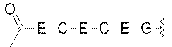 | - | 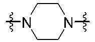 | *n*-C₃H₆ | NMe |
| **FAPI-72*** | F/F | 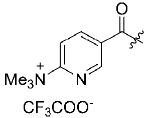 | - | 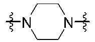 | *n*-C₃H₆ | NMe |
| **FAPI-73*** | F/F | 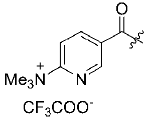 | - | 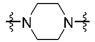 | *n*-C₃H₆ | O |
| **FAPI-74** | H/H | 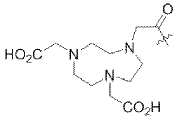 | - | 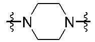 | *n*-C₃H₆ | O |
| **FAPI-75** | F/F | 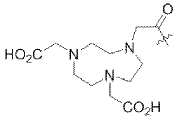 | 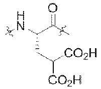 | 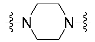 | *n*-C₃H₆ | O |
| **FAPI-76** | F/F | 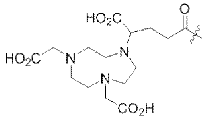 | - | 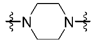 | *n*-C₃H₆ | O |
| **FAPI-77** | F/F | 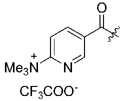 | 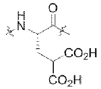 | 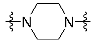 | *n*-C₃H₆ | O |
| **FAPI-77 protected** | F/F | 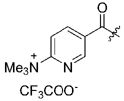 | 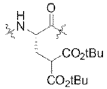 | 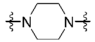 | *n*-C₃H₆ | O |
| **FAPI-78** | F/F | 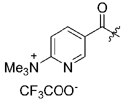 | 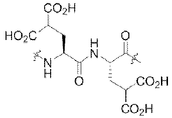 | 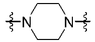 | *n*-C₃H₆ | O |
| **FAPI-78 protected** | F/F | 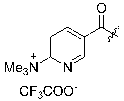 | 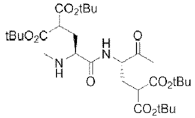 | 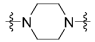 | *n*-C₃H₆ | O |
| **FAPI-79** | F/F | 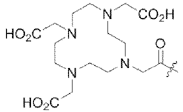 | 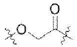 | 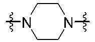 | *n*-C₃H₆ | O |
| **FAPI-80** | F/F | 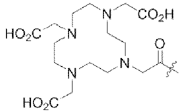 | 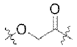 | 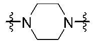 | *n*-C₃H₆ | NMe |

| **Table 4:** Compounds of special interest. Q, R, U, D are absent; R¹ and R² are fluorine atoms located at the 4-pyrrolidine position; 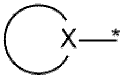 is 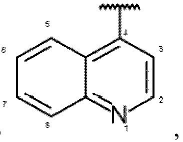 R⁵, R⁶ are H; R⁷ is attached to the 6-quinolyl position; V is C=O; W is NH; Y is CH₂; Z is C=O; R³ is -CN; B is 1,4-piperazine; E is 1,3-propane; A is O. | | |
|---|---|---|
| **name** | **Purpose** | **R⁸** |
| ¹⁸⁸Re-FAPI-60 | radiotherapy (β⁻) | 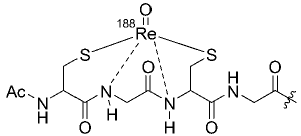 |
| Al¹⁸F-FAPI-42 | PET | 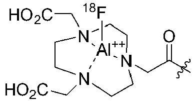 |
| ¹⁸F-FAPI-73 | PET | 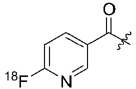 |

**Table 5: Preferred precursors for radiolabelling with ^{§} F-18; ^{$} Cu-64; ^{€} Ga-68; ^{£} Tc-99m, Re-188; * Y-90, Sm-153, Lu-177.**

| | |
|---|---|
| FAPI-02^{€,}* | 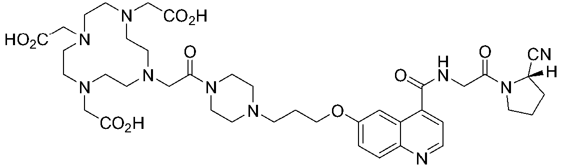 |
| FAPI-04^{€,}* | 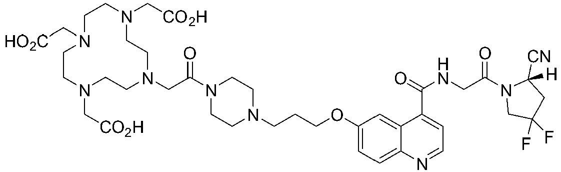 |
| FAPI-34^{£} | 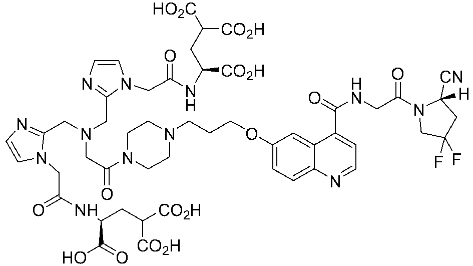 |
| FAPI-42^{§,$,€} | 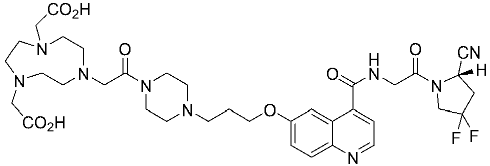 |
| FAPI-46^{€,}* | 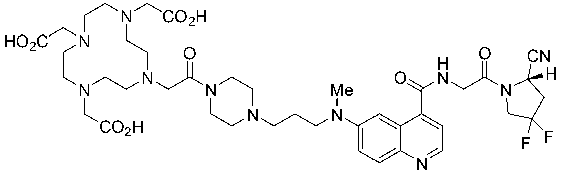 |
| FAPI-52^{§,$,€} | 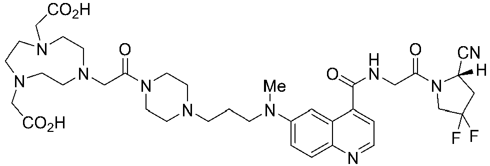 |
| FAPI-69^{£} | 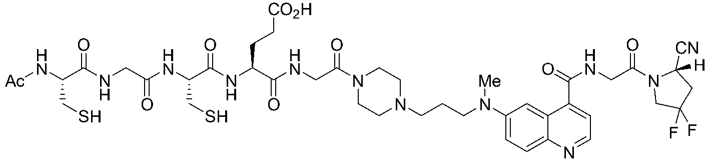 |
| FAPI-70^{£} | 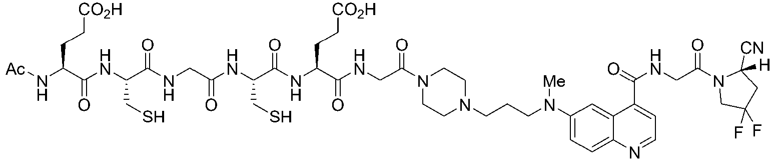 |
| FAPI-71^{£} | 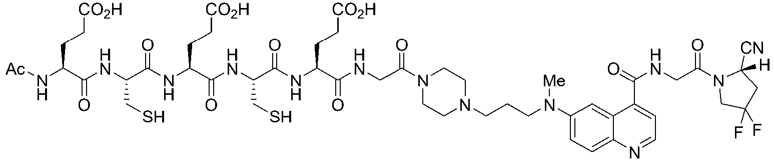 |
| FAPI-72^{§} | 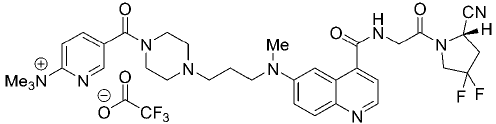 |
| FAPI-73^{§} | 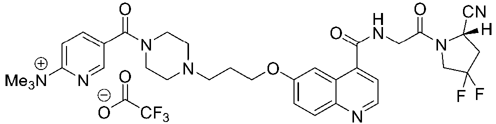 |
| FAPI-74^{§,$,€} | 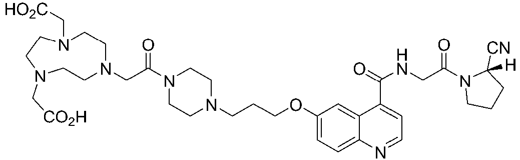 |
| FAPI-75^{§,$,€} | 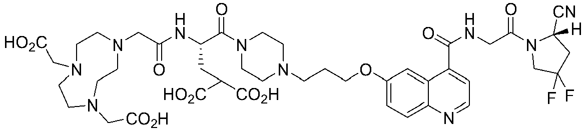 |

In a further preferred embodiment of the first aspect of the present invention, R⁸ is a radioactive moiety, wherein the radioactive moiety is a fluorescent isotope, a radioisotope, a radioactive drug or combinations thereof. Preferably, the radioactive moiety is selected from the group consisting of alpha radiation emitting isotopes, beta radiation emitting isotopes, gamma radiation emitting isotopes, Auger electron emitting isotopes, X-ray emitting isotopes, fluorescence emitting isotopes, such as ¹¹C, ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁸Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ²²⁷Th and ¹⁹⁹Ag. Preferably ¹⁸F, ⁶⁴Cu, ⁶⁸Ga, ⁹⁰Y, ^{99m}Tc, ¹⁵³Sm, ¹⁷⁷Lu, ¹⁸⁸Re.

In a further preferred embodiment of the first aspect of the present invention, R⁸ is a fluorescent dye select from the group consisting of the following classes of fluorescent dyes: Xanthens, Acridines, Oxazines, Cynines, Styryl dyes, Coumarines, Porphines, Metal-Ligand-Complexes, Fluorescent proteins, Nanocrystals, Perylenes, Boron-dipyrromethenes and Phtalocyanines as well as conjugates and combinations of these classes of dyes.

In a further preferred embodiment of the first aspect of the present invention, R⁸ is a chelating agent which forms a complex with divalent or trivalent metal cations. Preferably, the chelating agent is selected from the group consisting of 1,4,7,10-tetraazacyclododecane-N,N',N,*N'*-tetraacetic acid (DOTA), ethylenediaminetetraacetic acid (EDTA), 1,4,7-triazacyclononane-1,4,7-triacetic acid (NOTA), triethylenetetramine (TETA), iminodiacetic acid, diethylenetriamine-N,N,N',N',N"-pentaacetic acid (DTPA), bis-(carboxymethylimidazole)glycine and 6-Hydrazinopyridine-3-carboxylic acid (HYNIC).

In a further preferred embodiment of the first aspect of the present invention, R⁸ is a contrast agent which comprises or consists of a paramagnetic agent, preferably, wherein the paramagnetic agent comprises or consists of paramagnetic nanoparticles.

In a further preferred embodiment of the first aspect of the invention, R⁸ is selected from any R⁸ of tables 1 to 5.

In a second aspect, the present invention relates to a pharmaceutical composition comprising or consisting of at least one compound of the first aspect, and, optionally, a pharmaceutically acceptable carrier and/or excipient.

In a third aspect, the present invention relates to the compound of the first aspect or the pharmaceutical composition of the second aspect for use in the diagnosis or treatment of a disease characterized by overexpression of fibroblast activation protein (FAP) in an animal or a human subject. Preferably, the disease characterized by overexpression of fibroblast activation protein (FAP) is selected from the group consisting of cancer, chronic inflammation, atherosclerosis, fibrosis, tissue remodeling and keloid disorder.

Preferably, if the disease characterized by overexpression of fibroblast activation protein (FAP) is cancer, the cancer is selected from the group consisting of breast cancer, pancreatic cancer, small intestine cancer, colon cancer, rectal cancer, lung cancer, head and neck cancer, ovarian cancer, hepatocellular carcinoma, esophageal cancer, hypopharynx cancer, nasopharynx cancer, larynx cancer, myeloma cells, bladder cancer, cholangiocellular carcinoma, clear cell renal carcinoma, neuroendocrine tumor, oncogenic osteomalacia, sarcoma, CUP (carcinoma of unknown primary), thymus carcinoma, desmoid tumors, glioma, astrocytoma, cervix carcinoma and prostate cancer. Preferably, the cancer is glioma, breast cancer, colon cancer, lung cancer, head and neck cancer, liver cancer or pancreatic cancer. More preferably, the cancer is glioma.

Preferably, if the disease characterized by overexpression of fibroblast activation protein (FAP) is chronic inflammation, the chronic inflammation is selected from the group consisting of rheumatoid arthritis, osteoarthritis and Crohn's disease. Preferably, the chronic inflammation is rheumatoid arthritis.

Preferably, if the disease characterized by overexpression of fibroblast activation protein (FAP) is fibrosis, the fibrosis is selected from the group consisting of pulmonary fibrosis, such as idiopathic pulmonary fibrosis and liver cirrhosis.

Preferably, if the disease characterized by overexpression of fibroblast activation protein (FAP) is tissue remodeling, the tissue remodeling occurs after myocardial infarction.

Preferably, if the disease characterized by overexpression of fibroblast activation protein (FAP) is a keloid disorder, the keloid disorder is selected from the group consisting of scar formation, keloid tumors and keloid scar.

In a fourth aspect, the present invention relates to a kit comprising or consisting of the compound of the first aspect or the pharmaceutical composition of the second aspect and instructions for the diagnosis or treatment of a disease. Preferably, the disease is a disease as specified above.

### Examples

### Example 1: Compound synthesis and radiochemistry

Based on a FAP-α specific inhibitor (Jansen et al., *ACS Med Chem Lett,* 2013) two radiotracers were synthesized. Radioiodine labeled FAPI-01 was obtained via an organotin stannylated precursor, which was prepared through palladium catalyzed bromine/tin exchange. FAPI-02 is a precursor for the chelation of radio metals which was synthesized in five steps. By application of the same or slightly modified procedures additional compounds were prepared. The structures of these compounds are listed in table 1 and 2. Radioiodinations of the stannylated precursor were performed with peracetic acid. For chelation with Lu-177 and Ga-68 the pH of the reaction mixture was adjusted with sodium acetate and heated to 95 °C for 10 min. Stability in human serum was analyzed by precipitation and radio-HPLC analysis of the supernatant.

### Reagents

All solvents and non-radioactive reagents were obtained in reagent grade from ABCR (Karlsruhe, Germany), Sigma-Aldrich (München, Germany), Acros Organics (Geel, Belgium) or VWR (Bruchsal, Germany) and were used without further purification. Atto 488 NHS-ester was obtained from AttoTec (Siegen, Germany). 2,2',2"-(10-(2-(4-nitrophenyl)oxy)-2-oxoethyl)-1,4,7,10-tetraazacyclo-dodecane-1,4,7triyl)triacetic acid (DOTA-PNP) was synthesized following the protocol of Mier et al. (Mier et al., Bioconjug Chem, 2005). The intermediates 6-methoxyquinoline-4-carboxylic acid (7), 5-bromoquinoline-4-carboxylic acid (**3**) and (S)-1-(2-aminoacetyl)pyrrolidine-2-carbonitrile 4-methylbenzenesulfonate were synthesized following the protocols of Jansen et al. (Jansen et al., *ACS Med Chem Lett,* 2013)*.* The substance (*S*)-*N*-(2-(2-cyanopyrrolidin-1-yl)-2-oxoethyl)-5-bromoquinoline carboxamide was synthesized by a modified HBTU amidation protocol.

### Compound synthesis

Scheme 1 depicts the initial synthesis of FAPI-01 which was achieved by performing a Br/Li-exchange with n-butyllithium at 5-bromoquinolie-4-carboxylic acid (**3**) and quenching with elemental iodine to obtain iodoquinoline 4. This compound was coupled to the Gly-Pro-CN fragment by HBTU/HOBt-activation to provide non-radioactive reference material of FAPI-01 (1).

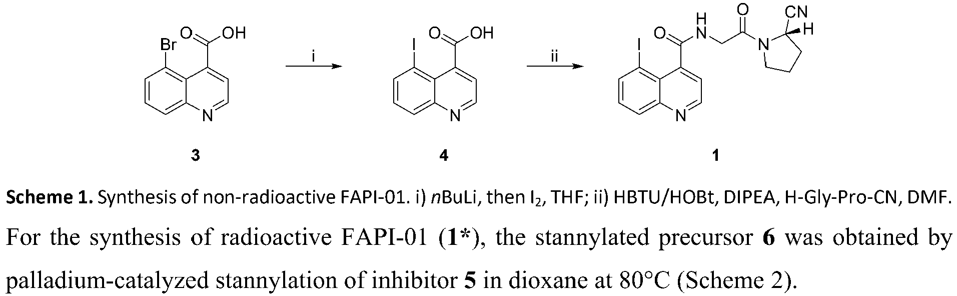

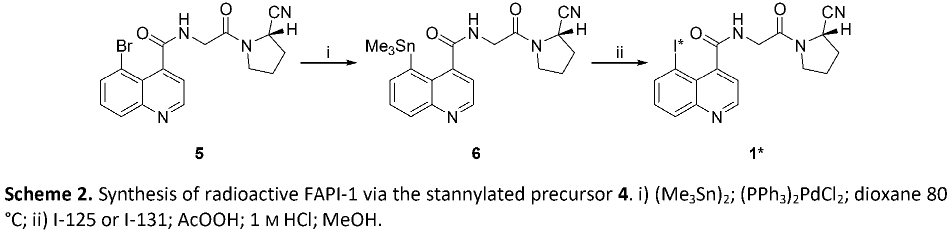

To enable radiolabeling by incorporation of radiometals, the chelator DOTA was chemically linked to the basic scaffold of the FAP-inhibitor. As shown by Jansen et al. (Jansen et al., ACS Med Chem Lett, 2013), modifications at the 6-position of the quinoline-4-carboxylic acid are well tolerated without impairing target affinity and specificity. Therefore, a bifunctional linker was attached to the hydroxyl group of **8** via an ether linkage, leading way to the synthesis shown in Scheme 3. Ready available 1-bromo-3-chloropropane was chosen to create a spacer, which is unharmed during the saponification of the simultaneously formed ester bond at the end of the one-pot-process. Compound **9** was converted to the *N*-Boc protected quinolinecarboxylic acid **10** which was further coupled to H-Gly-Pro-CN by HBTU. Due to the high hygroscopicity of the free amine, compound **11** was directly converted to FAPI-02 (**2**) after the Boc-removal, solvent exchange and neutralization of excess *p*-toluenesulfonic acid.

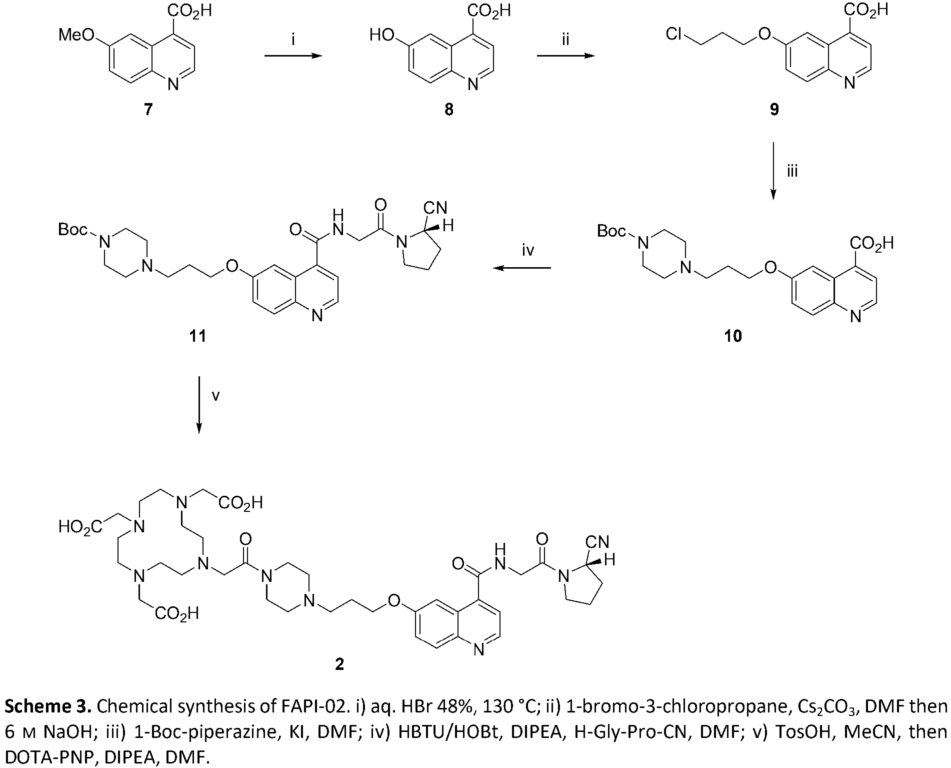

In case of compounds incorporating group A ≠ O, the quinoline-4-carboxylic acid intermediates were synthesized by a different reaction scheme. The key step of this approach is a palladium catalyzed coupling reaction (e.g. Buchwald-Hartwig cross-coupling), which requires additional protection before and deprotection of the carboxylic acid function after the cross-coupling reaction (scheme 4).

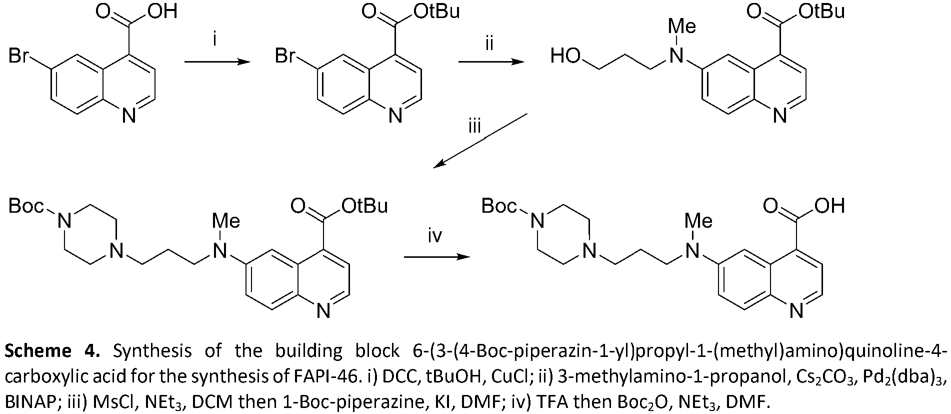

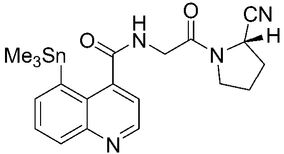

(*S*)-*N*-(2-(2-cyanopyrrolidin-1-yl)-2-oxoethyl)-5-trimethylstannylquinoline caboxamide (**6**) 3.88 mg (10.0 µmol) (*S*)-*N*-(2-(2-cyanopyrrolidin-1-yl)-2-oxoethyl)-5-bromoquinoline caboxamide, 20 µL (32 mg; 96 µmol) hexamethylditin and 0.75 mg (1.07 µmol) bis(triphenylphosphine)palladium(II) dichloride in 1 mL dry dioxane are stirred at 80 °C over night under an inert atmosphere. Volatiles are removed and the residue is taken up in 2 mL 50% acetonitrile/water and filtered through a C18-light cartridge before HPLC-purification. 2.78 mg (5.90 µmol; 59%) of the product are obtained after freeze drying.

**LC-MS** Rₜ 14.77 min, m/z 473.0786 [M(¹²⁰Sn)+H]⁺

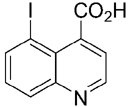

### 5-iodoquinolie-4-carboxylic acid (4)

5.42 mg (136 µmol) of sodium hydride suspension (60% in mineral oil) are added to an solution of 30.27 mg (120 µmol) 5-bromoquinolie-4-carboxylic acid (**3**) in 3 mL dry THF under Ar at 0°C. The ice bath is removed and the reaction mixture is cooled to -78 °C before 100 µL (160 µmol) nBuLi (1.6 m in hexanes) are added dropwise. After 15 min 64.71 mg (254 µmol) iodine in 2 mL THF are added dropwise and the reaction is stirred for 30 min at -78 °C before allowed to reach room temperature. After 1 h the reaction is quenched by addition of 1 mL 0.5 M NaHCO₃ and ca. 30 mg (170 µmol) sodium dithionite to remove excessive iodine. After the removal of THF under reduced pressure the mixture is acidified to pH 2 and extracted three times with ethyl acetate (25 mL). The combined organic phases are evaporated to dryness and purified by HPLC. 18.14 mg (60.7 µmol; 45%) of the title compound are obtained after freeze drying.

**¹H NMR** (500 MHz, DMSO-d6) 13.95 (br, 0.3H), 8.93 (s, 1H), 8.34 (d, J =7.2 Hz, 1H), 8.12 (d, J = 8.4 Hz, 1H), 7.60 (s, 1H), 7.52 (t, J = 7.9 Hz, 1H); **¹³C NMR** (125 MHz, DMSO-d6) 168.8, 150.3, 148.8, 141.3, 130.6, 121.0, 109.5; **LC-MS** Rₜ 8.65 min, m/z 299.9383 [M+H]⁺

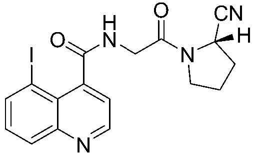

### (S)-N-(2-(2-cyanopyrrolidin-1-yl)-2-oxoethyl)-5-trimethylstannylquinoline caboxamide (1; FAPI-01)

9.07 mg (23.9 µmol) HBTU in 50 µL DMF are added to a solution of 6.21 mg (20.8 µmol) 5-iodoquinoline-4-carboxylic acid, 7.45 mg (55.2 µmol) HOBt and 10 µL DIPEA in 50 µL DMF. After 15 min (29.9 µmol) (S)-1-(2-aminoacetyl)pyrrolidine-2-carbonitrile 4-methylbenzenesulfonate in 50 µL DMF are added. The reaction is quenched with 850 µL water and purified by HPLC. Freeze drying provides 6.86 mg (15.8 µmol; 76%) of the product.

**¹H NMR** (600 MHz, DMSO-d6) 9.06, 8.97, 8.33, 8.13, 7.56, 7.51, 4.81, 4.34, 4.06, 3.74, 3.56, 2.21, 2.17, 2.09, 2.05; **¹³C NMR** (150 MHz, DMSO-d6) 167.1, 150.2, 148.8, 145.3, 141.5, 130.7, 125.3, 121.9, 119.3, 92.0, 46.3, 45.4, 42.1, 29.5, 24.9; **LC-MS** Rₜ 11.95 min, m/z 435.0102 [M+H]⁺

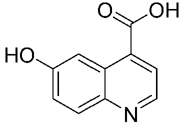

### 6-Hydroxyquinoline-4-carboxylic acid (8)

105 mg (477 µmol) of raw 6-methoxyquinoline-4-carboxylic acid (**7**) are dissolved in 3 mL of 48% hydrobromic acid in water. The solution is heated to 130 °C for 4 h. The solution is brought to a slightly basic pH with 6 M NaOH after reaching room temperature. 79.2 mg (419 µmol; 88%) of the product are obtained after by HPLC-purification and lyophilization.

**¹H NMR** (500 MHz, DMSO-d6) 13.65 (br, 0.6H) 10.24 (s, 1H), 8.78 (d, J = 4.4 Hz, 1H), 8.06 (d, J = 2.6 Hz, 1H), 7.95 (d, J = 9.1 Hz, 1H), 7.84 (d, J = 4.4 Hz, 1H), 7.37 (dd, J = 9.1, 2.6 Hz, 1H), **¹³C NMR** (125 MHz, DMSO-d6) 167.7, 156.9, 146.5, 144.1, 133.4, 131.2, 126.2, 122.3, 122.6, 106.5; **LC-MS** Rₜ 6.66 min, m/z 190.0415 [M+H]⁺

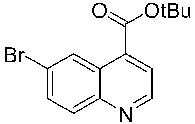

### tert-butyl 6-bromoquinoline-4-carboxylate

98.3 mg (390 µmol) 6-bromoquinolie-4-carboxylic acid (raw) were suspended in 5 mL tetrahydrofuran and 25.0 µL (18.3 mg;181 µmol) triethylamine and added to O-*tert*-butyl-N,N'-dicyclohexylisourea (prepared the day before from neat 426 mg (2.07 mmol) dicyclohexylcarbodiimide, 173 mg (2.33 mmol) *tert*-butanol and 10.2 mg (103 µmol) copper(I)chloride). The mixture was heated to 50 °C over night. The mixture was filtered, solvents evaporated and the product isolated by HPLC. 49.7 mg (161 µmol; 41%) of the title compound were obtained after freeze drying.

**LC-MS** Rₜ 20.40 min, m/z 251.9642 [M-*t*Bu]⁺

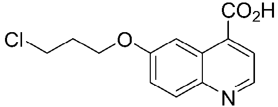

### 6-(3-chloro-1-propoxy)quinoline-4-carboxylic acid (9)

42.4 µL (67.4 mg; 430 µmol) 1-bromo-1-chloropropane are added to a suspension of 23.2 mg (123 µmol) 6-hydroxyquinoline-4-carboxylic acid (**8**) and 190 mg (1.38 µmol) potassium carbonate in 250 µL DMF and heated to 60 °C over night. The reaction mixture is cooled to room temperature, diluted with 500 µL water and 500 µL acetonitrile before 100 µL 6 M NaOH are added. The reaction mixture is directly purified via HPLC (5-40%) after the complete ester hydrolysis is accomplished. 26.45 mg (99.4µmol; 81%) of the product are obtained after lyophilization.

**¹H NMR** (500 MHz, DMSO-d6) 13.75 (br, 0.4H), 8.88 (d, J = 4.4 Hz, 1H), 8.19 (d, J = 2.0 Hz, 1H), 8.04 (d, J = 9.2 Hz, 1H), 7.94 (d, J = 4.4 Hz, 1H), 7.52 (dd, J = 9.2, 2.0 Hz, 1H), 4.24 (t, J = 5.95 Hz, 2H), 3.85 (t, J = 6.5 Hz, 2H), 2.27 (m, 2H); **¹³C NMR** (125 MHz, DMSO-d6) 167.6, 157.5, 147.6, 144.8, 134.0, 131.2, 125.9, 122.7, 122.2, 104.5, 64.7, 41.9, 31.6; **LC-MS** Rₜ 11.46 min, m/z 266.0461 [M+H]⁺

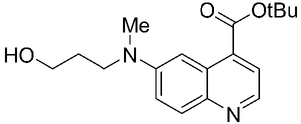

### tert-butyl 6-(3-hydroxypropylmethylamino)quinoline-4-carboxylate

204.6 mg (664 µmol) *tert-*butyl 6-bromoquinoline-4-carboxylate, 34.10 mg (54.7 µmol) BINAP, 21.51 mg (23.5 µmol) Pd₂(dba)₃ and 480.3 mg (1.47 mmol) cesium carbonate were dissolved in 6 mL toluene and 128.0 µL (118 mg; 1.32 mmol) N-methyl-1,3-propanolamine were added. The mixture was stirred at 90 °C over night before solvents were removed, the residue suspended in water/acetonitrile 1:1 and filtered before HPLC-purification. 172.7 mg (547 µmol; 82%) of the title compound were obtained after freeze drying.

**LC-MS** Rₜ 13.41 min, m/z 261.1213 [M-*t*Bu+H]⁺

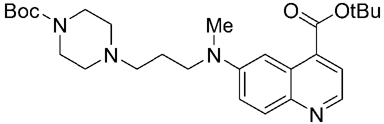

### tert-butyl 6-(3-(4-Boc-piperazin-1-yl)propyl-1-(methyl)amino)quinoline-4-carboxylate

62.8 mg (199 µmol) *tert*-butyl 6-(3-hydroxypropylmethylamino)quinoline-4-carboxylate were dissolved in 5 mL dichloromethane and 90.0 µL (66.6 mg; 659 µmol) triethylamine. 20.0 µL (29.6 mg; 258 µmol) methanesulfonyl chloride were added at 0 °C and the mixture reacted for 60 min. 194.6 mg (1.05 mmol) 1-Boc-piperazine were added before volatiles were removed. 500 µL dimethylformamide and 47.4 mg (286 µmol) potassium iodide were added to the residue. The mixture was shaken at 60 °C for 120 minutes before the product was isolated by HPLC. 81.05 mg (167 µmol; 84%) of the title compound were obtained after freeze drying.

**LC-MS** Rₜ 13.99 min, m/z 485.3086 [M+H]⁺

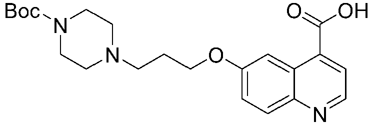

### 6-(3-(4-tert-butoxycarbonylpiperazin-1-yl)-1-propoxy)quinoline-4-carboxylic acid (10)

15.13 mg (56.9 µmol) of 6-(3-chloro-1-propoxy)quinoline-4-carboxylic acid (**9**), 55.43 mg (298 µmol) *N-tert*-butoxycarbonylpiperazine and 51.05 mg (30.8 µmol) potassium iodide are dissolved in 250 µL DMF. The reaction is shaken at 60 °C over night. The resulting suspension is diluted with 750 µL water before the product is purified by HPLC. After freeze drying 28.73 mg (54.3 µmol; 95%) of the product are obtained as the corresponding TFA-salt.

**¹H NMR** (500 MHz, D₂O) 8.93 (d, J = 5.5 Hz, 1H), 8.17 (d, J = 9.3 Hz, 1H), 7.94 (d, J = 5.5 Hz, 1H), 7.79 (dd, J = 9.3, 2,5 Hz, 1H), 7.65 (d, J = 2.5 Hz, 1H), 4.36 (t, J = 5.6 Hz, 2H), 4.27 (d, J = 13.55 Hz, 2H), 3.67 (d, J = 11.95 Hz), 3.47 (t, J = 15.5 Hz, 2 H), 3.27 (t, J = 12.7 Hz), 3.12 (td, J = 12.2, 2.65 Hz), 2.37 (m2 H), 1.47 (s, 9H); **¹³C NMR** (125 MHz, D₂O) 155.5, 153.5, 149.0, 141.4, 134.4, 127.9, 126.6, 122.3, 118.4, 110.0, 105.1, 82.8, 65.5, 54.3, 51.5, 48.6, 40.7, 29.6, 27.4; **LC-MS** Rₜ 10.62 min, m/z 416.1997 [M+H]⁺

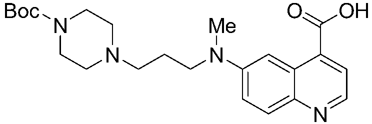

### 6-(3-(4-Boc-piperazin-1-yl)propyl-1-(methyl)amino)quinoline-4-carboxylic acid

100.12 mg (206 µmol) *tert-*butyl 6-(3-(4-Boc-piperazin-1-yl)propyl-1-(methyl)amino)quinoline-4-carboxylate were treated with 900 µL trifluoroacetic acid, 25 µL triisopropylsilane, 25 µL water and 50 µL trifluoromethanesulfonic acid for 60 min. The deprotected compound was precipitated with diethyl ether, dried and reacted with 60.83 mg (279 µmol) di-*tert*-butyldicarbonate and 50.0 µL (36.5 mg; 361 µmol) triethylamine in 1 mL dimethylformamide for another 60 min. 55.42 mg (129 µmol; 65% over 2 steps) were obtained after HPLC-purification and freeze-drying.

**LC-MS** Rₜ 10.52 min, m/z 429.2463 [M+H]⁺

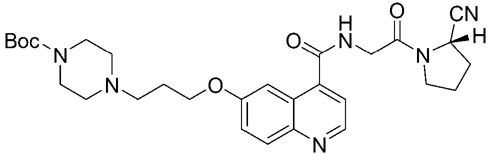

### (S)-N-(2-(2-cyanopyrrolidin-1-yl)-2-oxoethyl)-6-(3-(4-tert-butoxycarbonylpiperazin-1-yl)-1-propoxy)quinoline-4-carboxamide (11)

9.43 mg (24.9 µmol) HBTU in 50 µL DMF are added to a solution of 10.56 mg (19.9 µmol) 6-(3-(4-*tert*-butoxycarbonylpiperazin-1-yl)-1-propoxy)quinoline-4-carboxylic acid (**10**), 5.38 mg (39.8 µmol) HOBt and 10 µL DIPEA in 50 µL DMF. After 15 min (29.9 µmol) (S)-1-(2-aminoacetyl)pyrrolidine-2-carbonitrile 4-methylbenzenesulfonate in 50 µL DMF are added. The reaction is quenched with 850 µL water and purified by HPLC. Freeze drying provides 12.88 mg (19.4 µmol; 97%) of the title compound.

**¹H NMR** (500 MHz, DMSO-d6) 9.04 (d, J = 5.5 Hz, 1H), 8.24 (d, J = 9.6 Hz, 1H), 8.10 (d, J = 5.5 Hz, 1H), 7.89 (d, J = 2.3 Hz, 1H), 7.85 (dd, J = 9.6, 2.3 Hz, 1H), 4.84 (t, J = 6 Hz, 1 H), 4.46-4.36 (m, 4H), 4.26 (d, J = 12.0 Hz, 2H), 3.83 (m, 1H), 3.67 (m, 3H), 3.47 (t, J = 7.7 Hz, 2H), 3.27 (br, 2H), 3.11 (t, J = 11.5 Hz), 2.37 (m, 4H), 2.22 (m, 2H), 1.46 (s, 9H); **¹³C NMR** (125 MHz, DMSO-d6) 168.6, 168.0, 159.4, 155.5, 147.7, 141.8, 135.1, 128.2, 127.5, 123.1, 120.0, 119.1, 104.7, 82.9, 66.0, 54.3, 51.5, 47.0, 46.3, 42.3, 29.4, 27.4, 24.7, 23.1; **LC-MS** Rₜ 11.81 min, m/z 551.2736 [M+H]⁺

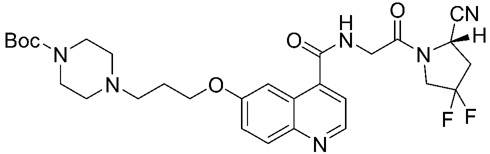

### (S)-N-(2-(2-cyano-4,4-difluoropyrrolidin-1-yl)-2-oxoethyl)-6-(3-(4-tert-butoxycarbonyl-piperazin-1-yl)-1-propoxy)quinoline-4-carboxamide

13.2 mg (22.4 µmol; 75%) were obtained following the previous protocol.

**LC-MS** Rₜ 11.84 min, m/z 605.2610 [M+H]⁺

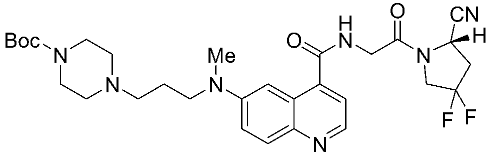

### N-(2-(2-cyano-4,4-difluoropyrrolidin-1-yl)-2-oxoethyl)-6-(3-(4-Boc-piperazin-1-yl)propyl-1-(methyl)amino)quinoline-4-carboxamide

1.17 mg (1.95 µmol; 92%) were obtained following the previous protocol.

**LC-MS** Rₜ 12.66 min, m/z 600.3057 [M+H]⁺ 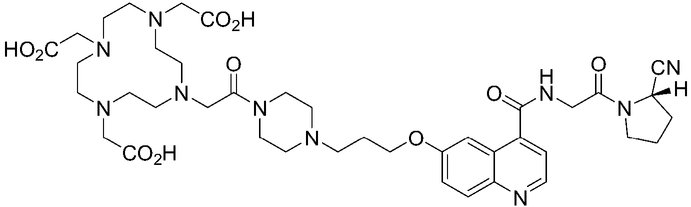 FAPI-02 (**2**)

4.85 mg (8.80 mmol) (S)-N-(2-(2-cyanopyrrolidin-1-yl)-2-oxoethyl)-6-(3-(4-tert-butoxycarbonyl-piperazin-1-yl)-1-propoxy)quinoline-4-carboxamide (**11**) are dissolved in 1 mL acetonitrile and 4.2 mg (22.0 µmol) 4-methylbenzenesulfonic acid monohydrate are added. The reaction is shaken at 45 °C over night, bevore volatiles are removed under reduced pressure. The residue is taken up in 190 µL dimethylformamide and 10 µL (7.3 mg; 72 µmol) triethylamine before 6.77 mg (12.9 mmol) of DOTA-p-nitrophenol ester are added. The reaction mixture is diluted with 1 mL water and purified by HPLC after shaking for two hours. 5.04 mg (6.02 µmol; 68%) are obtained after freeze drying.

**¹H NMR** (600 MHz, D₂O) 9.02, 8.23, 8.07, 7.87, 7.83, 4.85, 4.45, 4.41, 4.40, 4.39, 3.83, 3.67, 3.50, 3.49, 2.40, 2.38, 2.36, 2.26, 2.22, 2.16; **¹³C NMR** (150 MHz, D₂O) 167.9, 159.1, 147.2, 141.8, 135.4, 127.9, 127.2, 119.8, 119.0, 104.5, 65.8, 54.1, 46.8, 46.1, 42.1, 29.2, 24.5, 23.0: LC-MS Rₜ 8.37 min, m/z 837.3872 [M+H]⁺ 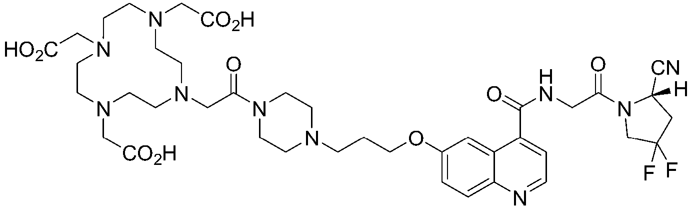 FAPI-04

3.97 mg (4.55 µmol; 57%) were obtained following the previous protocol.

**LC-MS** Rₜ 8.80 min, m/z 873.3664 [M+H]⁺ 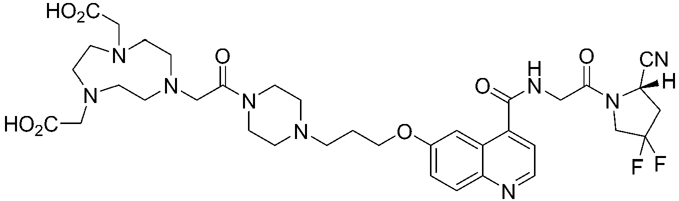 FAPI-42

1.91 mg (2.47 µmol; 88%) were obtained following the previous protocol.

**LC-MS** Rₜ 9.37 min, m/z 386.6807 [M+2H]²⁺ 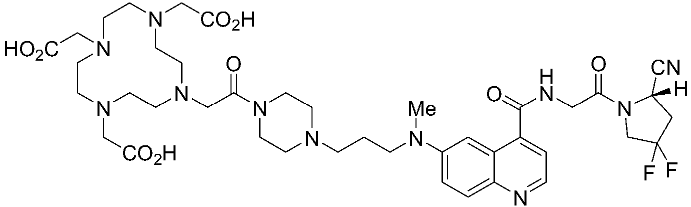 FAPI-46

39.21 mg (44.3 µmol; 85%) were obtained following the previous protocol.

**LC-MS** Rₜ 9.03 min, m/z 443.7196 [M+2H]²⁺ 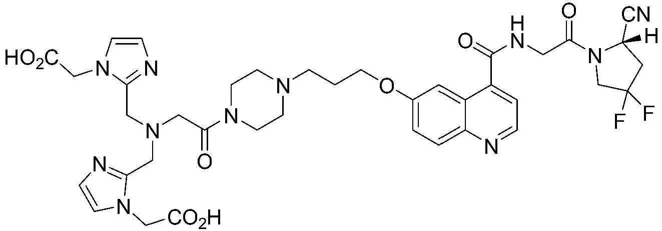 FAPI-19

1.09 mg (1.86 µmol) of (*S*)-*N*-(2-(2-cyano-4,4-difluoropyrrolidin-1-yl)-2-oxoethyl)-6-(3-(4-*tert*-butoxycarbonylpiperazin-1-yl)-1-propoxy)quinoline-4-carboxamide were Boc-deprotected by the method applied for FAPI-02 and reacted with 2.74 mg (5.91 µmol) bis((1-(2-(tert-butoxy)-2-oxoethyl)-1H-imidazol-2-yl)methyl)glycine, which were preactivated with 2.13 mg (5.62 µmol) HBTU and 2.50 µL (1.85 mg; 14.3 µmol) DIPEA. After HPLC purification and solvent removal the residue was treated with 200 µL of 2.5% trifluoromethanesulfonic acid in acetonitrile/trifluoroacetic acid 1:1. After precipitation with diethyl ether and HPLC purification 1.06 mg (1.29 µmol; 70%) of the title compound were obtained.

**LC-MS** Rₜ 8.91 min, m/z 820.2933 [M+H]⁺ 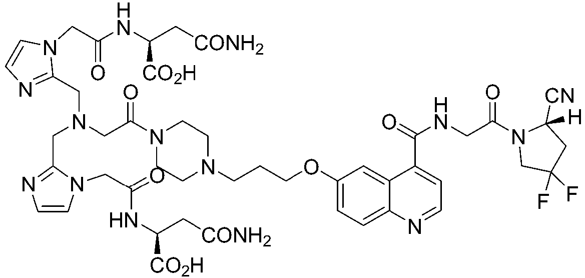 FAPI-28

1.00 µL (0.74 mg; 5.73 µmol) DIPEA was added to a solution of 0.95 mg (1.16 µmol) FAPI-19, 0.42 mg (3.14 µmol) HOBt and 1.10 mg (2.89 µmol) HBTU in 50 µL DMF. After 10 min 2.30 mg (5.34 µmol) H-Asn(Trt)-OtBu were added and reacted for 120 min. The *tert-*butyl protecting groups were removed by 2.5% TfOH in TFA/acetonitrile 8:2. After HPLC-purification and freeze-drying. 0.79 mg (0.75 µmol; 65%) of the title compound were obtained. **LC-MS** Rₜ 9.23 min, m/z 524.7100 [M+2H]²⁺ 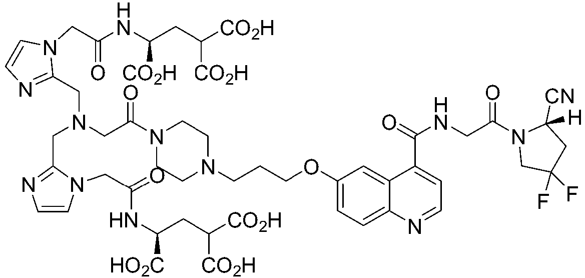 FAPI-34

1.01 mg (0.87 µmol; 52%) were obtained following the previous protocol.

**LC-MS** Rₜ 8.87 min, m/z 583.6988 [M+2H]²⁺ 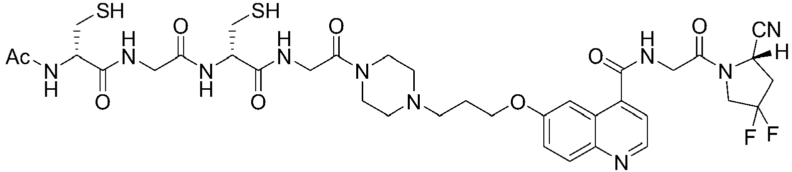 FAPI-60

3.91 mg (6.66 µmol) of (*S*)-*N*-(2-(2-cyano-4,4-difluoropyrrolidin-1-yl)-2-oxoethyl)-6-(3-(4-*tert*-butoxycarbonylpiperazin-1-yl)-1-propoxy)quinoline-4-carboxamide were deprotected for 30 min by 50 µL acetonitrile and 100 µL trifluoroacetic acid. After evaporation of the solvents and washing with diethyl ether a 10 min preincubated mixture of 8.02 mg (9.27 µmol) acetyl-Cys(Trt)-Gly-Cys(Trt)-Gly-OH, 4.31 mg (31.9 µmol) HOBt and 4.47 mg (11.8 µmol) HBTU in 150 µL dimethylformamide and 2.50 µL (1.85 mg; 14.3 µmol) DIPEA was added to the residue and reacted for 120 min. 4.66 mg (3.49 µmol; 52%) of the S-trityl protected title compound were obtained after HPLC-purification and freeze-drying.

3.36 mg (2.52 µmol) of the trityl protected compound were dissolved in 50 µL acetonitrile. 3 µL Triethylsilane were and 100 µL trifluoroacetic acid were added and reacted for 30 min. 2.01 mg (2.36 µmol; 94%; 49% over two steps) of the title compound were obtained after HPLC purification and freeze-drying.

**LC-MS** Rₜ 10.26 min, m/z 871.2703 [M+Na]⁺ 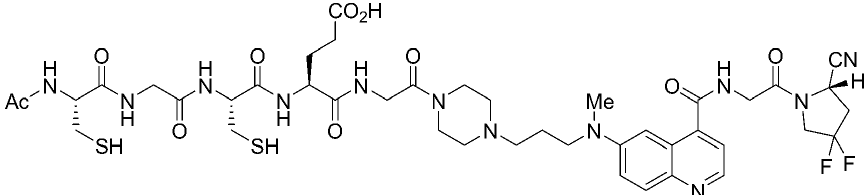 FAPI-69

0.59 mg (0.60 µmol; 39%) were obtained following the previous protocol.

**LC-MS** Rₜ 10.25 min, m/z 991.3490 [M+H] ⁺ 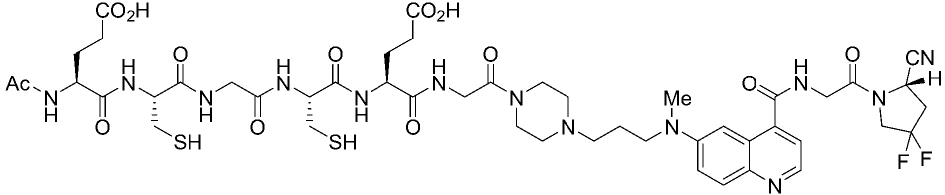 FAPI-70

0.61 mg (0.54 µmol; 33%) were obtained following the previous protocol.

**LC-MS** Rₜ 10.14 min, m/z 1120.3884 [M+H] ⁺ 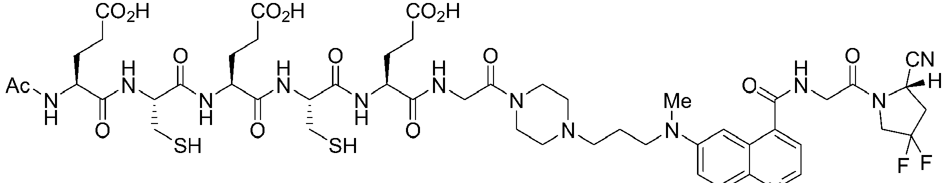 FAPI-71

0.79 mg (0.66 µmol; 34%) were obtained following the previous protocol.

**LC-MS** Rₜ 10.17 min, m/z 596.7075 [M+2H]²⁺ 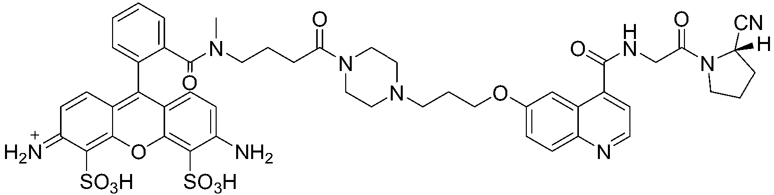 Atto488-FAPI-02 (14)

0.66 mg (1.20 µmol) of **11** are treated with 1.33 mg (6.96 µmol) 4-methylbenzenesulfonic acid monohydrate in 250 µL acetonitrile at 45 °C for 4 hours. After removal of the solvent the residue is dissolved in 95 µL dimethylformamide and 5 µL (3.65 mg; 36.1 µmol) triethylamine. 0.54 mg (0.55 µmol) Atto 488 NHS-ester in 25 µL DMSO were added. After 60 minutes 0.49 mg (0.43 µmol; 78%) of the title compound were isolated by HPLC and freeze drying.

**LC-MS** Rₜ 10.19 min, m/z 1022.2706 [M]⁺ 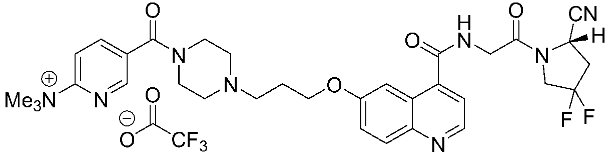 FAPI-73

10.95 mg (18.7 µmol) of (S)-N-(2-(2-cyano-4,4-difluoropyrrolidin-1-yl)-2-oxoethyl)-6-(3-(4-*tert*-butoxycarbonylpiperazin-1-yl)-1-propoxy)quinoline-4-carboxamide were deprotected for 30 min by 100 µL acetonitrile and 200 µL trifluoroacetic acid. After evaporation of the solvents and washing with diethyl ether 15.02 mg (9.27 µmol) N,N,N-trimethyl-5-((2,3,5,6-tetrafluorophenoxy)-carbonyl)pyridine-2-aminium chloride was added and the mixture dissolved in 200 µL dimethylformamide and 10.0 µL (7.30 mg; 72.3 µmol) triethylamine. After 120 min the mixture was purified by HPLC and 11.24 mg (14.7 µmol; 79%) of the title compound were obtained freeze-drying.

**LC-MS** Rₜ 9.37 min, m/z 649.2892 [M-CF₃CO₂]⁺ 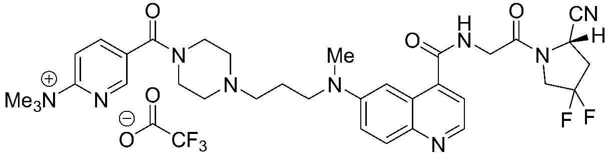 FAPI-72

9.80 mg (12.6 µmol; 70%) were obtained following the previous protocol.

**LC-MS** Rₜ 9.28 min, m/z 662.3237 [M-CF₃CO₂]⁺

### General Attachment of side chain protected Fmoc-amino acids

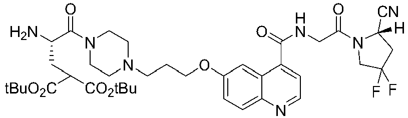

### (S)-N-(2-(2-cyano-4,4-difluoropyrrolidin-1-yl)-2-oxoethyl)-6-(3-(4-(γ,γ-di-tert-butyl)-L-carboxy-glutamylpiperazin-1-yl)-1-propoxy)quinoline-4-carboxamide

14.04 mg (23.9 µmol) of (*S*)-*N*-(2-(2-cyano-4,4-difluoropyrrolidin-1-yl)-2-oxoethyl)-6-(3-(1-*tert*-butoxycarbonyl-piperidin-4-yl)-1-propoxy)quinoline-4-carboxamide were dissolved in 50 µL acetonitrile and 100 µL trifluoroacetic acid. After 10 min the volatiles were removed; the residue was washed with diethyl ether. A solution of 14.95 mg (28.4 µmol) Fmoc-L-Gla(tBu)₂-OH, 7.74 mg (57.4 µmol) HOBt, 13.46 mg (35.5 µmol) HBTU and 20.0 µL (14.8 mg; 115 µmol) DIPEA in 200 µL dimethylformamide was added to the dried residue. After 60 min 50.0 µL (50.4 mg; 578 µmol) morpholine were added and the product was isolated by HPLC after 30 min. 15.95 mg (20.7 µmol; 86%) of the title compound were obtained after freeze drying.

**LC-MS** Rₜ 12.85 min, m/z 772.3643 [M+H]⁺ 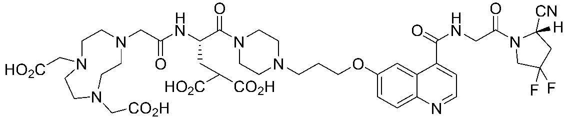 FAPI-75

3.37 mg (4.37 µmol) of (*S*)-*N*-(2-(2-cyano-4,4-difluoropyrrolidin-1-yl)-2-oxoethyl)-6-(3-(4-(γ,γ-di-*tert*-butyl)-L-carboxyglutamylpiperazin-1-yl)-1-propoxy)quinoline-4-carboxamide were and 4.52 mg (10.7 µmol) NOTA-*p*-nitrophenol were dissolved in 100 µL dimethylformamide and 10.0 µL (7.30 mg; 72.3 µmol) triethylamine. After HPLC-purification and freeze-drying the intermediate compound was deprotected by a 60 min incubation in a solution of 50 µL acetonitrile, 100 µL trifluoroacetic acid, 2.5 µL triisopropylsilane and 2.5 µL water. 2.62 mg (2.77 µmol; 63%) were obtained after HPLC-purification and freeze-drying.

**LC-MS** Rₜ 9.38 min, m/z 945.3668 [M+H]⁺ 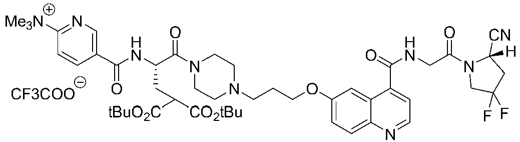 FAPI-77-precursor

3.23 mg (3.06 µmol; 73%) were obtained following the general active ester modification protocol. Note: The tert-butyl protecting groups were removed after radio fluorination, HPLC-purification and evaporation of solvents by treatment with neat TFA at 95 °C for 3 min followed by SPE work up.

**LC-MS** Rₜ 16.02 min, m/z 1219.5858 [M+H]⁺

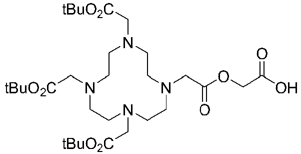

### 2-(2-(4,7,10-tris(2-(tert-butoxy)-2-oxoethyl)-1,4,7,10-tetraazacyclododecan-1-yl)acetoxy)acetic acid

28.99 mg (50.6 µmol) tris-tBu-DOTA, 90.65 (278 µmol) cesium carbonate and 10.28 µL (15.0 mg; 65.5 µmol) benzyl 2-bromoacetate were suspended in 300 µL dimethylformamide and shaken for 2 h. The product was isolated by HPLC, freeze dried and dissolved in 25 ml 10% acetic acid in methanol. 50 mg 10% Pd/C and hydrogen (ambient pressure) were added. After 2 hours. Solvents wer removed and the title compound isolated by HPLC. After freeze drying 25.19 mg (39.9 µmol; 79%) of the title compound were obtained.

**LC-MS** Rₜ 14.14 min, m/z 631.4784 [M+H]⁺ 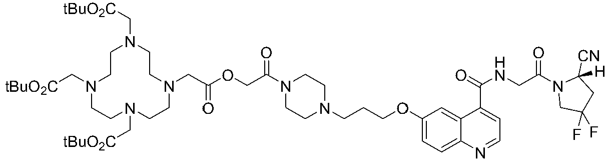 tBu-FAPI-79

2.00 mg (3.41 µmol) (*S*)-*N*-(2-(2-cyano-4,4-difluoropyrrolidin-1-yl)-2-oxoethyl)-6-(3-(1-*tert-*butoxycarbonyl-piperidin-4-yl)-1-propoxy)quinoline-4-carboxamide were dissolved in 50 µL acetonitrile and 100 µL trifluoroacetic acid. After 10 min the volatiles were removed; the residue was washed with diethyl ether. 4.20 mg (6.60 µmol) 2-(2-(4,7,10-tris(2-(*tert-*butoxy)-2-oxoethyl)-1,4,7,10-tetraazacyclododecan-1-yl)acetoxy)acetic acid and 3.35 mg (8.84 µmol) HBTU dissolved in 100 µL dimethylformamide and 10.0 µL (7.40 mg; 57.4 µmol) DIPEA were added to the dried residue and reacted for 60 min. 2.26 mg (2.06 µmol; 60%) of the title compound were obtained after HPLC purification and freeze drying.

**LC-MS** Rₜ 12.98 min, m/z 1099.7481 [M+H]⁺ 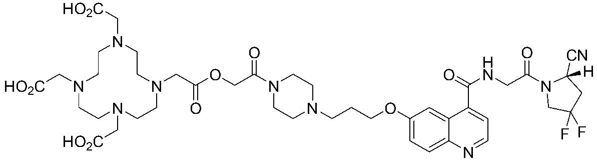 FAPI-79

2.26 mg (2.06 µmol) tBu-FAPI-79 were dissolved in 25 µL acetonitrile and 100 µL trifluoroacetic acid and shaken at 35 °C for 30 min. After evaporation of the solvents the product was isolated by HPLC. 1.58 mg (1.70 µmol; 82%) of the title compound were obtained after freeze drying.

**LC-MS** Rₜ 8.84 min, m/z 466.2737 [M+2H]²⁺

### Compound analysis

Reverse-phase high-performance liquid chromatography (RP-HPLC) was conducted using linear gradients of acetonitrile in water (0-100% acetonitrile in 5 min; 0.1% TFA; flowrate 2 mL/min) on a Chromolith Performance RP-18e column (100 × 3 mm; Merck KGaA Darmstadt, Germany). UV-absorbance was detected at 214 nm. An additional γ-detector was used for the HPLC-analysis of radioactive compounds. HPLC-MS characterization was performed on an ESI mass spectrometer (Exactive, Thermo Fisher Scientific, Waltham, MA, USA) connected to an Agilent 1200 HPLC system with a Hypersil Gold C18 1.9 µm column (200 × 2.1 mm; 0-100% acetonitrile in 20 min; flowrate 200 µL/min). Analytical Radio-HPLC was performed using a Chromolith Performance RP-18e column (100×3mm; Merck; 0-30% acetonitrile in 10 min; flowrate 2 mL/min). HPLC-purifications were performed on a LaPrep P110-System (Knauer, Berlin, Germany) and a Reprosil Pur 120 column (C18-aq 5 µm 250 × 25mm; Dr. Maisch, Ammerbuch-Entringen, Germany). The water/acetonitrile-gradient (15 or 25 min; 0.1% TFA; flowrate 20 mL/min) was modified for the individual products.

### Radiochemistry

Radioiodine (I-125) was purchased from Hartmann Analytik (Göttingen, Germany); radioactive lutetium (Lu-177) was obtained from ITG (München, Germany); radioactive gallium (Ga-68) was eluted from a Ge-68/Ga-68 generator purchased from Themba Labs (Somerset West, South Africa). Tc-99m was eluted from a Mo-99/Tc-99m generator (Curium Pharma, Berlin, Germany). Cu-64 was provided by UKT Tübingen (Tübingen, Germany). Sm-153 was provided by DSD Pharma (Purkersdorf, Austria). Pb-203 was provided by Lantheus (N. Billerica MA, USA). F-18-FDG and F-18-flouride were provided by the ZAG Zyklotron AG (Eggenstein, Germany). CRS Kit for tricarbonyl was obtained from Paul Scherrer Institut (Villingen-PSI, Switzerland).

For iodinations 10 µL of the organotin precursor of FAPI-01 (1 µmol/mL in ethanol) were diluted with 10 µL of 1 M HCl and 10 µL water before 1-20 MBq iodine-125 in 0.05 M NaOH were added. The reaction was started by addition of 5 µL of a fresh 1.9% solution of peracetic acid in glacial acetic acid. After 60 s 15 µL of 1 M NaOH were added and the reaction was quenched by addition of 5 µL of 5% ascorbic acid in water before HPLC purification. The obtained solution was directly used for *in vitro* experiments or evaporated to dryness under reduced pressure and taken up in 0.9% NaCl (Braun, Melsungen, Germany) in case of animal studies.

Cu-64, Lu-177 and Pb-203 labeling of DOTA-compounds was performed by addition of 5 MBq of the radionuclide to 100 µL of a 10 µM solution of the individual precursor in 0.1 M NaOAc (pH 5) and incubation at 95 °C for 10 min. The solution is directly used for *in vitro* experiments or diluted with 0.9% NaCl (Braun, Melsungen, Germany) in case of biodistribution studies. For imaging studies in mice (scintigraphy, PET) the radiotracer was worked up by solid phase extraction (sep-pak light C18, Waters).

Tc(I) labeling was preceded by addition of 1 mL of the Tc-99m-pertechnetate in 0.9% saline to a CRS Kit and incubation for 20 min. After cooling to room temperature a mixture of 25.0 µL of the precursor (1 mM in water), 150 µL phosphate buffer (0.4 M, pH 7.4) and 240 µL hydrochloric acid (1.0 M) was added and the final mixture adjusted to pH 5 if necessary. The reaction was performed at 95 °C for 20 minutes and worked up by solid phase extraction (sep-pak light C18, Waters). For in vivo experiments and animal studies the labeling was performed with one fifth of the reagents and 200 µL of the CRS Kit solution after Tc(VII) reduction.

Tc(V) labeling was preceded by incubation of 30 µL SnCl₂-solution containing 200 mM glucoheptonate with 200 µL Tc-99m-pertechnetate in 0.9% saline for 10 min at room temperature. 5.00 µL of the precursor (1 mM in water) and 3.75 µL sodium hydroxide solution (0.1 M in water) were added and the final mixture was reacted at 95 °C for 20 min. For imaging studies in mice (scintigraphy) the radiotracer was worked up by solid phase extraction (sep-pak light C18, Waters).

Labeling with Ga-68 for animal studies was performed by incubating 255 µL generator eluate (0.6 M HCl; approx. 230 MBq) with a mixture of 1 nmol DOTA-precursor, 1 µL of 20% ascorbic acid in water and 72 µL NaOAc (2.5 M) at 95 °C for 10 min. Remaining free radioactivity was removed by dilution with 2 mL water, solid phase extraction (sep-pak light C18, Waters), washing with 2 mL water and elution of the product with 1 mL water/ethanol 1:1. The obtained solution was evaporated to dryness under reduced pressure and the residue taken up in 0.9% NaCl (Braun).

For the formation of AlF-NOTA complexes F-18 fluoride was trapped on a waters Sep-Pak QMA plus light cartridge (46 mg sorbent; preconditioned with 0.5 M NaOAc, pH 3.9), washed with water and eluted with 500 µL 0.1 M NaOAc (pH 3.9). For animal studies 150 µL of the eluate were preincubated with 2 µL of an AlCl₃ solution (10 mM in water) and 50 µL DMSO. After 5 min the mixture was added to 40 nmol NOTA-precursor (10 µL of a 4 mM solution in water) and 1 µL of 20% ascorbic acid in water. The solution was reacted at 95 °C for 15 min. The product was isolated by HPLC (0-20% acetonitrile in 10 min), freed from solvents and taken up in 0.9% saline before injection.

For the formation of 6-fluoronicotinamides F-18 fluoride was trapped on a waters Sep-Pak QMA plus light cartridge (46 mg sorbent; preconditioned with 0.5 M KHCO₃), washed with water, dried and eluted with a mixture of 7.50 mg (19.9 µmol) cryptofix 222, 1.99 mg (1.99 µmol) KHCO₃ in 450 µL acetonitrile and 50 µL water. After removal of the solvent the residue was dried by azeotropic distillation with 3×1 mL acetonitrile. The residue was taken up in 100 µL *tert*-butanol/acetonitril 1:1 and added to 1 mg (ca. 1.3 µmol) of a trimethylpyridin-2-aminium precursor. The solution was reacted at 75 °C for 10 min. The product was isolated by HPLC (0-30% acetonitrile in 10 min), freed from solvents and taken up in 0.9% saline before injection.

Alternatively 6-fluoronicotinamides were synthesized by trapping F-18 fluoride on a waters Sep-Pak QMA plus light cartridge (46 mg sorbent; preconditioned with 0.5 M KHCO₃), washed with acetonitrile, dried and eluted with 0.5 mg (ca. 0.4 - 0.6 µmol) of the (protected) FAPI-precursor in 0.5 mL methanol. The solvent was removed *in vacuo* and the residue taken up in 100 µL acetonitrile/*tert*-butanol 1:4. After 20 min at 70 °C the reaction mixture was diluted with water and the protected intermediates worked up by solid phase extraction (sep-pak light C18, Waters). The solvents were removed and 200 µL of trifluoroacetic acid were added to the residue. The mixture was heated to 95 for 3 min, dried *in vacuo* and diluted with water before the product was isolated by HPLC, which was directly performed with the diluted reaction mixture in case of compounds lacking protecting groups. The products were freed from solvents and taken up in 0.9% saline before injection in case of animal studies. (Uncorrected radiochemical yield approx. 25%)

For determination of the stability in human serum the radiolabeled compounds (approx. 2.5 MBq for 1-125 or 15 MBq for Lu-177) were purified (HPLC or solid phase extraction) and freed from solvent. The residues were taken up in 250 µL human serum (Sigma-Aldrich) and incubated at 37 °C. Samples were precipitated with 30 µL acetonitrile and analyzed by HPLC (0-30% acetonitrile in 10 min).

### Example 2: In vitro characterization of FAPI derivatives

In vitro binding studies were performed using the human tumor cell lines BxPC3, Capan-2, MCF-7 (purchased from Sigma Aldrich Chemie GmbH) and SK-LMS-1 (purchased from ATCC) as well as stably transfected FAP-cell lines HT-1080-FAP, HEK-muFAP and the CD26 expressing cell line HEK-CD26 (obtained from Stefan Bauer, NCT Heidelberg). All cells were cultivated in Dulbecco's modified Eagle's medium (DMEM) containing 10% fetal calf serum at 37°C/5% carbon dioxide. For fluorescence internalization experiments, cells were seeded on coverslips and stained with F API-02-Atto488 and DAPI for cell nucleus staining. Images were acquired on a laser scanning confocal microscope using a 63x oil immersion objective. Radioligand binding studies were performed using HT-1080-FAP cells. The radiolabeled compound was added to the cell culture and incubated for different time intervals ranging from 10 min to 24 h. Competition experiments were performed by simultaneous exposure to unlabeled (10⁻⁵ M to 10⁻⁹ M) and radiolabeled compound for 60 min. For efflux experiments, radioactive medium was removed after incubation for 60 min and replaced by non-radioactive medium for time intervals ranging from 1 to 24 h. For internalization experiments, surface bound activity was removed by incubating the cells with 1 M glycine-HCl buffer for 10 min. The radioactivity was measured using a γ-counter, normalized to 1 mio cells and calculated as percentage of applied dose (%ID).

### Cell staining and microscopy

For internalization experiments HT-1080-FAP and HEK muFAP cells were seeded on uncoated coverslips in a 24-well plate and cultivated in culture medium containing 10% fetal calf serum to a final confluence of approx. 80-90%. The medium was removed and cells were washed with 0.5 mL PBS pH 7.4 for 2 times. FAPI-02-Atto488 (20 µM in DMEM) was added to the cells and incubated for 2 hrs at 37°C. Cells were washed with 0.5 mL PBS pH 7.4 for 3 times and fixed with paraformaldehyde (2% in PBS) for 15 min. The overgrown coverslips were placed on microscope slides using mounting medium containing DAPI for cell nucleus staining (Fluoroshield, Sigma-Aldrich). Images were acquired on a laser scanning confocal microscope (Zeiss LSM 700; Zeiss, Oberkochen, Germany) using the Zeiss Plan-Apochromat 63x/1.4 Oil DIC III immersion objective at xy pixel settings of 0.099 x 0.099 µm and 1 Airy unit pinhole size for each fluorophore used (488 nm for FAPI-02-Atto488, 405 nm for DAPI). The pictures were processed consistently using the ZEN 2008 software and ImageJ.

### Radioligand binding studies

For radioligand binding studies, cells were seeded in 6-well plates and cultivated for 48 h to a final confluence of approx. 80-90% (1.2 - 2 mio cells/well). The medium was replaced by 1 mL fresh medium without fetal calf serum. The radiolabeled compound was added to the cell culture and incubated for different time intervals ranging from 10 min to 24 h. Competition experiments were performed by simultaneous exposure to unlabeled (10⁻⁵ M to 10⁻⁹ M) and radiolabeled compound for 60 min. For efflux experiments, radioactive medium was removed after incubation for 60 min and replaced by non-radioactive medium for time intervals ranging from 1 to 24 h. In all experiments, the cells were washed with 1 mL phosphate-buffered saline pH 7.4 for 2 times and subsequently lysed with 1.4 ml lysis buffer (0.3 M NaOH, 0.2% SDS). Radioactivity was determined in a γ-counter (Cobra II, Packard), normalized to 1 mio cells and calculated as percentage of the applied dose (%ID). Each experiment was performed 3 times, and 3 repetitions per independent experiment were acquired.

For internalization experiments the cells were incubated with the radiolabeled compound for 60 min at 37 °C and 4 °C. Cellular uptake was terminated by removing medium from the cells and washing 2 times with 1 mL PBS. Subsequently, cells were incubated with 1 mL of glycine-HCl (1 M in PBS, pH 2.2) for 10 min at room temperature to remove the surface bound activity. The cells were washed with 2 mL of ice-cold PBS and lysed with 1.4 mL of lysis buffer to determine the internalized fraction. For the cells incubated at 4 °C, all washing and elution steps were carried out using ice-cold buffers. The radioactivity was measured using a γ-counter, normalized to 1 mio cells and calculated as percentage of applied dose (%ID).

### FAPI-01 selectively targets human and murine FAP-α.

In order to analyze the binding properties of FAPI-01 to its target protein, radioligand binding assays were performed using different cancer cell lines and cell lines transfected with human and murine FAP as well as the closely related membrane protein CD26, also known as DPPIV. Both murine FAP and CD26 show a high homology to human FAP-α (muFAP: 90 % identity and 94 % similarity on amino acid level; CD26: 52 % identity and 71 % similarity with high structural resemblance) (Kelly T., Drug Resist Updat, 2005)*.*

As shown in Figure 1A, FAPI-01 shows no significant binding to FAP-negative cancer cell lines while targeting human and murine FAP-α expressing cells with high affinity (IC₅₀ human FAP-α = 39.4 nM). Additionally, no substantial binding to CD26 expressing cells was observed (0.05 ± 0.01 %), proving that FAPI-01 is selectively targeting FAP-α. This is of particular importance as CD26 is highly expressed in a variety of normal tissues including the kidneys, the liver and the small intestine. To avoid a high background signal due to unspecific CD26-binding, high selectivity of the ligand to FAP-α is of great advantage, resulting in optimal image quality.

### FAPI-01 rapidly internalizes in FAP-positive cells but shows time-dependent efflux and robust deiodination.

Cell-based internalization assays demonstrate a rapid uptake of FAPI-01 into the cells (Figure 1B). After 10 min of incubation, 95 % of the total bound fraction is located intracellularly (total 19.70 ± 0.28 %). In the course of 4 h, only a marginal decrease in activity is observed (total 17.00 ± 0.40 %, of which internalized 94 %).

Iodine-labeled compounds often show a time-dependent enzymatic deiodination. This was also observed for FAPI-01 resulting in low intracellular radioactivity of this compound after longer incubation times (3.25 ± 0.29 % after 24 h). Deiodination can be minimized by reduction of deiodinase activity after lowering the temperature to 4°C, resulting in an increased radioactivity of 26.66 ± 1.59 % after 24 h.

### FAPI-02 shows enhanced binding and uptake to human FAP-α as compared to FAPI-01.

To avoid rapid loss of activity of FAPI-01 due to enzymatic deiodination, the non-halogen derivative FAPI-02 was designed in which the FAP-binding moiety is chemically linked to the chelator DOTA. In addition to the resulting enhanced stability, this modification offers the possibility to easily incorporate either diagnostic or therapeutic radionuclides, allowing FAPI-02 to be used as a theranostic compound. Similar to its iodized analogue, FAPI-02 specifically binds to human and murine FAP-α (IC₅₀ human FAP-α = 21 nM) expressing cells without addressing CD26 (%ID = 0.13 ± 0.01 %; Figure 1A). FAPI-02 internalizes rapidly into FAP-α expressing cells (20.15 ± 1.74 %ID after 60 min, of which 96 % internalized; Figure 1B), showing more stable and higher uptake rates in the course of time. Compared to the binding of FAPI-01 after 10 min of incubation, only 5 % of the activity remains after 24 h. In contrast, 34 % of the initial radioactivity of FAPI-02 is detected after 24 h of incubation. Efflux experiments demonstrate that FAPI-02 gets eliminated significantly slower than FAPI-01, showing retention of 12 % of the originally accumulated radioactivity after 24 h (FAPI-01 1.1 % ID after 24 h; Figure 1E).

Robust internalization of FAPI-02 into human and murine FAP-α expressing cells was confirmed by fluorescence laser scanning microscopy. To this end, HT-1080-FAP and HEK-muFAP cells were stained with a fluorescently labeled FAPI-02 derivative (FAPI-02-Atto488) for 1 to 2 hrs. As shown in Figure 1D, the compound gets completely internalized and accumulates in the inner of FAP-α expressing cells whereas no uptake is detectable in FAP-α negative HEK-CD26 cells.

### Design of FAPI derivatives with enhanced binding properties and pharmacokinetics

Further variants of FAPI-02 were designed to increase tumor retention time, aiming for the development of a theranostic FAP-targeting agent. The variants FAPI-03 to FAPI-15 have been characterized with respect to target binding, internalization rate and target specificity. The results are shown in Figure 2.

### Example 3: PET imaging and biodistribution analysis in mice

All experiments were performed in accordance with the German animal protection laws and complied with European Commission regulations for the care and use of laboratory animals. The mice were anaesthetized using isoflurane inhalation.

For *in vivo* experiments, 8 week old BALB/c *nu*/*nu* mice (Charles River) were subcutaneously inoculated into the right trunk with 5 × 10⁶ with HT-1080-FAP, Capan-2 or SK-LMS-1 cells, respectively. When the size of the tumor reached approximately 1 cm³, the radiolabeled compound was injected via the tail vein (~10 MBq for small-animal PET imaging; ~1 MBq for organ distribution). PET imaging was performed up to 140 min after intravenous injection of 1 MBq of Ga-68 labeled compound per mouse using the Inveon PET small-animal PET scanner (Siemens). Images were reconstructed iteratively using the 3D-OSEM+MAP method and were converted to standardized uptake value (SUV) images. Quantification was done using a ROI technique and expressed as SUV mean. For organ distribution of Lu-177 labeled compound (approx. 10 MBq per mouse), the animals (*n* = 3 for each time point) were sacrificed after indicated time points (from 30 min to 24 h). The distributed radioactivity was measured in all dissected organs and in blood using a γ-counter (Cobra Autogamma, Packard). The values are expressed as percentage of injected dose per gram of tissue (%ID/g).

For pharmakokinetic modeling the transport constant K1 and the rate constants k2-k4 were calculated using a two-tissue compartment model implemented in the PMOD software [4], taking into account the vascular fraction (vB), which is associated with the volume of blood exchanging with tissue in a VOI. The rate constants that describe the compartmental fluxes include k1 (binding to the receptor), k2 (detachment) as well as k3 (internalization) and k4 (efflux) in the tumor tissue. In this model the fractional volume of distribution (DV=K1/k2) is the proportion of the region of interest in which ¹⁵O-labelled water is distributed.

**FAPI variants accumulate in human FAP-expressing xenografts as well as in xenografts without FAP expression by recruitment and activation of mouse fibroblasts.**

Tumor accumulation of FAPI-02 and -04 was assessed by small-animal PET imaging of mice bearing xenografts from both human FAP-positive and negative tumor cells. In both cases the radiotracer gets rapidly enriched within the tumor and is maintained for at least 140 min (Figure 3A, C, E, G). At the same time, FAPI-02 and -04 show negligibly low unspecific binding and get quickly eliminated from the blood predominantly via the kidneys and bladder resulting in a low background and beneficial tumor-to-organ ratios. Simultaneous administration of unlabeled compound as competitor resulted in a complete absence of radioactivity in the tumor, demonstrating the specificity of the radiotracer to its target protein (Figure 4). Interestingly, a high tumor uptake of FAPI-02 was observed in mice bearing FAP-α positive (HT-1080-FAP) as well as FAP-α negative (Capan-2) tumor cell lines due to recruitment and activation of activated mouse fibroblasts. Pharmacokinetic characteristics of the radiotracer, calculated from PET data using a two-tissue compartment model according to Burger et al., Nucl Med, 1997, are given in Table 6.

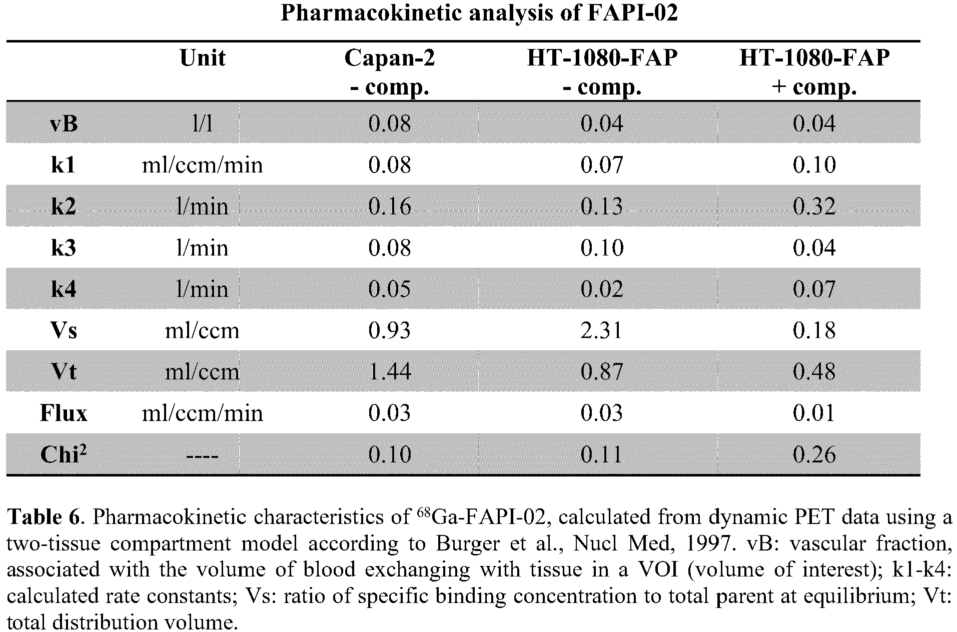

These observations were confirmed using ¹⁷⁷Lu-FAPI-02 and -04 in a biodistribution study, proving rapid tumor accumulation in both human FAP-α positive and negative tumors with very low activity in all the other organs (quantified uptake values see Table 7), resulting in beneficial tumor-to-organ ratios (Figure 5D-F). Similar results were obtained for ¹⁷⁷Lu-FAPI-04 in HT-1080-FAP tumor bearing mice. Compared to FAPI-02, FAPI-04 shows a higher tumor uptake especially after 24 h (Figure 5C). A calculation of the area under curve (AUC) is shown in Table 8.

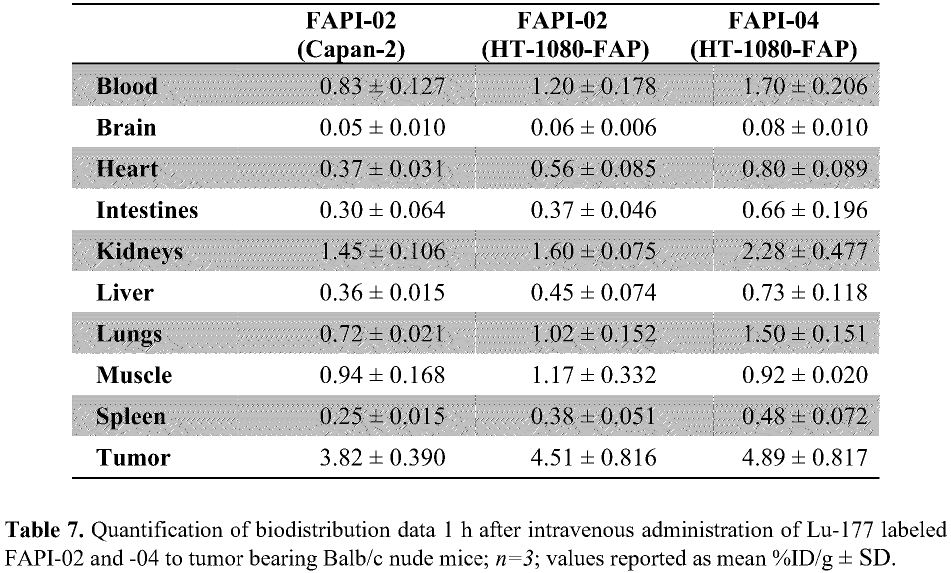

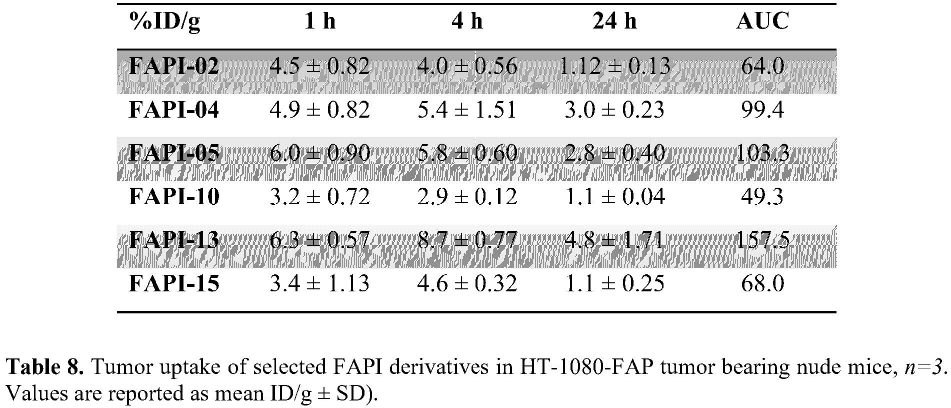

### Example 4: Clinical PET/CT studies.

Diagnostic imaging of more than 100 patients was performed under the conditions of the updated declaration of Helsinki, § 37 (Unproven interventions in clinical practice) and in accordance to the German Pharmaceuticals Law §13 (2b) for medical reasons using either ⁶⁸Ga-FAPI-02 or -04, which was applied intravenously (20 nmol, 122-336 MBq), 10 min, 1 and 3 hours post tracer administration. Variation of injected radiotracer activity is due to the short half-life of ⁶⁸Ga and the variable elution efficiencies obtained during the lifetime of the ⁶⁸Ge/⁶⁸Ga generator. FDG imaging of one patient was conducted 1 h after intravenous injection of 358 MBq ¹⁸F-FDG. The PET/CT scans were performed with a Biograph mCT Flow^{™} PET/CT-Scanner (Siemens Medical Solution) using the following parameters: slice thickness of 5 mm, increment of 3-4 mm, soft-tissue reconstruction kernel, care dose. Immediately after CT scanning, a whole-body PET was acquired in 3D (matrix 200x200) in FlowMotion^{™} with 0.7 cm/min. The emission data were corrected for random, scatter and decay. Reconstruction was conducted with an ordered subset expectation maximisation (OSEM) algorithm with 2 iterations/21 subsets and Gauss-filtered to a transaxial resolution of 5 mm at full-width half-maximum (FWHM). Attenuation correction was performed using the low-dose non-enhanced CT data. The quantitative assessment of standardized uptake values (SUV) was done using a region of interest technique.

### FAPI-02 and -04 rapidly accumulate in breast, pancreatic, lung, HNO, small intestine and ovarian cancer metastases in humans.

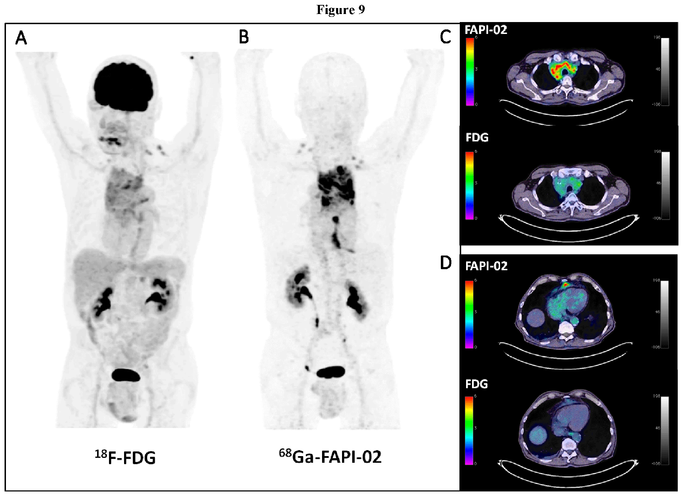

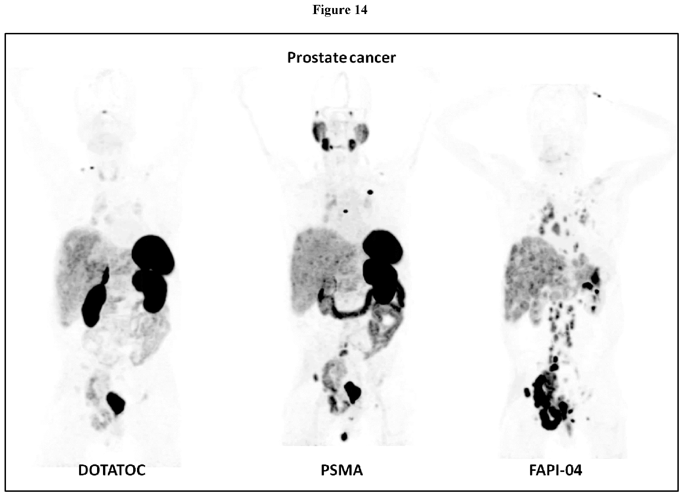

Diagnostic PET/CT scans were performed 1 h after intravenous administration of ⁶⁸Ga-FAPI-02 and -04 in patients with metastasized breast, lung, pancreatic, HNO, small intestine and ovarian cancer. In all patients a robust accumulation of the tracer was observed in the primary tumor as well as in lymph node and bone metastases with maximum SUV values of 48.0. In contrast, tracer uptake into normal tissue was very low (Figures 6-14). The radioactivity was cleared rapidly from the blood stream and excreted predominantly via the kidneys, resulting in high contrast images. Comparative imaging in one patient with locally advanced lung adenocarcinoma revealed an obvious advantage of FAPI-02 compared to the commonly used PET tracer ¹⁸F-FDG. As shown in Figure 9, FAPI-02 shows a higher uptake with lower background activity leading to a higher contrast with better visibility of metastatic lesions. In contrast to FDG, which is highly accumulating in cells with high glucose consumption e.g. the brain, FAPI-02 selectively targets tissues where FAP-α is expressed. Comparative imaging in one patient with prostate cancer revealed an obvious advantage of FAPI-04 compared to the commonly used PET tracers ⁶⁸Ga-DOTATOC and ⁶⁸Ga-PSMA, allowing the detection of smaller tumor lesions with reduced tracer accumulation in the kidneys (Figure 14).

### Discussion

The reliable diagnosis of primary tumors, metastatic lesions and affected lymph nodes is of upmost importance to allow for effective and adequate therapy planning including tumor staging and choice of treatment. For this purpose, imaging techniques represent indispensable tools for the assessment of many cancer types. Due to its high diagnostic accuracy and the possibility to evaluate both anatomic and physiologic details, combined PET/CT is the method of choice for modern tumor diagnostics. In contrast to non-invasive imaging techniques such as MRT or CT alone, combined PET/CT, however, requires the use of radiotracers with a high affinity to target structures with enhanced expression in tumors as compared to normal tissues. An ideal tracer should specifically bind to its target protein to ensure reliable differentiation of cancerous and healthy tissue as well as low background signals resulting in high-contrast images. Affinity and specificity become even more important if the radiotracer represents a theranostic compound, i.e. offers the possibility to be loaded with either diagnostic or therapeutic nuclides, which facilitates and improves targeted and personalized treatment. Regarding a potential application of the tracer for therapeutical purposes, high target specificity assures reduced side-effects, which is especially important for the protection of radiation sensitive tissue such as bone marrow, reproductive and digestive organs.

With that in mind, the inventors developed a theranostic tracer targeting cancer-associated fibroblasts which form a major component of the tumor stroma. They are known to play a critical role in tumor growth, migration and progression and are genetically more stable than cancer cells, therefore, being less susceptible to the development of therapy resistance. In contrast to normal fibroblasts, CAFs express particular proteins which can be used as tumor-specific markers. Among these is the membrane protein FAP-α which is broadly expressed in the microenvironment of a variety of tumors and thus enables targeting of different tumor entities including pancreas, breast and lung cancer, which account for a large part of the entirety of solid tumors.

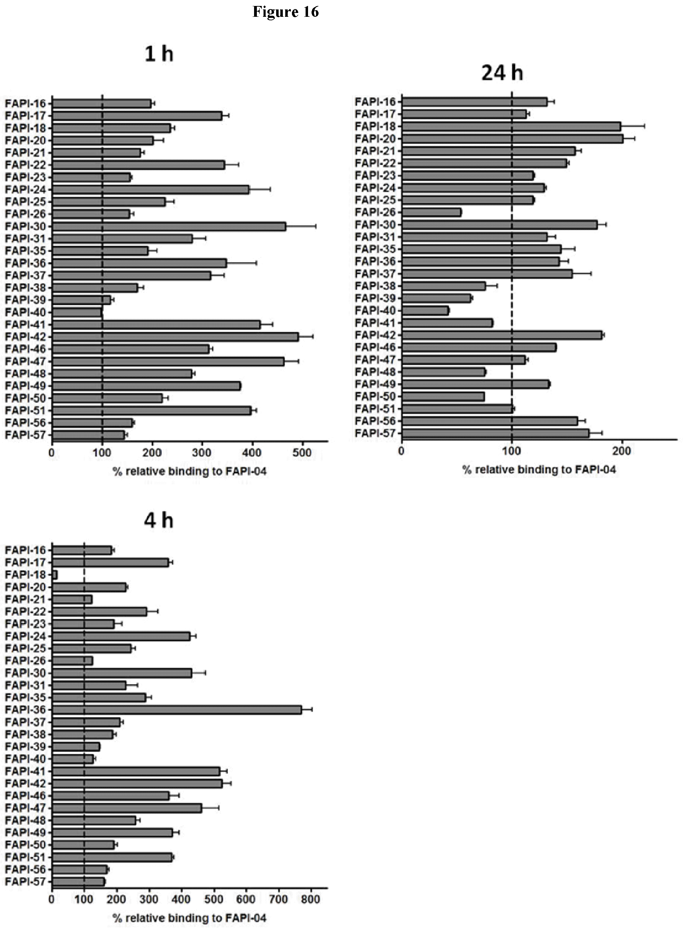

Based on a small molecule enzyme inhibitor with high affinity to its target protein, we developed the radiotracers FAPI-01 to FAPI-73 by focused chemical modification. All compounds show specific binding to human and murine FAP-α with a rapid and almost complete internalization without addressing the closely related protein CD26/DPP4. Since iodinated molecules undergo an enzymatic deiodination with efflux of free iodine, longer incubation times result in a low intracellular radioactivity. On this account, FAPI-02 and subsequent compounds were designed with the FAP-binding moiety being chemically linked to the chelator DOTA. This results in a set of theranostic compounds with favorable pharmacokinetic and biochemical properties, of which FAPI-02, FAPI-04, FAPI-46, FAPI-34, FAPI-42, FAPI-52, FAPI-69, FAPI-70, FAPI-71, FAPI-72 and FAPI-73represent the most favored ligands. FAPI-02 and FAPI-04 both get eliminated significantly slower than FAPI-01, with retention of 12 % (FAPI-02) and 49 % (FAPI-04) of the originally accumulated radioactivity after 24 h (FAPI-01 1.1 %) with the other favored compounds having an even stronger binding (figure 16). They rapidly internalize into FAP-α expressing cells and show high tumor uptake rates in both tumor bearing mice and patients with metastasized epithelial carcinomas. In contrast, there is no accumulation in normal tissue and rapid clearance from the blood system, resulting in high-contrast images. The robust internalization into both human and murine FAP-α expressing cells was confirmed by confocal microscopy using fluorescence-labeled FAPI-02. In contrast to the first generation FAP-antibody F19, which has high affinity to its target protein without being internalized, FAPI-02 shows complete intracellular uptake after 1 h of incubation. The mechanism of internalization after FAP binding has been studied by Fischer et al. using FAP antibody fragments (Fabs) and a DyLight 549 anti-mouse antibody in SK-Mel-187 cells. Incubation at 37 °C led to internalization of the FAP-antibody complexes. As with our small molecule, the internalization process occurred rapidly with an almost complete internalization. Colocalization of the Fabs with a marker for early endosomes was observed after 20 minutes and with a marker for late endosomes and lysosomes after 40 minutes. Fab-mediated FAP-α internalization was suppressed by an inhibitor for dynamin dependent endocytosis, indicating that endocytosis occurs by a dynamin-dependent mechanism.

FAPI-02 and -04 get quickly eliminated from the organism by renal clearance without being retained in the renal parenchyma. In contrast to ¹⁸F-FDG, which is highly accumulating in cells with high glucose consumption including inflammatory tissue or the brain, FAPI-02 gets selectively enriched in tissues where its target protein is expressed. This opens new perspectives for the detection of malign lesions in these regions. Additionally, FAP-α was also shown to be expressed by rheumatoid myofibroblast-like synoviocytes in patients with rheumatoid arthritis and osteoarthritis, atherosclerosis, fibrosis as well as in ischemic heart tissue after myocardial infarction. These observations suggest the application of FAPI-02 and -04 as imaging tracers for further indications.

The limiting factor for the detection of tumor lesions is the degree of FAP-α expression within the tumor. This largely depends on the number of activated fibroblasts, i.e. the percentage of stromal content, and/or the number of FAP-α molecules per fibroblast which may be determined by the microenvironment. Since tumor growth exceeding a size of 1 to 2 mm essentially requires the formation of a supporting stroma, visualization of small lesions in the range of 3-5 mm should be possible using FAPI-PET/CT.

As with any other targeted approach, the FAPI derivatives only achieve optimal results in tissues with sufficiently high FAP-α expression which is known to be rather heterogeneous in different cancer types and patients. Besides breast, colon and pancreatic cancer, which are excellent candidates for FAPI imaging, further analyses have to explore whether other tumor entities such as lung cancer, head and neck cancer, ovarian cancer or hepatomas represent favorable targets.

Also, FAP-α expression was demonstrated in wound healing and fibrotic tissue, which should be kept in mind when interpreting radiological findings. These facts emphasize the necessity to properly evaluate which patients are likely to benefit from a potential FAPI therapy. Given the ability to use either diagnostic or therapeutic nuclides, FAPI-02 and -04 allow simple stratification of the appropriate patient cohort. Either way, it is already clear that both FAPI tracers represent ideal candidates for the development of a targeted radiopharmaceutical. Due to their high target affinity, rapid tumor internalization and fast body clearance, they are already ideally suitable for tumor imaging.

### Example 5: FAPI characterization in vitro and in vivo

### Experimental procedures and clinical evaluation

All *in vitro* and *in vivo* experiments as well as the clinical evaluation of the FAPI derivatives have been performed as described above and according to Loktev *et al*.¹ and Lindner *et al*.² A preliminary dosimetry estimate for FAPI-02 and FAPI-04 was based on two patients examined at 0.2 h, 1 h and 3 h post tracer injection using the QDOSE dosimetry software suit. Further PET/CT scans of tumor patients were acquired 1 h after injection of either FAPI-02 (n=25) or FAPI-04 (n=25); for 6 patients an intra-individual related FDG-scan (also acquired 1 h p.i.) was available. For the normal tissue of 16 organs, a 2 cm Spheric-VOI was placed in the parenchyma, for tumor lesions a threshold segmented VOI was used to quantify SUVmean/max³.

### In vitro characterization of DOTA-FAPI derivatives

To assess target binding and internalization rate of the DOTA-FAPI derivatives as compared to FAPI-04, Lu-177-labeled compounds were incubated with FAP-expressing HT-1080 cells for 1, 4 and 24 h, respectively (figure 16). The membrane-bound fraction was removed by acidic elution using glycine-HCl pH 2.2 followed by alkaline cell lysis to determine the internalized fraction. As shown in Figure 16, all derivatives demonstrate higher cell binding as compared to FAPI-04 with binding values up to 500 % of the lead compound after 1 h of incubation (up to 750 % after 4 h).

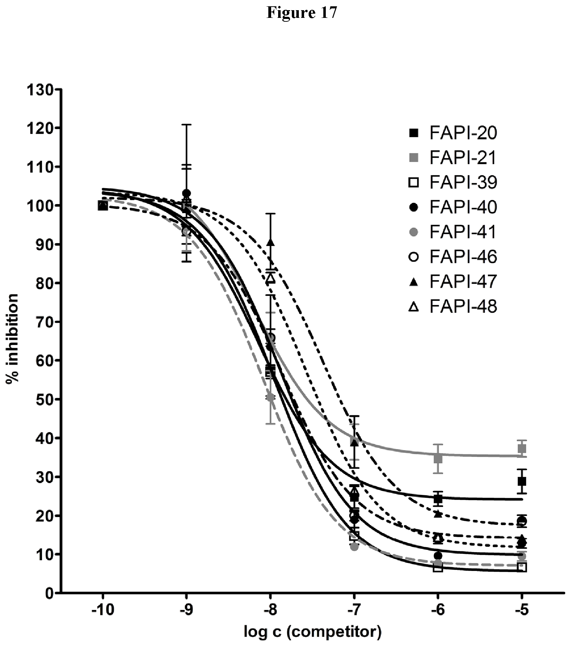

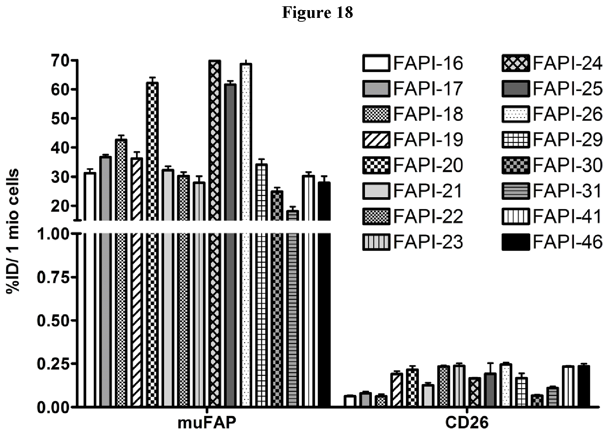

To evaluate target affinity and specificity, competitive binding assays were performed using increasing concentrations of unlabeled compound as competitor of Lu-177-labeled compound (Figure 17; respective IC₅₀ values listed in Table 9). Specificity of binding was also confirmed in a radioligand binding assay using murine FAP- and CD26-expressing HEK cells (Figure 18).

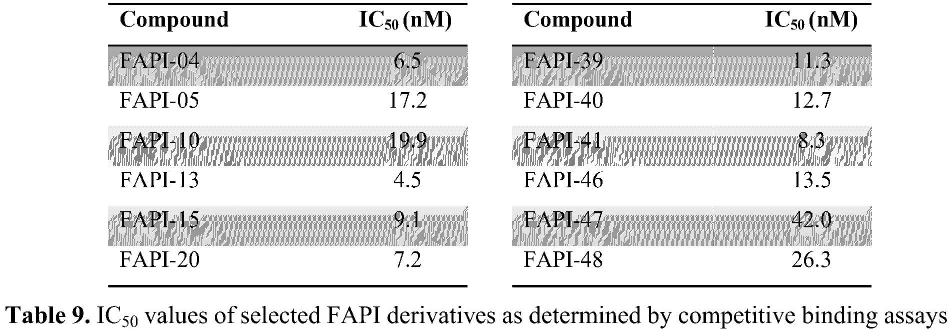

### Organ distribution of DOTA-FAPI derivatives in tumor-bearing mice

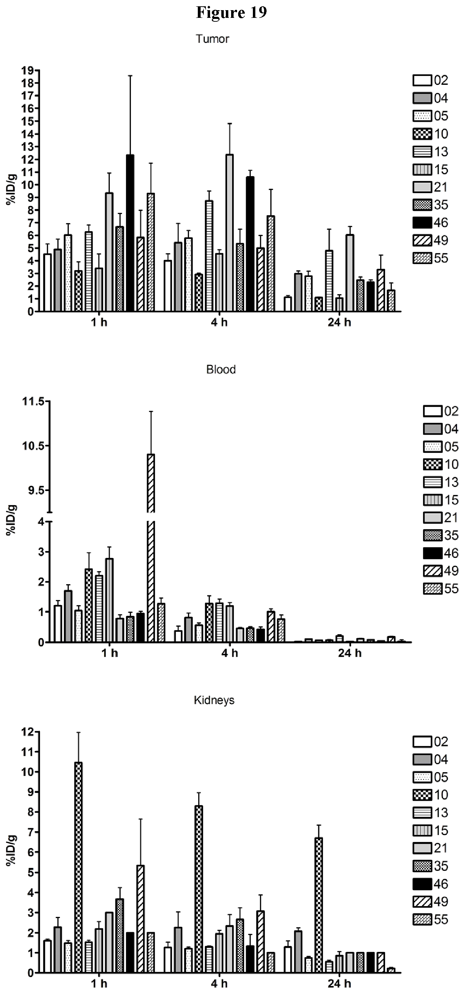

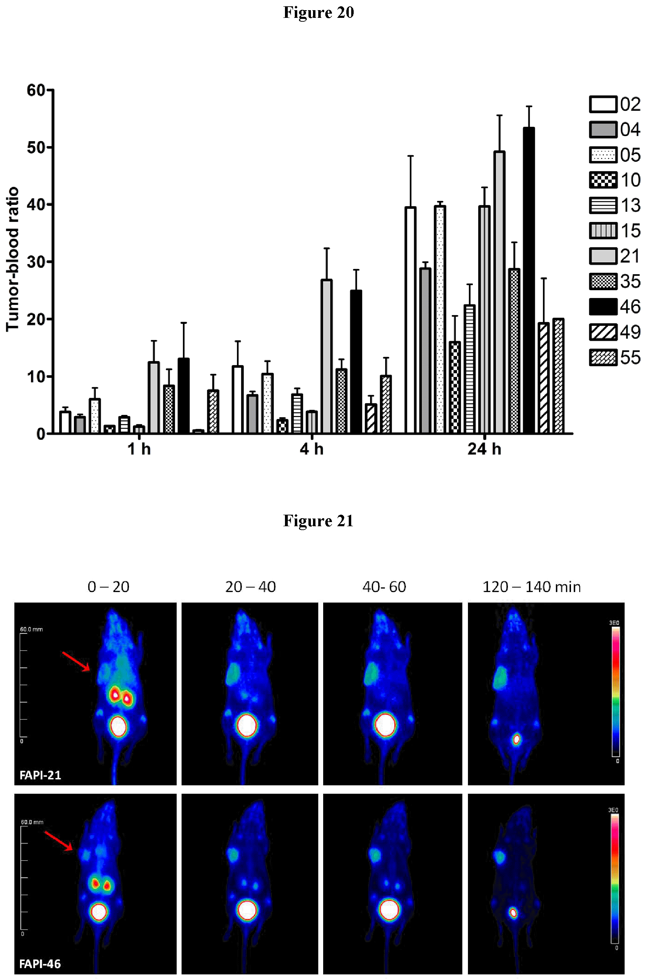

For analysis of pharmacokinetic profile as well as tumor uptake *in vivo,* Lu-labeled DOTA-FAPI derivatives were administered i.v. to HT-1080-FAP tumor-bearing mice. Organ distribution of the radiolabeled compounds was determined *ex vivo* in the blood, healthy tissues and the tumor. As shown in Figure 19, most of the compounds demonstrate higher tumor uptake rates as compared to FAPI-02 and FAPI-04, notably 24 h after administration. Due to increased lipophilicity, some of the radiotracers show higher blood activities as well as an increased retention in the kidneys. Determination of tumor-blood-ratios yet reveals a clear advantage of the compounds FAPI-21 and FAPI-46 which demonstrate significantly higher ratios than FAPI-04 at all times examined (Figure 20).

### Small-animal imaging of DOTA-FAPI derivatives in tumor-bearing mice

Based on these findings, small animal PET-imaging was performed using Ga-68-labeled DOTA-FAPI derivatives up to 140 min after i.v. administration of the radiotracers in HT-1080-FAP tumor-bearing mice. The beneficial tumor-blood ratios of FAPI-21 and FAPI-46 result in high contrast images, enabling excellent visualization of the FAP-positive tumors (Figure 21).

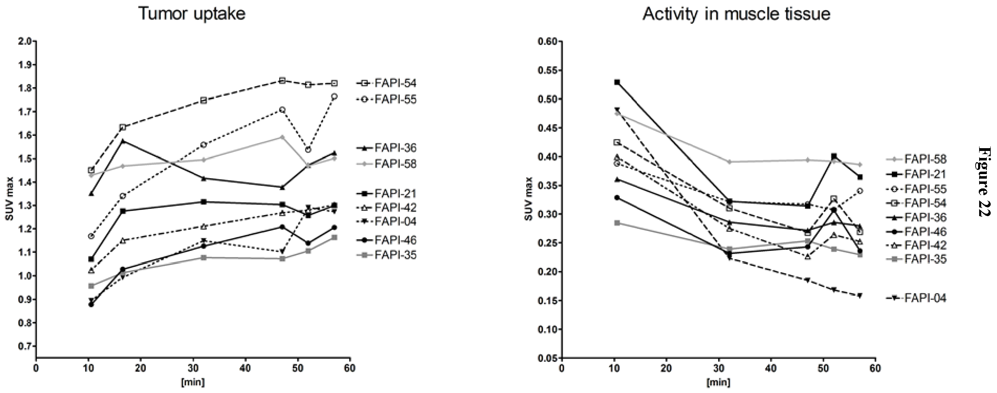

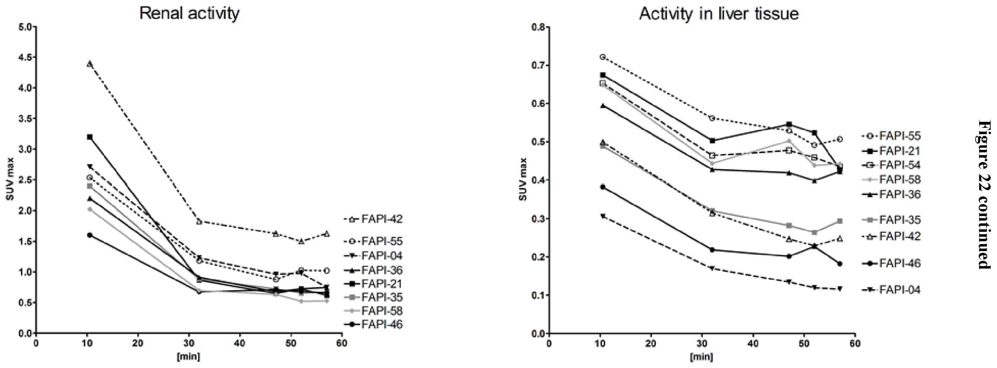

A quantitative analysis of the tracer accumulation in the tumor, the kidneys, the liver and muscle tissue (given as SUV max values) reveals slightly lower muscular, renal and hepatic activities of FAPI-46 as compared to FAPI-21 (Figure 22).

### Biodistribution and dosimetry estimate of FAPI-02 and FAPI-04 compared to FDG in cancer patients

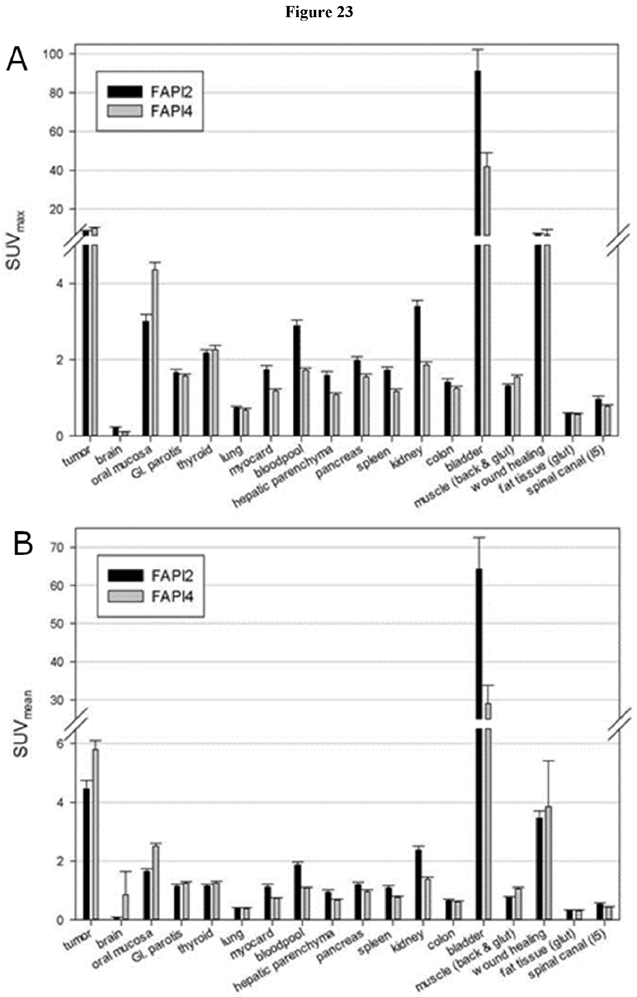

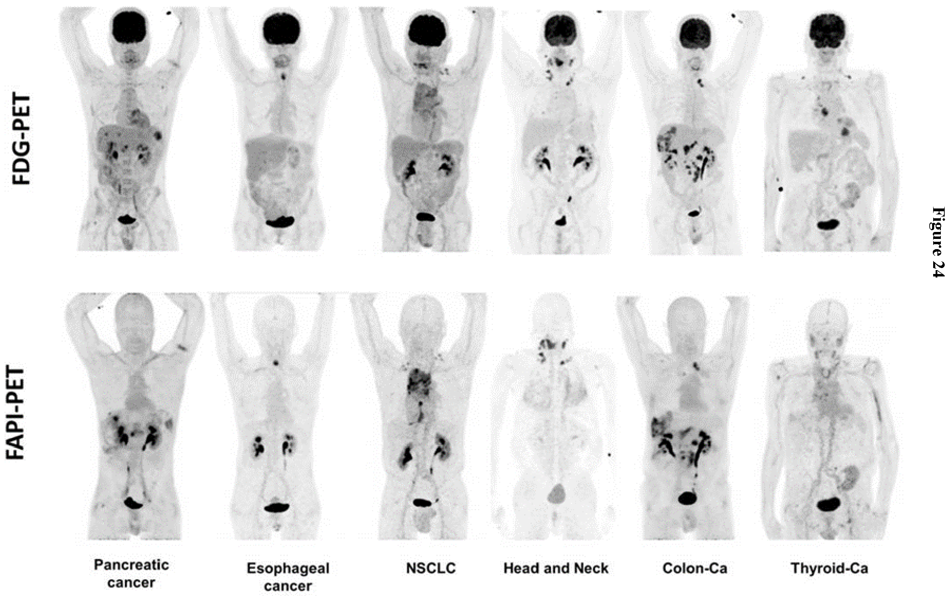

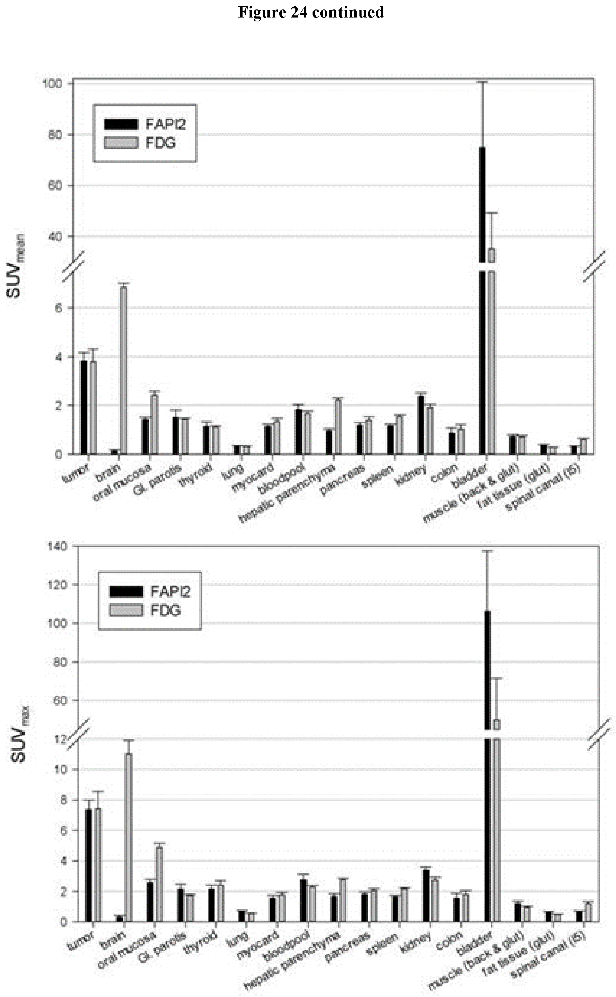

Very similar to literature values for F-18-FDG, Ga-68-DOTATATE or Ga-68-PSMA-11, an exam with 200 MBq Ga-68-FAPI-02 and -04 corresponds to an equivalent dose of approx. 3-4 mSv. After a fast clearance via the kidneys, the normal organs show a low tracer uptake with only minimal changes between 10 min and 3 h p.i. In FAPI-02, the tumor uptake from 1 h to 3 h p.i. decreases by 75 %, whereas the tumor retention is slightly prolonged with FAPI-04 (50 % washout). At 1 h p.i. both FAPI-tracers perform equally (Figure 23). In comparison to FDG, tumor uptake is almost equal (average SUV max FDG 7.41; SUV max FAPI-02 7.37; n.s.); the background uptake in brain (11.01 vs 0.32), liver (2.77 vs 1.69) and oral/pharyngeal mucosa (4.88 vs 2.57) is significantly lower with FAPI-02; other organs were not relevantly different between FDG and FAPI-02 (Figure 24). For detailed information and results see Giesel *et al.* ³ which is incorporated by reference herewith.

### PET imaging of FAPI-04 in patients with various cancers as well as non-cancerous malignancies

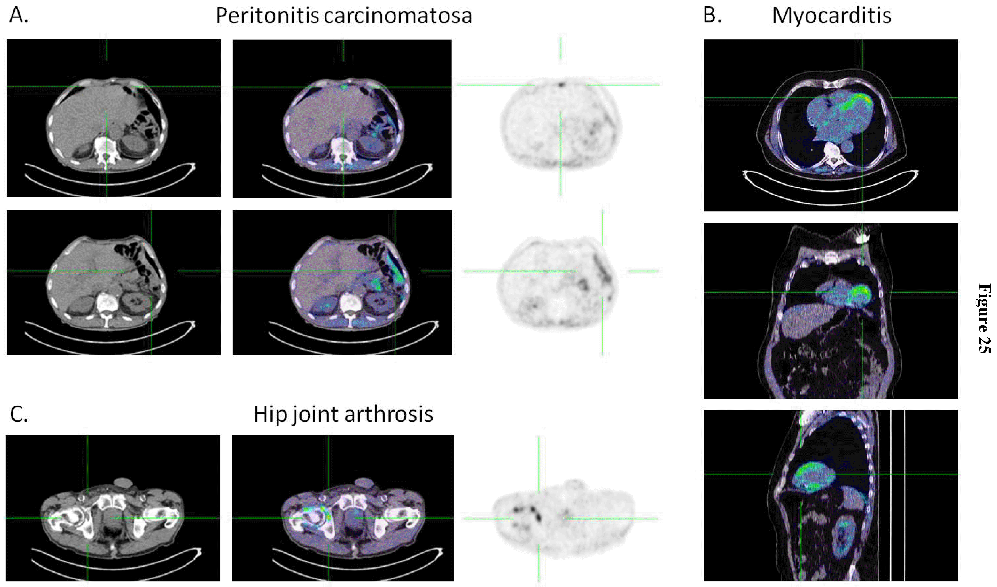

In addition to a rapid uptake of Ga-68-labeled FAPI-04 in different cancers including breast, pancreas, ovarial and HNO tumors, tracer accumulation was also demonstrated in peritonitis carcinomatosa (Figure 25A) as well as several inflammatory malignancies such as myocarditis (Figure 25B) and arthrosis (Figure 25C). These results indicate a potential application of Ga-68-labeled FAPIs for the detection of non-cancerous malignancies which are characterized by a chronical inflammation process involving recruitment of activated fibroblasts.

### PET imaging of FAPI-21 and FAPI-46 in patients with various cancers

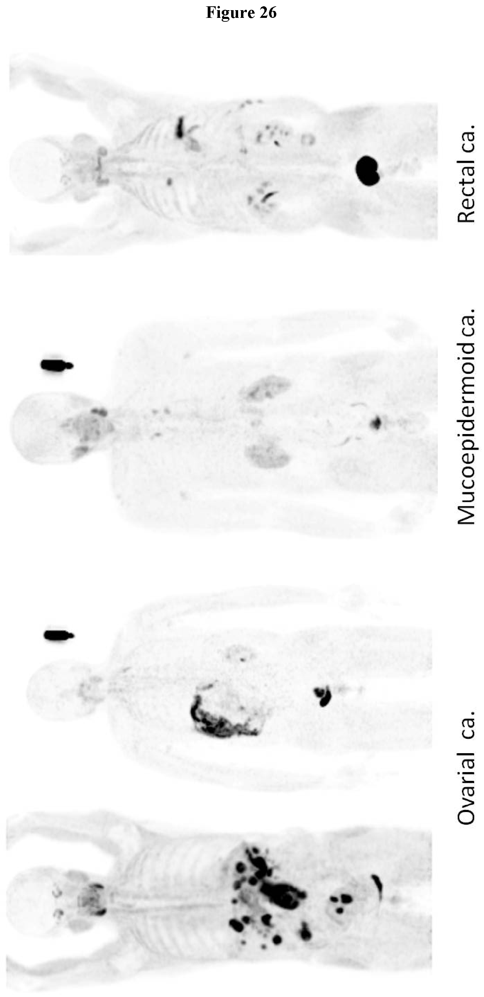

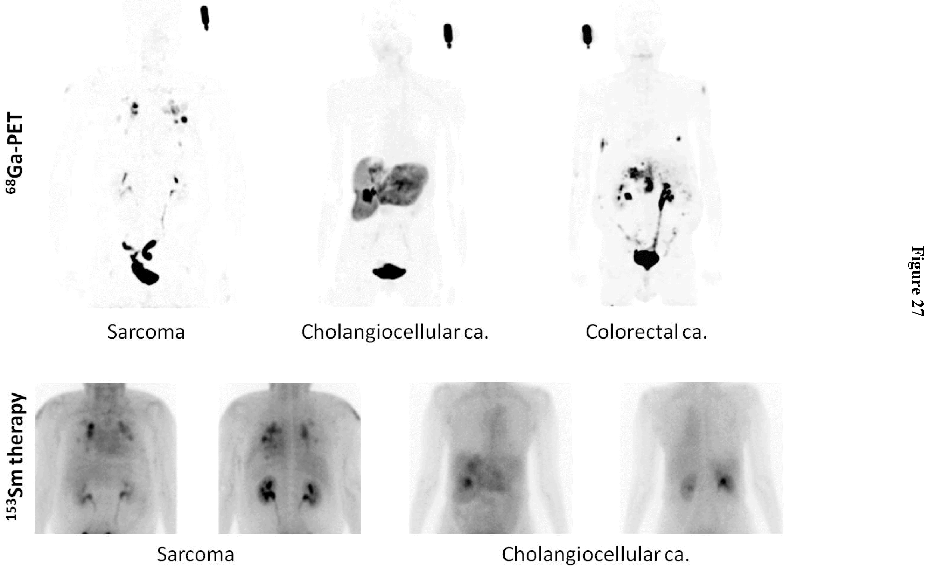

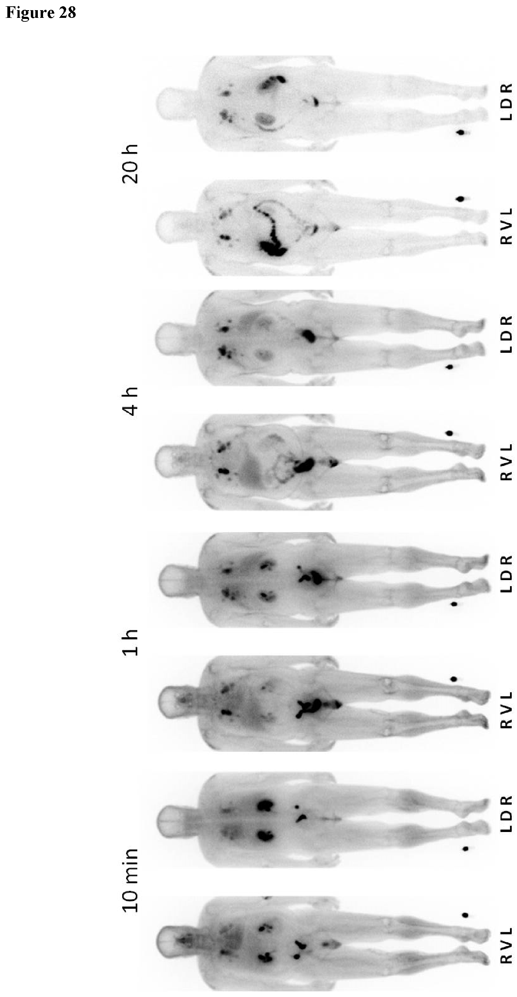

As shown in Figure 26, robust accumulation of Ga-68-labeled FAPI-21 was observed in different cancers including ovarian, rectal and mucoepidermoid carcinoma. Similar tumor uptake was shown for Ga-68-labeled FAPI-46 which rapidly accumulated in cholangiocellular and colorectal carcinoma, lung cancer as well as solitary fibrous sarcoma (Figure 27). Following PET/CT examination using Ga-68 labeled FAPI-46, a first therapeutical approach using the Sm-153 labeled radiotracer was taken in two cancer patients. As shown in Figure 28, robust tumor accumulation of the tracer is detectable up to 20 h after administration.

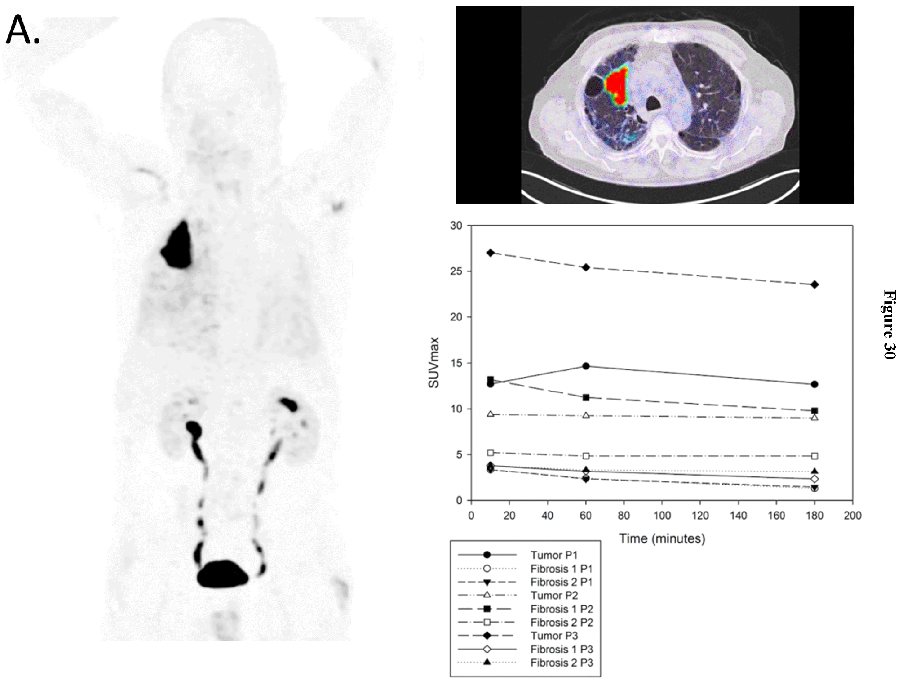

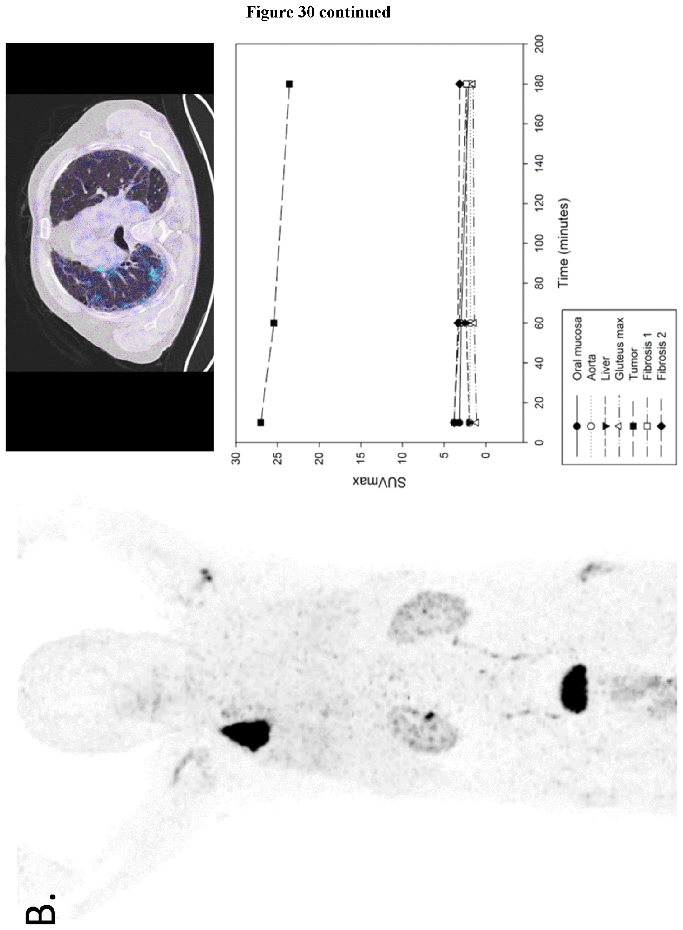

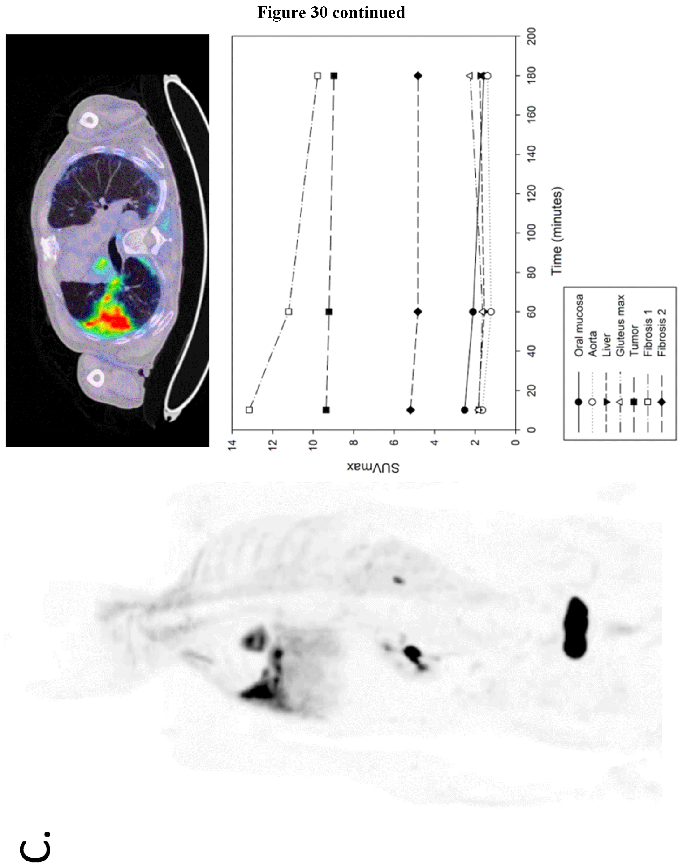

FAPI-46-PET/CT imaging of three lung cancer patients with idiopathic pulmonary fibrosis revealed a clear difference of tracer accumulation in the cancerous vs. fibrotic lesions. As shown in Figure 30, tumor uptake of Ga-68 labeled FAPI-46 was significantly higher in two patients (A, B) but slightly lower in one patient (C), as compared to the activity measured in the fibrotic tissue. The patient shown in Figure C suffered from an exacerbated lung fibrosis as compared to the two non-exacerbated cases. Therefore, the tracer is possibly useful for the differentiation of fibrosis patients with bad prognosis from patients with a good prognosis.

### FAPI derivatives for radiolabeling with alternative radionuclides, e.g. Tc-99m, Pb-203, Cu-64 and F18

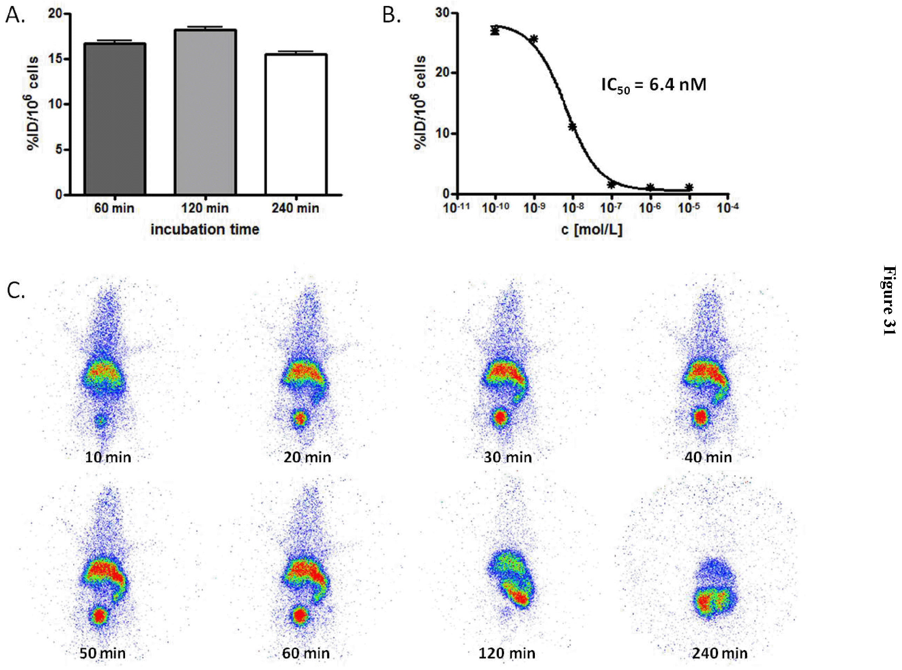

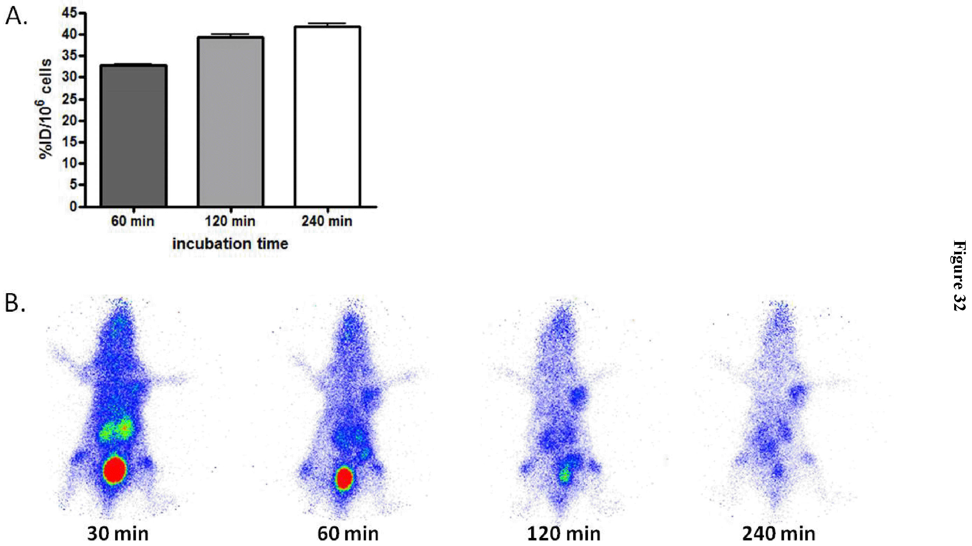

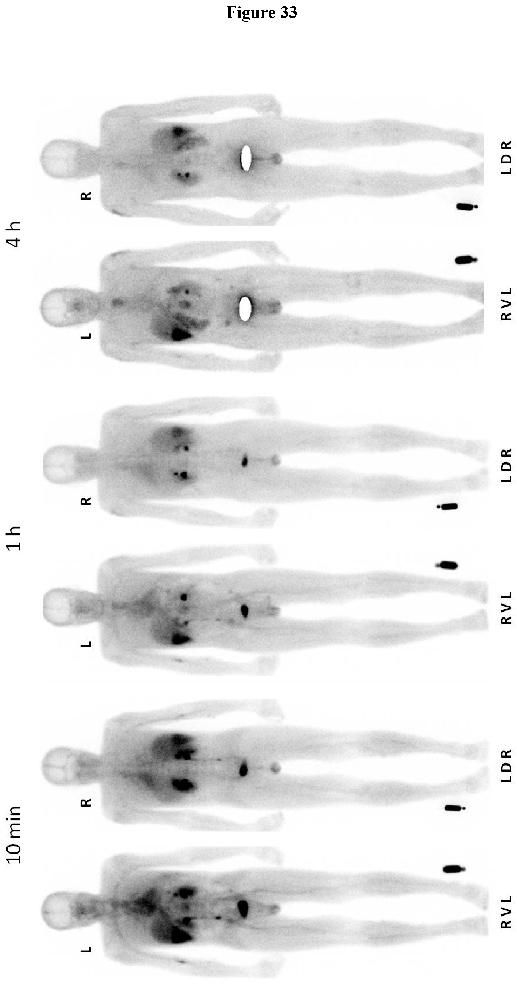

To enable the use of alternative radionuclides, a series of FAPI derivatives have been designed and characterized with respect to target affinity, specificity and pharmacokinetics. In some of these compounds, the original chelator DOTA has been replaced by different chelating moieties, which are ideally suitable for the incorporation of Tc-99m (FAPI-19, -27, -28, -29, - 33, -34, -43, -44, -45, -60, -61, -62). FAP affinity *in vitro* and biodistribution in HT-1080-FAP xenografted mice are shown exemplarily for FAPI-19 and FAPI-34. Both compounds demonstrate a robust binding to human FAP *in vitro* (IC₅₀ FAPI-19: 6.4 nM). In contrast to FAPI-19, which shows insufficient tumor uptake *in vivo* as well as a rapid accumulation in the liver due to a shift of renal towards hepatic elimination, FAPI-34 is continuously enriched within the tumor and demonstrates significantly less hepatic uptake (Figures 31, 32). A first diagnostic application of Tc-99m labeled FAPI-34 in a pancreas cancer patient with liver metastases shows a stable tumor accumulation of the tracer up to 4 h after administration. In addition, the overall background activity is comparably low, resulting in high-contrast images (Figure 33). This offers a widespread application for diagnosis by scintigraphy and therapy after labeling with Re-188.

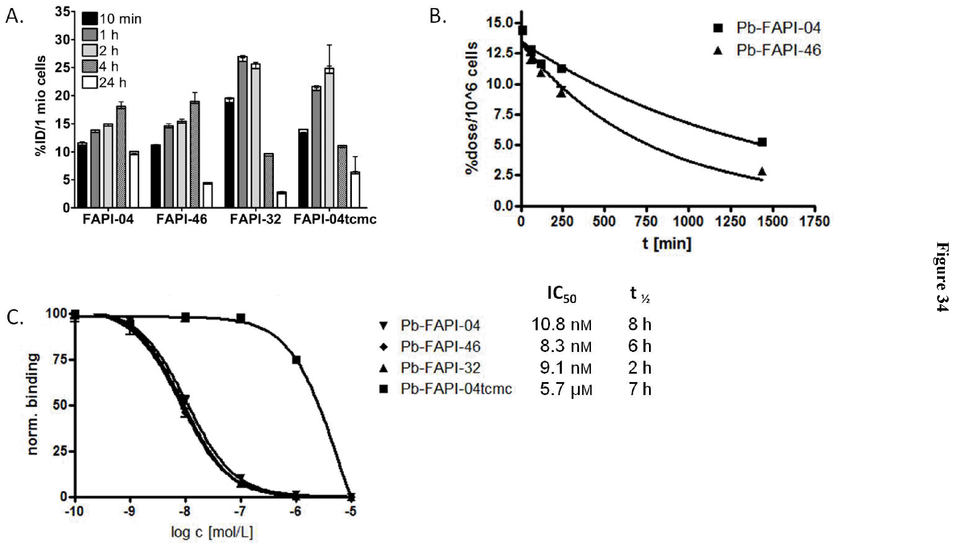

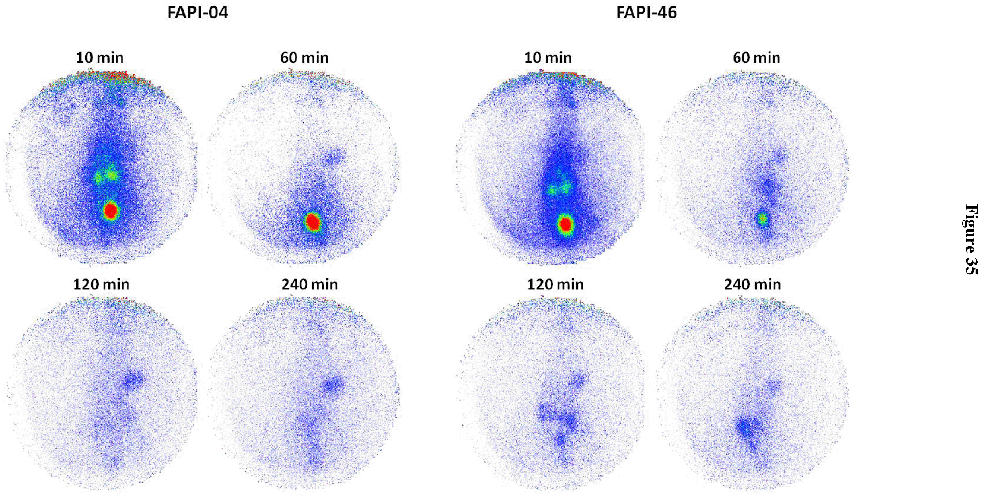

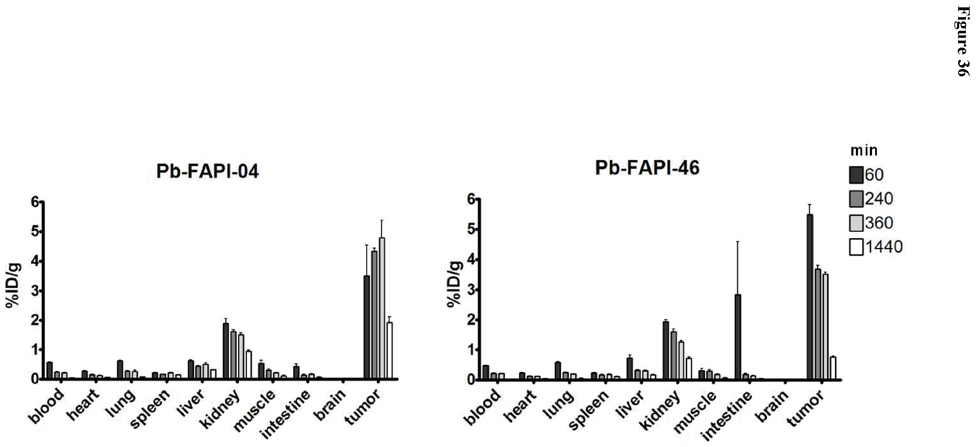

Pb-203 radiolabeled FAPI derivatives (FAPI-04, -32, -46 and FAPI-04tcmc) show comparable cell binding to HT-1080-FAP cells with FAPI-32 and FAPI-04tcmc reaching the highest binding values after 60 min of incubation (26.93 ± 0.846 and 21.62 ± 0.61 %ID/1 mio cells, Figure 34A). While FAPI-32 is rapidly eliminated from the tumor cells upon initial binding (t_{½} = 2 h), FAPI-04tcmc demonstrates considerably slower cell efflux (t_{½} = 7 h) but also the lowest FAP affinity as shown by the competition assay (IC₅₀ = 5.7 µM, Figure 34C). On this account, FAPI-04 and FAPI-46, characterized by optimal half-lives and IC₅₀-values, were selected for further analysis *in vivo.* As shown in Figure 35, both compounds get continuously enriched within the tumor while showing only negligibly low binding to healthy tissue. The scintigraphic findings are confirmed in a biodistribution study, where both radiotracers demonstrate a robust tumor uptake, overall low organ activities as well as rapid renal excretion (Figure 36).

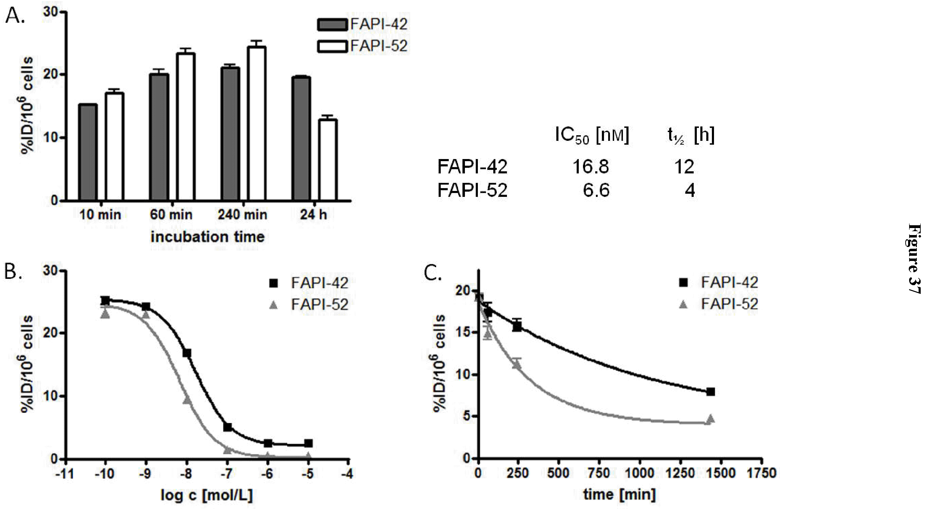

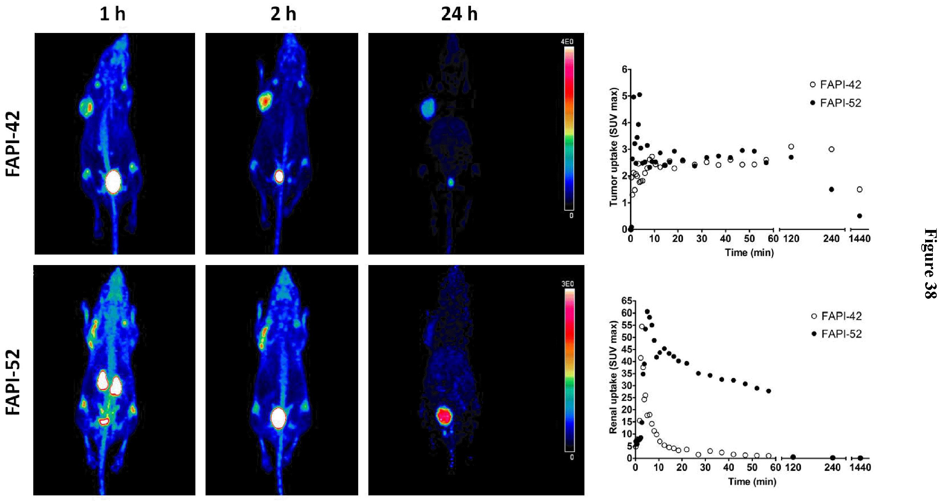

To enable radioactive labeling using Cu-64, the NOTA-derivatives FAPI-42 and FAPI-52 have been developed and characterized with respect to target affinity, specificity and pharmacokinetics. As shown in Figure 37, both tracers show a robust binding to HT-1080-FAP cells up to 24 h of incubation with similar IC₅₀-values in the lower nanomolar range (Figure 37A, B). Yet, FAPI-42 gets eliminated significantly slower than FAPI-52, resulting in a calculated *in vitro*-half-life of 12 h (Figure 37C). These results are confirmed by small animal imaging of HT-1080-FAP xenografted mice. As shown in Figure 38, both compounds demonstrate a robust tumor uptake as well as a rapid clearance from the blood stream *in vivo.* Notably, renal excretion of FAPI-42 occurs significantly faster as compared to FAPI-52, while its tumor activity remains slightly higher in the course of 2 to 24 h after administration.

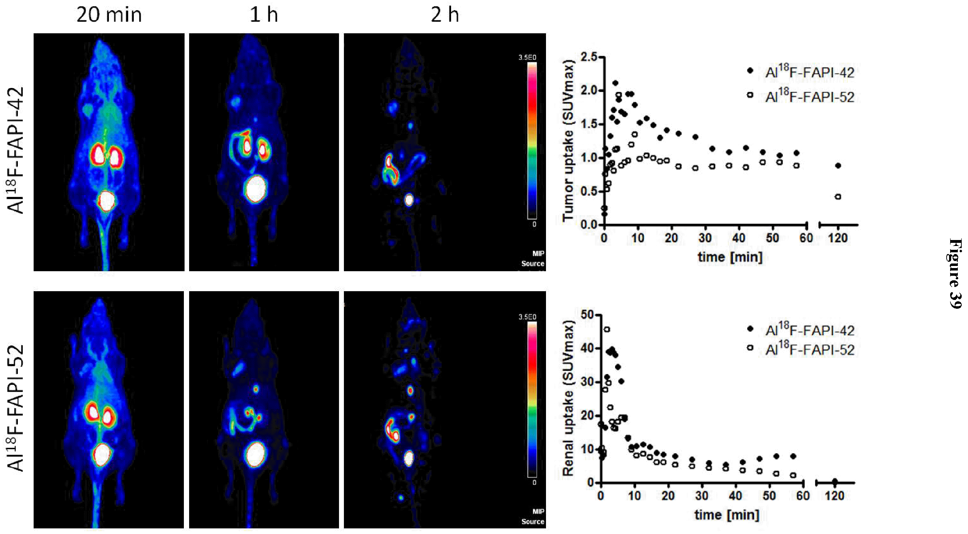

The NOTA-derivatives FAPI-42 and FAPI-52 have been deployed for the formation of aluminum fluoride complexes to allow imaging with F-18. As shown in Figure 39, both compounds demonstrate a rapid tumor uptake in small animal imaging of HT-1080-FAP xenografted mice. Although both compounds are mainly excreted by the renal pathway, a biliary elimination is also observed. While the renal excretion is faster for FAPI-52 the higher tumor accumulation, longer tumor retention and the lower proportion of the biliary pathway are in favor of FAPI-42.

### References

1 Loktev, A. et al. A new method for tumor imaging by targeting cancer associated fibroblasts. Journal of nuclear medicine : official publication, Society of Nuclear Medicine, doi:10.2967/jnumed.118.210435 (2018).
2 Lindner, T. et al. Development of quinoline based theranostic ligands for the targeting of fibroblast activation protein. Journal of nuclear medicine : official publication, Society of Nuclear Medicine, doi:10.2967/jnumed.118.210443 (2018).
3 Giesel, F. et al. FAPI-PET/CT: biodistribution and preliminary dosimetry estimate of two DOTA-containing FAP-targeting agents in patients with various cancers. Journal of nuclear medicine : official publication, Society of Nuclear Medicine, doi:10.2967/jnumed.118.215913 (2018).

### Example 6: FAPI characterization in vitro and in vivo

### Preclinical data

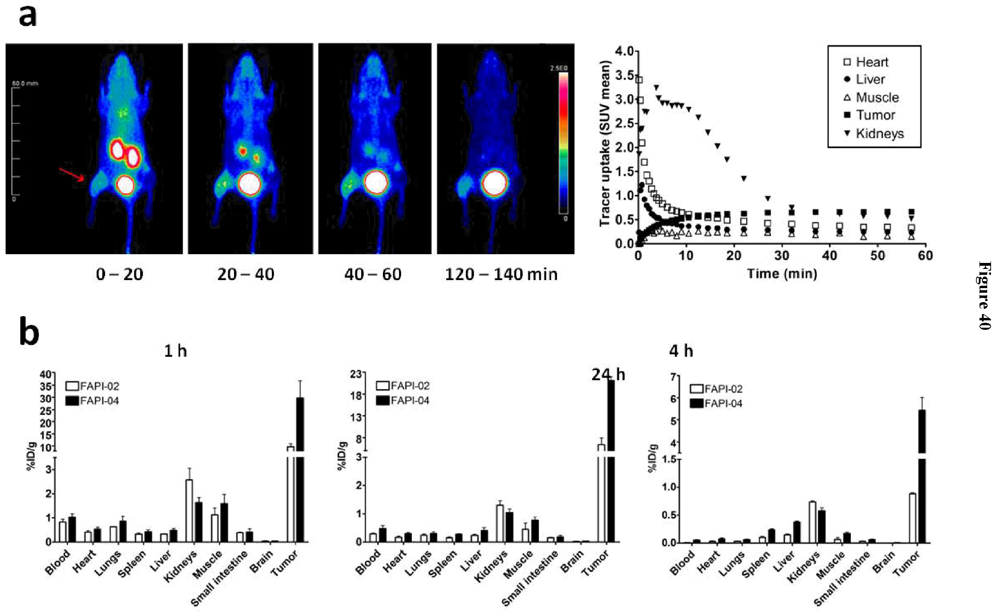

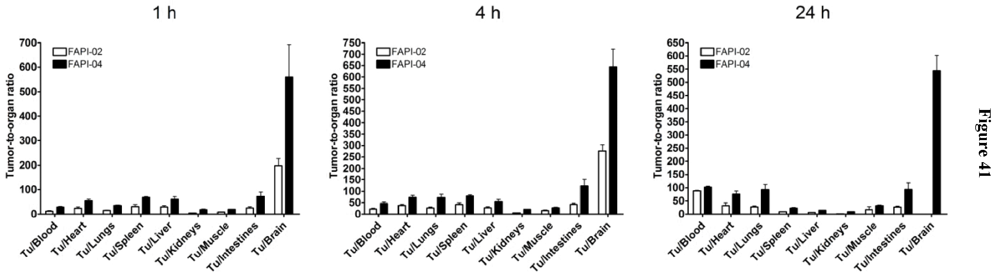

With the objective of selectively targeting FAP-positive brain tumors, initial experiments were performed in tumor bearing mice using the human glioblastoma xenograft model U87MG. Tumor accumulation and organ distribution of radiolabeled FAPI-02 and -04 were analyzed by small animal PET imaging as well as in a biodistribution study. As shown in Fig. 40 and 41, both FAPI-02 and -04 demonstrate rapid tumor uptake and negligibly low activities in healthy organs and the blood.

### Clinical data

According to the WHO classification of 2016, gliomas are subdivided in IDH-wildtype gliomas WHO grade I-IV and IDH-mutant gliomas WHO grade II-IV. The most frequent WHO grade IV gliomas are glioblastomas.

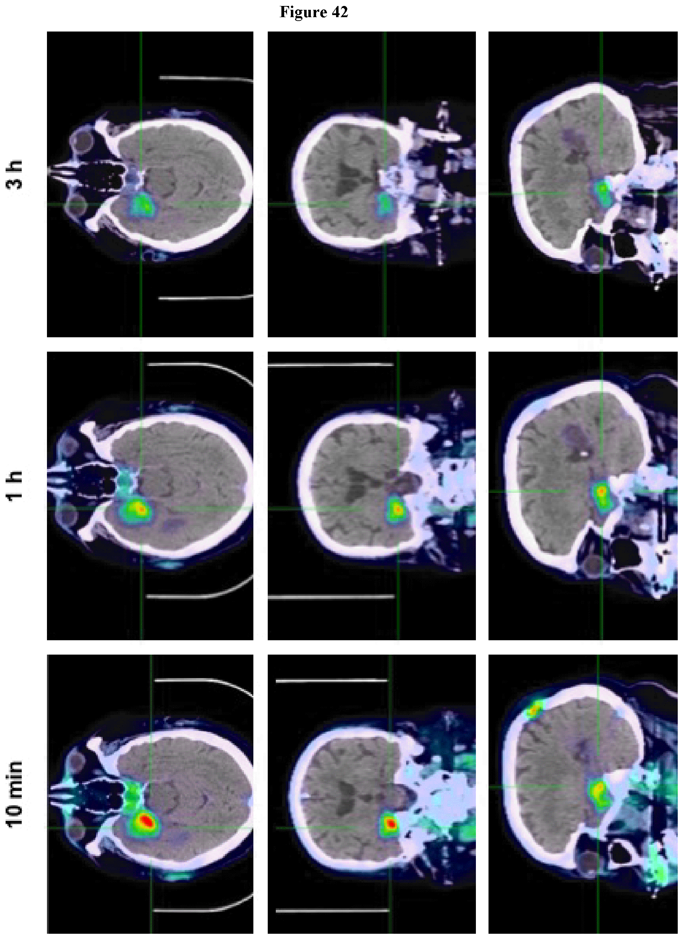

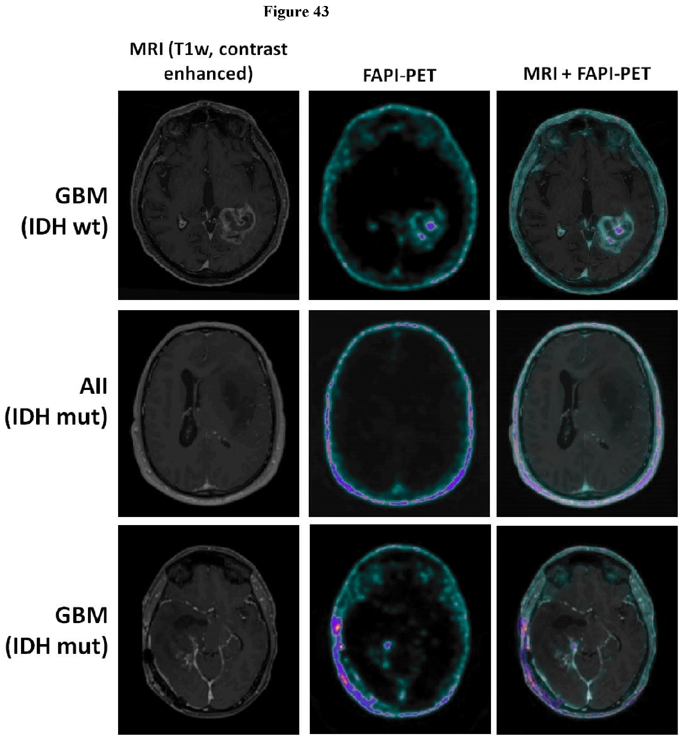

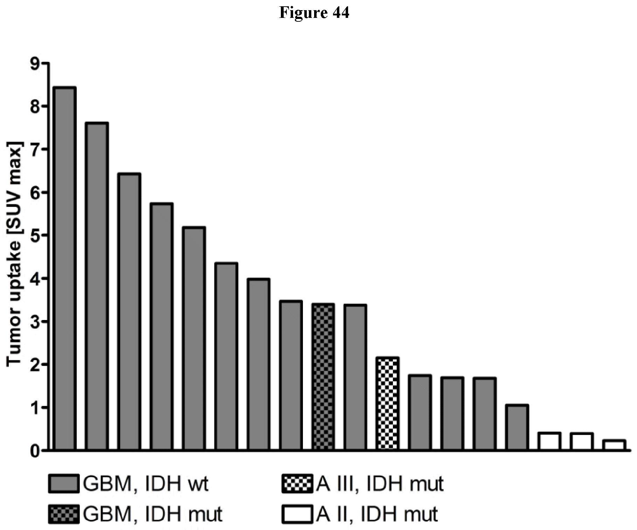

Clinical PET-imaging was performed in 18 glioma patients (5 IDH mutant gliomas, 13 IDH-wildtype glioblastomas; see Table 10). As shown in Fig. 42-44, IDH-wildtype glioblastomas and grade III/IV, but not grade II IDH-mutant gliomas showed elevated tracer uptake. In glioblastomas, spots with increased uptake in projection on contrast enhancing areas were observed.

### Conclusion

Increased tracer uptake in IDH-wildtype glioblastomas and high-grade IDH-mutant astrocytomas, but not in diffuse astrocytomas may allow non-invasive distinction between low-grade IDH-mutant and high-grade gliomas and be useful for follow-up studies. The heterogeneous tracer uptake in glioblastomas may be helpful for biopsy planning.

**Table 10: Patient characteristics**

| Patient Number | Age (years) | IDH status | Diagnosis | WHO Grade | Localization | Biopsy Surgery | Pretreatment |
|---|---|---|---|---|---|---|---|
| 1 | 57 | wildtype | Glioblastoma | IV | parietal right | Biopsy | Radio-chemotherapy |
| 2 | 65 | wildtype | Glioblastoma | IV | bifrontal | Biopsy | none |
| 3 | 66 | wildtype | Glioblastoma | IV | parieto-temporal left | Biopsy | none |
| 4 | 64 | wildtype | Glioblastoma | IV | temporal right | Resection | Radio-chemotherapy |
| 5 | 58 | wildtype | Glioblastoma | IV | Splenium | Biopsy | none |
| 6 | 48 | wildtype | Glioblastoma | IV | fronto-temporo-parietal left | Resection | Radio-chemotherapy |
| 7 | 20 | wildtype | Glioblastoma | IV | Thalamus / temporal right | Biopsy | none |
| 8 | 54 | wildtype | Glioblastoma | IV | temporal left | Resection | none |
| 9 | 56 | wildtype | Glioblastoma | IV | basal ganglia | Partial Resection | none |
| 10 | 61 | wildtype | Glioblastoma | IV | temporal right | Resection | none |
| 11 | 86 | wildtype | Glioblastoma | IV | Corona radiata left | Biopsy | Chemotherapy |
| 12 | 68 | wildtype | Glioblastoma | IV | Corpus callosum | Biopsy | none |
| 13 | 71 | wildtype | Glioblastoma | IV | temporal left | Biopsy | none |
| 14 | 29 | IDH1 R132H Mutation | Glioblastoma | IV | temporo-parietal right | Resection | none |
| 15 | 47 | IDH1 R132H Mutation | Anaplastic astrocytoma | III | frontal right | Resection | none |
| 16 | 47 | IDH1 R132H Mutation | Diffuse astrocytoma | II | parietal left | Biopsy | none |
| 17 | 47 | IDH1 R132C Mutation | Diffuse astrocytoma | II | frontal right | Resection | none |
| 18 | 43 | IDH1 R132H Mutation | Diffuse astrocytoma | II | fronto-temporal left | Biopsy | Chemotherapy |

### Example 7: FAPI characterization in vitro and in vivo

### Reuptake experiments

For reuptake experiments, ¹⁷⁷Lu-labeled FAPI-04 and -46 (5 MBq/nmol in DMEM) were added to HT-1080-FAP cells and incubated for 60 min at 4 and 37°C, respectively. Radioactive medium was removed and cells were washed twice with phosphate-buffered saline (PBS) pH 7.4. Subsequently, non-radioactive medium with and without unlabeled FAPI (1 µM) was added for time intervals ranging from 10 min to 6 h. The cells were washed twice with PBS pH 7.4. To remove the surface bound activity, the cells were incubated with glycine-HCl (1 M in PBS, pH 2.2) for 10 min at room temperature. After washing twice with ice-cold PBS, the cells were lysed with 1.4 mL of lysis buffer (0.3 M NaOH, 0.2% SDS) to determine the internalized fraction. For the cells incubated at 4 °C, all washing and elution steps were carried out using ice-cold buffers. The radioactivity was measured using a γ-counter (Packard Cobra II), normalized to 1 mio cells and calculated as percentage of applied dose (%AD; see Fig. 47).

### Enzyme inhibition assay

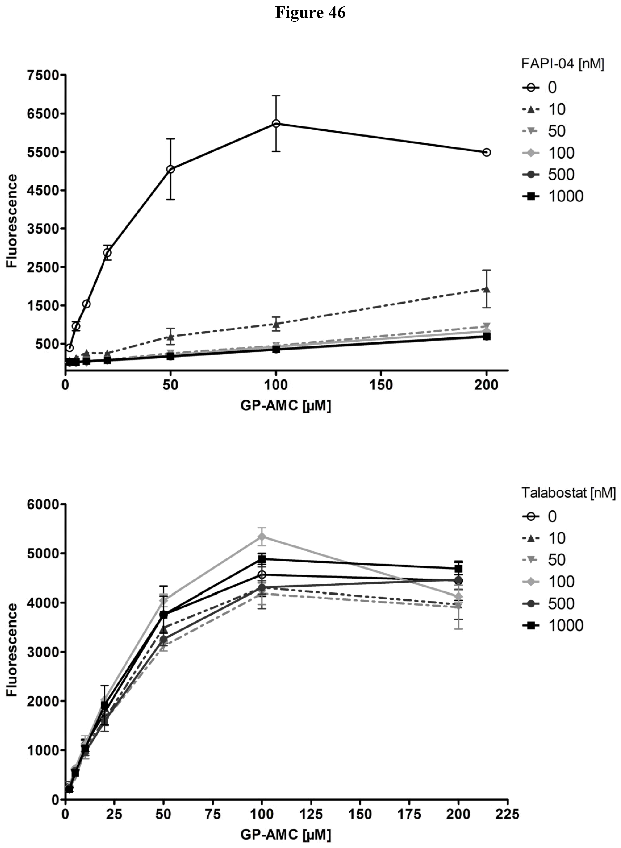

To determine potential inhibitory effects of FAPI-04 on the enzymatic FAP activity, enzyme inhibition assays were performed using recombinant human FAP protein (1 pmol/well) in a 48-well plate. After incubation of FAPI-04 or Talabostat (0 - 1000 nM/well) with human FAP for 30 min at 37 °C, the fluorogenic FAP substrate Z-GP-AMC was added to a final concentration of 0 - 200 µM/well and incubated for 60 min at 37°C. The enzymatic activity of FAP was determined by measuring the fluorescence intensity of the reaction product AMC at 360/460 nm using the SpectraMax M2 Plate Reader (Molecular Devices, San José, USA) (see Fig. 46).

### Multiple administration of FAPI-04 to HT-1080-FAP tumor bearing mice

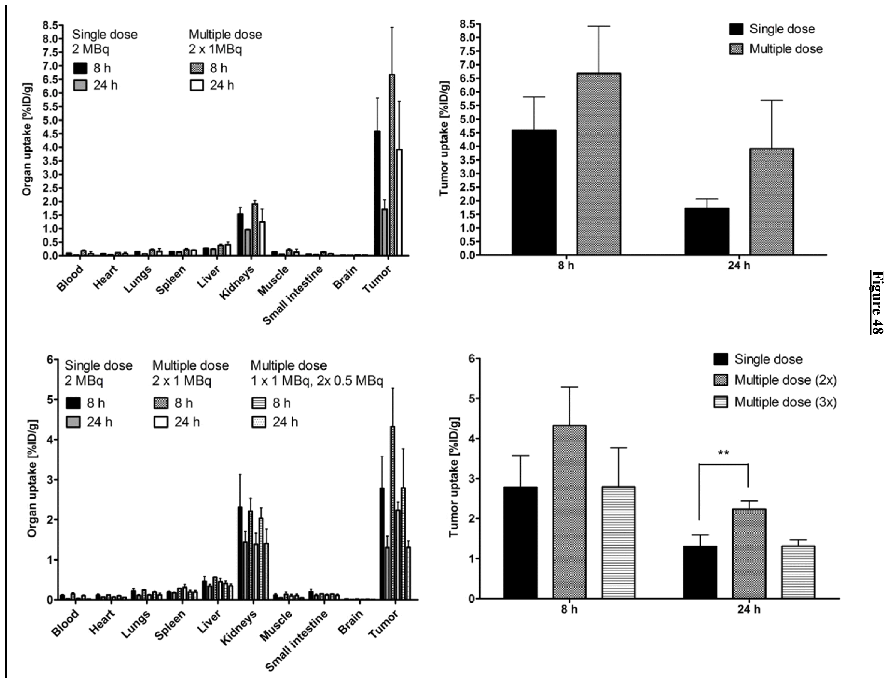

For biodistribution experiments, 8 week old BALB/c *nu*/*nu* mice (Charles River) were subcutaneously inoculated into the right trunk with 5 mio HT-1080-FAP cells, respectively. When the size of the tumor reached approximately 1 cm³, the radiolabeled compound was injected via the tail vein. The first group of animals was administered a single dose of ¹⁷⁷Lu-FAPI-04 (2 MBq per animal), whereas the second group received two doses of 1 MBq each, with the second dose given 4 h after the first injection. The third group was administered three doses in total, with an initial dose of 1 MBq per mouse, followed by 0.5 MBq 2 h and additional 0.5 MBq 4 h after the first injection. The animals (*n* = 3 for each time point) were sacrificed 8 and 24 h after the first injection. The distributed radioactivity was measured in all dissected organs and in blood using a γ-counter (Cobra Autogamma, Packard). The values are expressed as percentage of injected dose per gram of tissue (%ID/g) (see Fig. 48).

### Example 8: FAPI characterization in vitro and in vivo

### Experimental Procedures and Clinical Evaluation

All *in vitro* and *in vivo* experiments as well as the clinical evaluation of the FAPI derivatives have been performed as described in the initial document and according to Loktev *et al.* ¹ and Lindner *et al.* ²

### Results

### In vitro Characterization of F-18-FAPI Derivatives

All experiments were carried out analogous to FAPI-42 (AlF-18 labeling) or FAPI-72 (F-18 nicotinamide labeling).

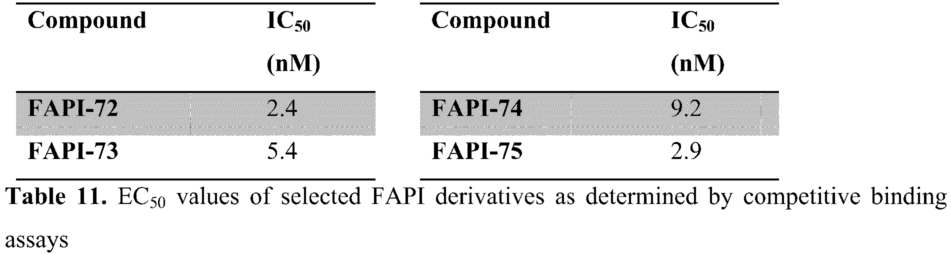

### Determination of blood pool clearance

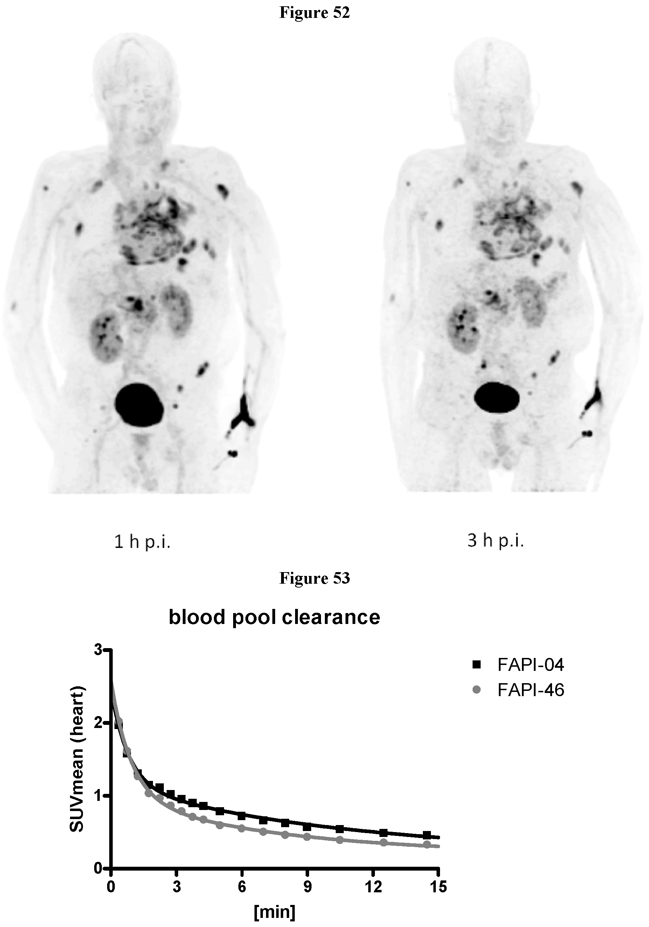

To estimate the rate of clearance of the compound, half-life times were calculated by a presumed two phase exponential decay from the SUVmean values (0.375-60 min) of the heart as representation of the blood pool. All selected compounds were cleared very fast with half-life times below 10 min. The calculated plateau values, which were higher for Ga-68 labeled FAPI-13, -21, -36 and AlF-18 labeled FAPI-74 theoretically correspond to a higher fraction of compound which is not cleared due to unspecific binding or by remaining in circulating (Table 12). As an example for the fast clearance the time activity curves for FAPI-04 and -46 between 0 and 15 min are shown in Figure 53.

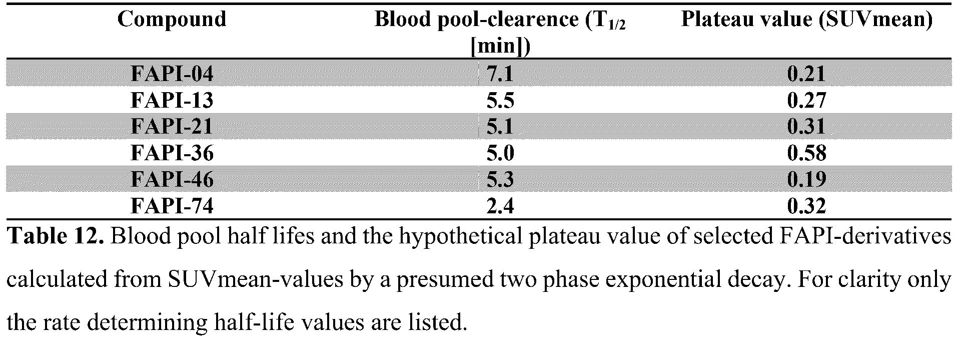

### Small-Animal Imaging of F-18-FAPI Derivatives in Tumor-Bearing Mice

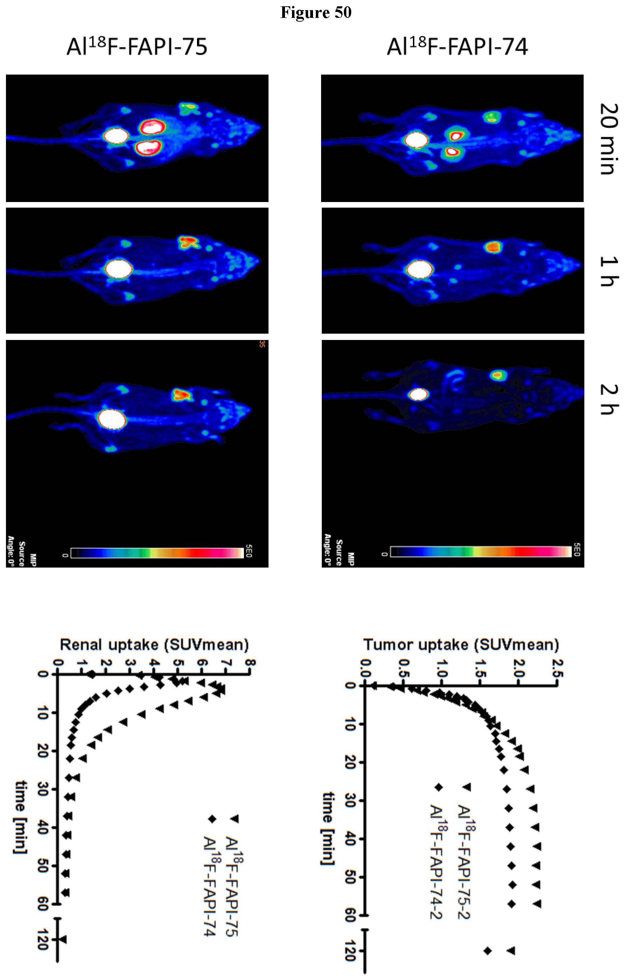

Based on these findings, small animal PET-imaging was performed using F-18-labeled NOTA- and F-18-nicotinamide-labeled FAPI derivatives up to 140 min after i.v. administration of the radiotracers in HT-1080-FAP tumor-bearing mice. The F-18-nicotinamide derivatives FAPI-72, -73 and -77 showed an unfavorable accumulation in the liver as well as a biliary excretion, while FAPI-78 was renally excreted but showed no tumor uptake. In case of the AlF-18 labeled NOTA derivatives FAPI-74 and -75 a high target specificity and fast clearance was observed, resulting in high contrast images, which enable excellent visualization of the FAP-positive tumors (Figure 50).

### Organ Distribution of F-18-FAPI Derivatives in Tumor-Bearing Mice

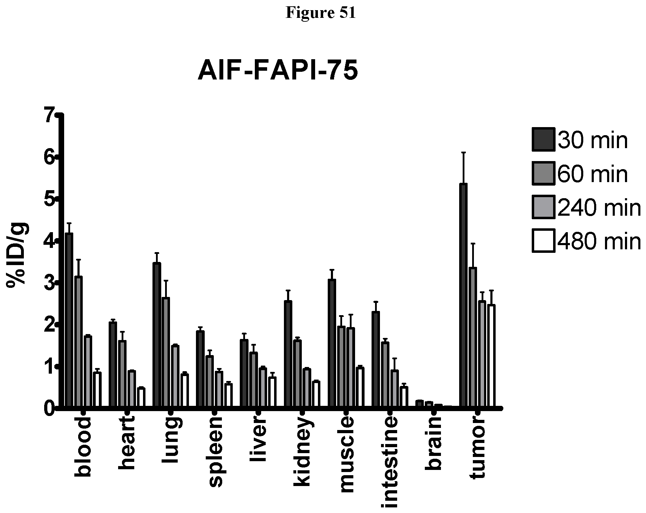

For analysis of pharmacokinetic profile as well as tumor uptake *in vivo,* AlF-18-labeled FAPI-75 was administered i.v. to HT-1080-FAP tumor-bearing mice. Organ distribution of the radiolabeled compound was determined *ex vivo* in the blood, healthy tissues and the tumor. As shown in Figure 51, the compounds demonstrates high tumor uptake, although in comparison to Ga-68 labeled DOTA derivatives a higher accumulation in healthy tissue is observed, while performance in PET imaging was equal.

### Items

The following items represent preferred embodiments of the present invention.
1. A compound of Formula (I) 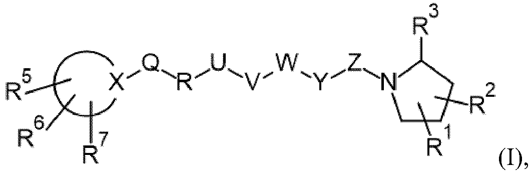 wherein
   Q, R, U, V, W, Y, Z are individually present or absent under the proviso that at least three of Q, R, U, V, W, Y, Z are present;
   Q, R, U, V, W, Y, Z are independently selected form the group consisting of O, CH₂, NR⁴, C=O, C=S, C=NR⁴, HCR⁴ and R⁴CR⁴, with the proviso that two Os are not directly adjacent to each other;
   R¹ and R² are independently selected from the group consisting of -H, -OH, halo, C₁₋₆-alkyl, -O-C₁₋₆-alkyl, S-C₁₋₆-alkyl;
   R³ is selected from the group consisting of -H, -CN , -B(OH)₂, -C(O) -alkyl, -C(O) -aryl-, -C=C-C(O) -aryl, -C=C-S(O)₂-aryl, -CO₂H , -SO₃H , -SO₂NH₂,-PO₃H₂, and 5-tetrazolyl; R⁴ is selected from the group consisting of -H, -C₁₋₆-alkyl, -O-C₁₋₆-alkyl, -S-C₁₋₆-alkyl, alkenyl, heteroalkenyl, cycloalkenyl, cycloheteroalkenyl, alkynyl, aryl, and -C₁₋₆-aralkyl, each of said -C₁₋₆-alkyl being optionally substituted with from 1 to 3 substituents selected from -OH, oxo, halo and optionally connected to Q, R, U, V, W, Y or Z;
   R⁵ is selected from the group consisting of -H, halo and C₁₋₆-alkyl;
   R⁶, and R⁷ are independently selected from the group consisting of -H, 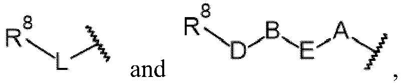 under the proviso that R⁶ and R⁷ are not at the same time H,
   wherein L is a linker,
   wherein D, A, E, and B are individually present or absent, preferably wherein at least A, E, and B are present, wherein when present:
      D is a linker;
      A is selected from the group consisting of NR⁴, O, S, and CH₂;
      E is selected from the group consisting of
         C₁₋₆-alkyl, 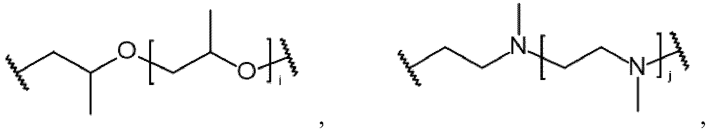 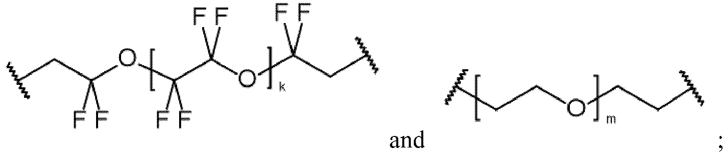
      wherein i is 1, 2, or 3;
      wherein j is 1, 2, or 3;
      wherein k is 1, 2, or 3;
      wherein m is 1, 2, or 3; A and E together form a group selected from: a cycloalkyl, heterocycloalkyl, aryl and heteroaryl, preferably heterocycloalkyl, wherein A and E can be mono-, bi- and multicyclic, preferably monocyclic; each A and E being optionally substituted with 1 to 4 substituents selected from -H, -C₁₋₆-alkyl, -O-C₁₋₆-alkyl, -S-C₁₋₆-alkyl, alkenyl, heteroalkenyl, cycloalkenyl, cycloheteroalkenyl, alkynyl, aryl, and -C₁₋₆-aralkyl, each of said -C₁₋₆-alkyl being optionally substituted with from 1 to 3 substituents selected from -OH, oxo, halo; and optionally connected to A, B, D, E or 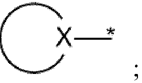 B is selected from the group consisting of S, NR⁴, NR⁴-O, NR⁴-C₁₋₆-alkyl, NR⁴-C₁₋₆-alkyl-NR⁴, and a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein NR⁴-C₁₋₆-alkyl-NR⁴ and the N-containing heterocycle is substituted with 1 to 3 substituents selected the group consisting of C₁₋₆-alkyl, aryl, C₁₋₆-aralkyl; and;
      R⁸ is selected from the group consisting of radioactive moiety, chelating agent, fluorescent dye, a contrast agent and combinations thereof; 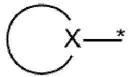 is a 1-naphtyl moiety or a 5 to 10- membered N-containing aromatic or non-aromatic mono- or bicyclic heterocycle, wherein there are 2 ring atoms between the N atom and X; said heterocycle optionally further comprising 1, 2 or 3 heteroatoms selected from O, N and S; and X is a C atom;
      or a pharmaceutically acceptable tautomer, racemate, hydrate, solvate, or salt thereof.
2. The compound of item 1, wherein
   (i) Q, R, U are CH₂ and are individually present or absent;
      V is CH₂, C=O, C=S or C=NR⁴;
      W is NR⁴;
      Y is HCR⁴; and
      Z is C=O, C=S or C=NR⁴; and/or
   (ii) Q and R are absent;
      U is CH₂ and is present or absent;
      R¹ and R² are independently selected from the group consisting of -H and halo;
      R³ is selected from the group consisting of -H, -CN, and -B(OH)₂;
      R⁴ is selected from the group consisting of -H and -C₁₋₆-alkyl, wherein the -C₁₋₆-alkyl is optionally substituted with from 1 to 3 substituents selected from -OH.
3. The compound of item 1 or 2, wherein 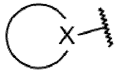 is selected from the group consisting of 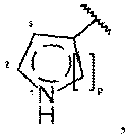 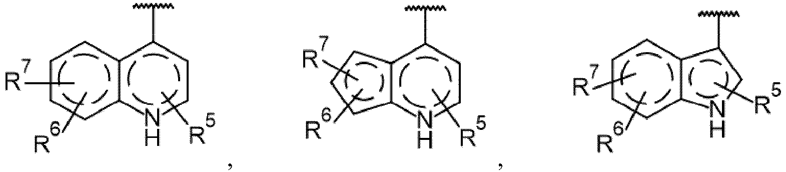 and 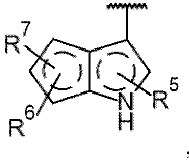 optionally further comprising 1 or 2 heteroatoms selected from O, N, and S.
4. The compound of any of the preceding items, wherein 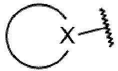 is selected from the group consisting of 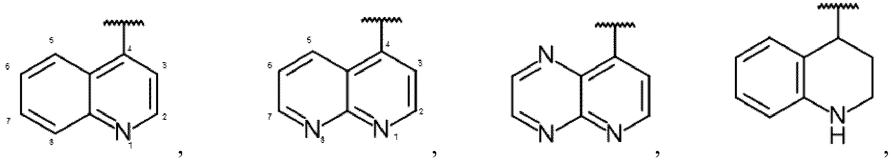 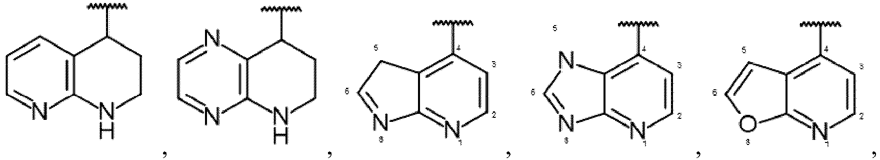 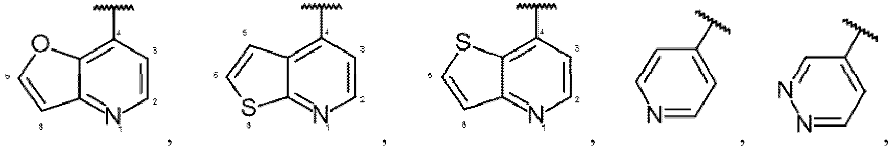 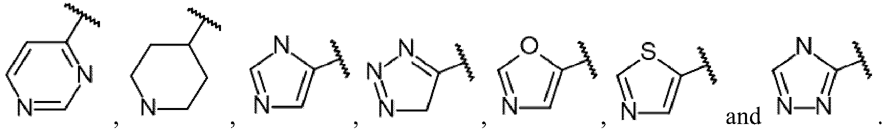
5. The compound of any of the preceding items, wherein
   R⁵ and R⁶ are H;
   R⁷ is 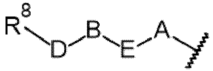 , wherein
   D is absent;
   A is O, S, CH₂, NH, NCH₃;
   E is C₁₋₆-alkyl or 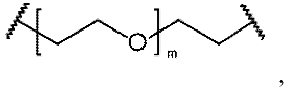 wherein m is 1, 2, or 3;
   A and E together form a group selected from: 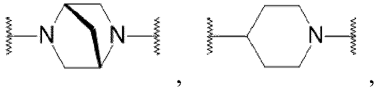 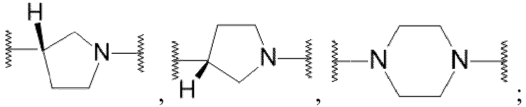
   B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl.
6. The compound of any of the preceding items, wherein
   (i) the N-containing heterocycle comprised in B is an aromatic or non-aromatic monocyclic heterocycle: 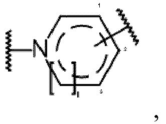 wherein
      the heterocycle optionally further comprises 1 or 2 heteroatoms selected form O, N and S, optionally further comprises 1 nitrogen; 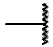
      is attached to position 1, 2, or 3, preferably to position 2;
      1 is 1 or 2;
      optionally wherein the N-containing heterocycle is substituted with a C₁₋₆-alkyl; and/or
   (ii) the N-containing heterocycle comprised in B is selected from the group consisting of: 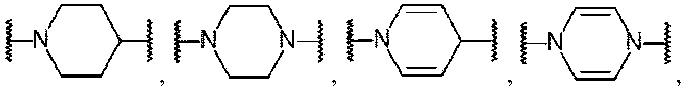 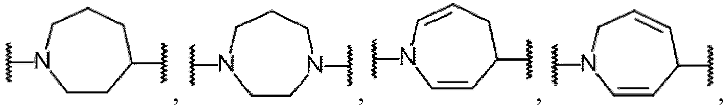 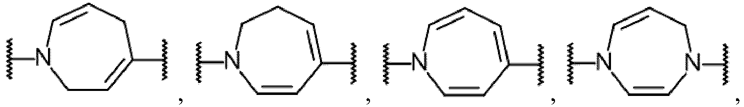 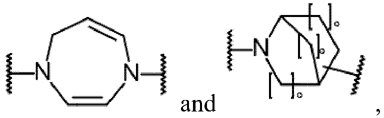
      optionally wherein the N-containing heterocycle is substituted with a C₁₋₆-alkyl;
      wherein if the N-containing heterocycle comprised in B is 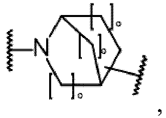 the heterocycle optionally further comprises 1 or 2 heteroatoms selected from O, N and S, optionally further comprises 1 nitrogen, optionally compromises one or more (e.g. amino acid derived) side chains; 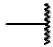
      is attached to position 1, 2, or 3, preferably to position 2;
      o is 1 or 2,
      preferably, if the N-containing heterocycle comprised in B is 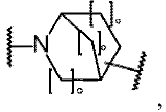 the N-containing heterocycle comprised in B is 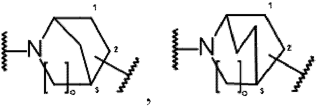 or 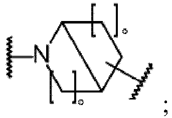 more preferably, if the N-containing heterocycle comprised in
      B is 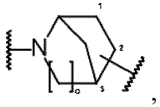 the N-containing heterocycle comprised in B is 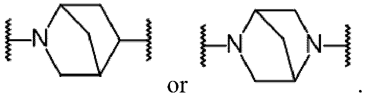
7. The compound of any of the preceding items, wherein
   Q, R, U are absent;
   V is C=O;
   W is NH;
   Y is CH₂;
   Z is C=O;
   R¹ and R² are independently selected from the group consisting of -H and halo;
   R³ is -CN;
   R⁵ and R⁶ are H;
   R⁷ is 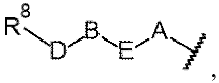 wherein
   D is absent;
   A is O, S, CH₂, NH, NCH₃;
   E is C₁₋₆-alkyl or 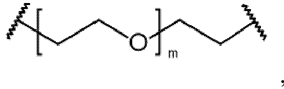 wherein m is 1, 2, or 3; or
   A and E together form a group selected from: 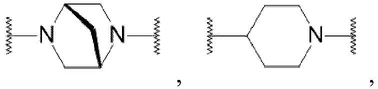 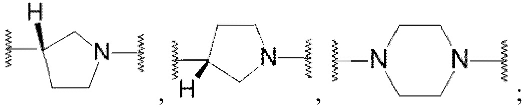
   B is NH-C₁₋₆-alkyl, 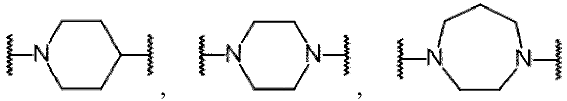 or 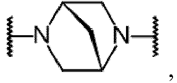 optionally B is substituted with a C₁₋₃ alkyl; and 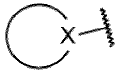 is 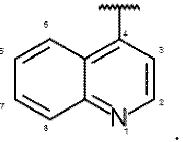
8. The compound of any of the preceding items, wherein C₁₋₆-alkyl is selected from the group consisting of methyl, ethyl, propyl, i-propyl, butyl, sec-butyl, tert-butyl, pentyl and hexyl, and/or
   wherein C₁₋₆-aralkyl is selected from the group consisting of benzyl, phenyl-ethyl, phenyl-propyl, and phenyl-butyl.
9. The compound of any of the preceding items, wherein R⁸ is a radioactive moiety, wherein the radioactive moiety is a fluorescent isotope, a radioisotope, a radioactive drug or combinations thereof, preferably wherein the radioactive moiety is selected from the group consisting of alpha radiation emitting isotopes, beta radiation emitting isotopes, gamma radiation emitting isotopes, Auger electron emitting isotopes, X-ray emitting isotopes, fluorescence emitting isotopes, such as ¹¹C, ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁸Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ²²⁷Th and ¹⁹⁹Ag, preferably ¹⁸F, ⁶⁴Cu, ⁶⁸Ga, ⁹⁰Y, ^{99m}Tc, ¹⁵³Sm, ¹⁷⁷Lu, ¹⁸⁸Re.
10. The compound of any of items 1 to 8, wherein R⁸ is a fluorescent dye select from the group consisting of the following classes of fluorescent dyes: Xanthens, Acridines, Oxazines, Cynines, Styryl dyes, Coumarines, Porphines, Metal-Ligand-Complexes, Fluorescent proteins, Nanocrystals, Perylenes, Boron-dipyrromethenes and Phtalocyanines as well as conjugates and combinations of these classes of dyes.
11. The compound of any of items 1 to 8, wherein R⁸ is a chelating agent which forms a complex with divalent or trivalent metal cations, preferably wherein the chelating agent is selected from the group consisting of 1,4,7,10-tetraazacyclododecane-N,N',N,N'-tetraacetic acid (DOTA), ethylenediaminetetraacetic acid (EDTA), 1,4,7-triazacyclononane-1,4,7-triacetic acid (NOTA), triethylenetetramine (TETA), iminodiacetic acid, diethylenetriamine-N,N,N',N',N"-pentaacetic acid (DTPA), bis-(carboxymethylimidazole)glycine and 6-Hydrazinopyridine-3-carboxylic acid (HYNIC).
12. The compound of any of items 1 to 8, wherein R⁸ is a contrast agent which comprises or consists of a paramagnetic agent, preferably, wherein the paramagnetic agent comprises or consists of paramagnetic nanoparticles.
13. Pharmaceutical composition comprising or consisting of at least one compound according to any of items 1 to 12; and, optionally, a pharmaceutically acceptable carrier and/or excipient.
14. The compound of any of items 1 to 12 or the pharmaceutical composition of item 13 for use in the diagnosis or treatment of a disease characterized by overexpression of fibroblast activation protein (FAP) in an animal or a human subject, preferably wherein the disease characterized by overexpression of fibroblast activation protein (FAP) is selected from the group consisting of cancer, chronic inflammation, atherosclerosis, fibrosis, tissue remodeling and keloid disorder, preferably wherein the cancer is selected from the group consisting of breast cancer, pancreatic cancer, small intestine cancer, colon cancer, rectal cancer, lung cancer, head and neck cancer, ovarian cancer, hepatocellular carcinoma, esophageal cancer, hypopharynx cancer, nasopharynx cancer, larynx cancer, myeloma cells, bladder cancer, cholangiocellular carcinoma, clear cell renal carcinoma, neuroendocrine tumor, oncogenic osteomalacia, sarcoma, CUP (carcinoma of unknown primary), thymus carcinoma, desmoid tumors, glioma, astrocytoma, cervix carcinoma and prostate cancer.
15. A kit comprising or consisting of the compound of any of items 1 to 12 or the pharmaceutical composition of item 13 and instructions for the diagnosis or treatment of a disease.

The following items also represent preferred embodiments of the present invention.
1. A compound of Formula (I) 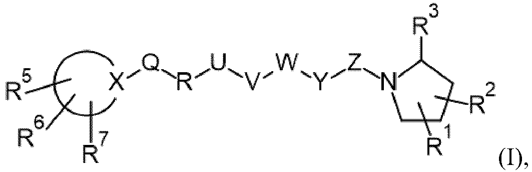 wherein
   Q, R, U, V, W, Y, Z are individually present or absent under the proviso that at least three of Q, R, U, V, W, Y, Z are present;
   Q, R, U, V, W, Y, Z are independently selected form the group consisting of O, CH₂, NR⁴, C=O, C=S, C=NR⁴, HCR⁴ and R⁴CR⁴, with the proviso that two Os are not directly adjacent to each other;
   R¹ and R² are independently selected from the group consisting of -H, -OH, halo, C₁₋₆-alkyl, -O-C₁₋₆-alkyl, S-C₁₋₆-alkyl;
   R³ is selected from the group consisting of -H, -CN , -B(OH)₂, -C(O) -alkyl, -C(O) -aryl-, -C=C-C(O) -aryl, -C=C-S(O)₂-aryl, -CO₂H , -SO₃H , -SO₂NH₂,-PO₃H₂, and 5-tetrazolyl; R⁴ is selected from the group consisting of -H, -C₁₋₆-alkyl, -O-C₁₋₆-alkyl, -S-C₁₋₆-alkyl, alkenyl, heteroalkenyl, cycloalkenyl, cycloheteroalkenyl, alkynyl, aryl, and -C₁₋₆-aralkyl, each of said -C₁₋₆-alkyl being optionally substituted with from 1 to 3 substituents selected from -OH, oxo, halo and optionally connected to Q, R, U, V, W, Y or Z;
   R⁵ is selected from the group consisting of -H, halo and C₁₋₆-alkyl;
   R⁶, and R⁷ are independently selected from the group consisting of -H, 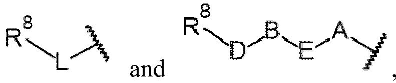 under the proviso that R⁶ and R⁷ are not at the same time H,
   wherein L is a linker,
   wherein D, A, E, and B are individually present or absent, preferably wherein at least A, E, and B are present, wherein when present:
      D is a linker;
      A is selected from the group consisting of NR⁴, O, S, and CH₂;
      E is selected from the group consisting of
         C₁₋₆-alkyl, 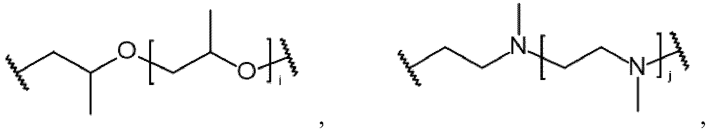 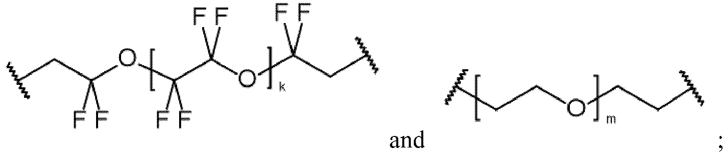
      wherein i is 1, 2, or 3;
      wherein j is 1, 2, or 3;
      wherein k is 1, 2, or 3;
      wherein m is 1, 2, or 3; B is selected from the group consisting of S, NR⁴, NR⁴-O, NR⁴-C₁₋₆-alkyl, NR⁴-C₁₋₆-alkyl-NR⁴, and a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein NR⁴-C₁₋₆-alkyl-NR⁴ and the N-containing heterocycle is substituted with 1 to 3 substituents selected the group consisting of C₁₋₆-alkyl, aryl, C₁₋₆-aralkyl; and;
      R⁸ is selected from the group consisting of radioactive moiety, chelating agent, fluorescent dye, a contrast agent and combinations thereof; 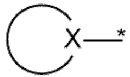
      is a 1-naphtyl moiety or a 5 to 10- membered N-containing aromatic or non-aromatic mono- or bicyclic heterocycle, wherein there are 2 ring atoms between the N atom and X; said heterocycle optionally further comprising 1, 2 or 3 heteroatoms selected from O, N and S; and X is a C atom;
      or a pharmaceutically acceptable tautomer, racemate, hydrate, solvate, or salt thereof.
2. The compound of item 1, wherein
   (i) Q, R, U are CH₂ and are individually present or absent;
      V is CH₂, C=O, C=S or C=NR⁴;
      W is NR⁴;
      Y is HCR⁴; and
      Z is C=O, C=S or C=NR⁴; and/or
   (ii) Q and R are absent;
      U is CH₂ and is present or absent;
      R¹ and R² are independently selected from the group consisting of -H and halo;
      R³ is selected from the group consisting of -H, -CN, and -B(OH)₂;
      R⁴ is selected from the group consisting of -H and -C₁₋₆-alkyl, wherein the -C₁₋₆-alkyl is optionally substituted with from 1 to 3 substituents selected from -OH.
3. The compound of item 1 or 2, wherein 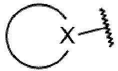 is selected from the group consisting of 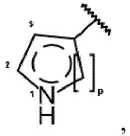 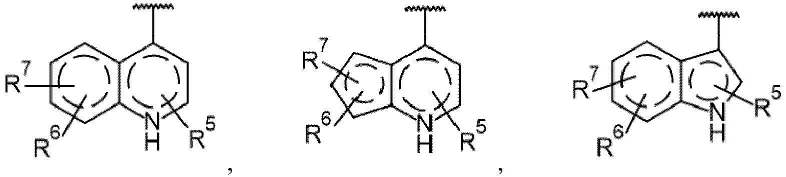 and 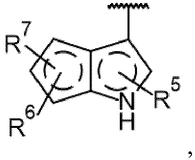 optionally further comprising 1 or 2 heteroatoms selected from O, N, and S.
4. The compound of any of the preceding items, wherein 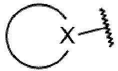 is selected from the group consisting of 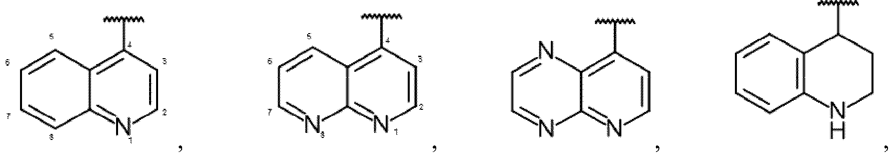 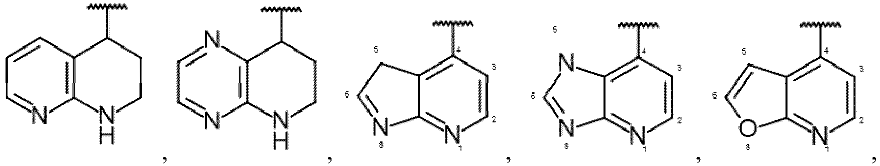 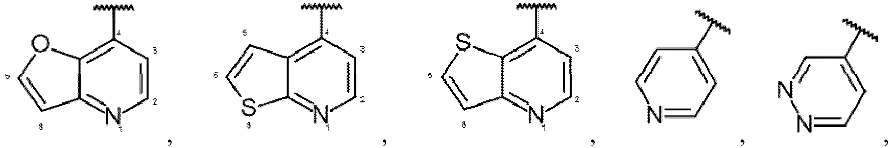 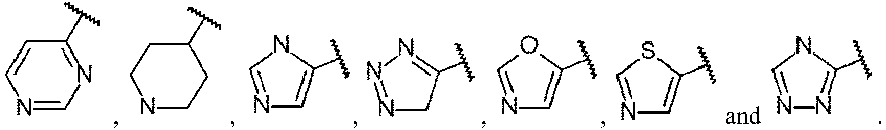
5. The compound of any of the preceding items, wherein
   R⁵ and R⁶ are H;
   R⁷ is 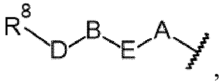 wherein
   D is absent;
   A is O;
   E is C₁₋₆-alkyl or 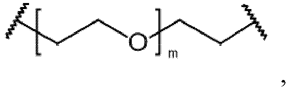 wherein m is 1, 2, or 3;
   B is NR⁴-C₁₋₆-alkyl or a 5- to 10-membered N-containing aromatic or non-aromatic mono- or bicyclic heterocycle, preferably further comprising 1 or 2 heteroatoms selected from O, N, and S, preferably further comprising 1 or 2 nitrogen atoms, preferably wherein the N-containing heterocycle is substituted with 1 to 3 substituents selected the group consisting of C₁₋₆-alkyl, aryl, C₁₋₆-aralkyl.
6. The compound of any of the preceding items, wherein
   (i) the N-containing heterocycle comprised in B is an aromatic or non-aromatic monocyclic heterocycle: 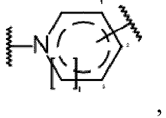 wherein
      the heterocycle optionally further comprises 1 or 2 heteroatoms selected form O, N and S, optionally further comprises 1 nitrogen; 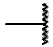
      is attached to position 1, 2, or 3, preferably to position 2;
      1 is 1 or 2; and/or
   (ii) the N-containing heterocycle comprised in B is selected from the group consisting of: 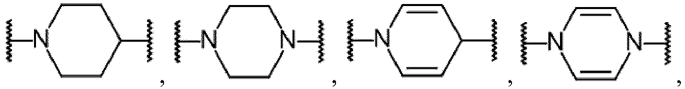 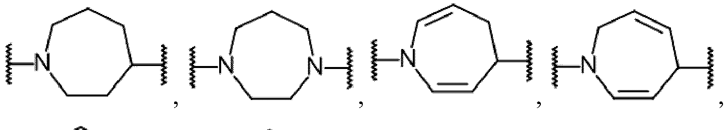 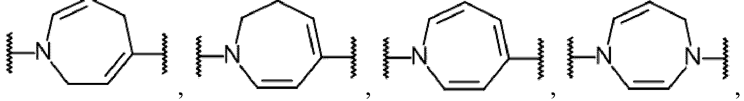 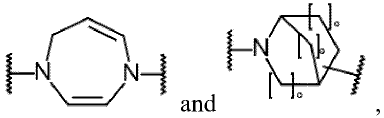
      wherein if the N-containing heterocycle comprised in B is 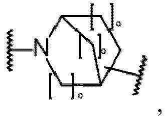 the heterocycle optionally further comprises 1 or 2 heteroatoms selected from O, N and S, optionally further comprises 1 nitrogen, optionally compromises one or more (e.g. amino acid derived) side chains; 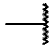
      is attached to position 1, 2, or 3, preferably to position 2;
      o is 1 or 2,
      preferably, if the N-containing heterocycle comprised in B is 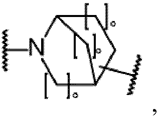 the N-containing heterocycle comprised in B is 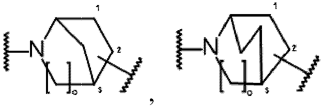 or 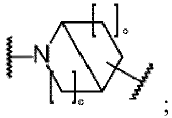 more preferably, if the N-containing heterocycle comprised in
      B is 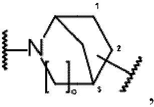 the N-containing heterocycle comprised in B is 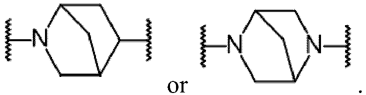
7. The compound of any of the preceding items, wherein
   Q, R, U are absent;
   V is C=O;
   W is NH;
   Y is CH₂;
   Z is C=O;
   R¹ and R² are independently selected from the group consisting of -H and halo;
   R³ is -CN;
   R⁵ and R⁶ are H;
   R⁷ is 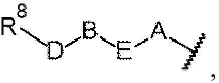 wherein
   D is absent;
   A is O;
   E is C₁₋₆-alkyl or 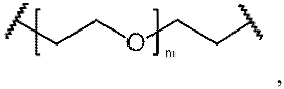 wherein m is 1, 2, or 3;
   B is NH-C₁₋₆-alkyl, 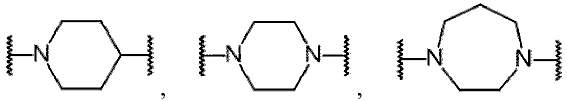 or 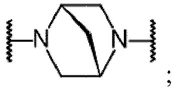 and 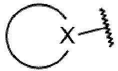 is 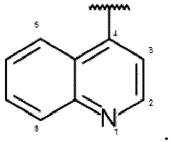
8. The compound of any of the preceding items, wherein C₁₋₆-alkyl is selected from the group consisting of methyl, ethyl, propyl, i-propyl, butyl, sec-butyl, tert-butyl, pentyl and hexyl, and/or
   wherein C₁₋₆-aralkyl is selected from the group consisting of benzyl, phenyl-ethyl, phenyl-propyl, and phenyl-butyl.
9. The compound of any of the preceding items, wherein R⁸ is a radioactive moiety, wherein the radioactive moiety is a fluorescent isotope, a radioisotope, a radioactive drug or combinations thereof, preferably wherein the radioactive moiety is selected from the group consisting of alpha radiation emitting isotopes, beta radiation emitting isotopes, gamma radiation emitting isotopes, Auger electron emitting isotopes, X-ray emitting isotopes, fluorescence emitting isotopes, such as ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁸Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ²²⁷Th and ¹⁹⁹Ag.
10. The compound of any of items 1 to 8, wherein R⁸ is a fluorescent dye select from the group consisting of the following classes of fluorescent dyes: Xanthens, Acridines, Oxazines, Cynines, Styryl dyes, Coumarines, Porphines, Metal-Ligand-Complexes, Fluorescent proteins, Nanocrystals, Perylenes, Boron-dipyrromethenes and Phtalocyanines as well as conjugates and combinations of these classes of dyes.
11. The compound of any of items 1 to 8, wherein R⁸ is a chelating agent which forms a complex with divalent or trivalent metal cations, preferably wherein the chelating agent is selected from the group consisting of 1,4,7,10-tetraazacyclododecane-N,N',N,N'-tetraacetic acid (DOTA), ethylenediaminetetraacetic acid (EDTA), 1,4,7-triazacyclononane-1,4,7-triacetic acid (NOTA), triethylenetetramine (TETA), iminodiacetic acid, diethylenetriamine-N,N,N',N',N"-pentaacetic acid (DTPA), bis-(carboxymethylimidazole)glycine and 6-Hydrazinopyridine-3-carboxylic acid (HYNIC).
12. The compound of any of items 1 to 8, wherein R⁸ is a contrast agent which comprises or consists of a paramagnetic agent, preferably, wherein the paramagnetic agent comprises or consists of paramagnetic nanoparticles.
13. Pharmaceutical composition comprising or consisting of at least one compound according to any of items 1 to 12; and, optionally, a pharmaceutically acceptable carrier and/or excipient.
14. The compound of any of items 1 to 12 or the pharmaceutical composition of item 13 for use in the diagnosis or treatment of a disease characterized by overexpression of fibroblast activation protein (FAP) in an animal or a human subject, preferably wherein the disease characterized by overexpression of fibroblast activation protein (FAP) is selected from the group consisting of cancer, chronic inflammation, atherosclerosis, fibrosis, tissue remodeling and keloid disorder, preferably wherein the cancer is selected from the group consisting of breast cancer, pancreatic cancer, small intestine cancer, colon cancer, rectal cancer, lung cancer, head and neck cancer, ovarian cancer, hepatocellular carcinoma, esophageal cancer, hypopharynx cancer, nasopharynx cancer, larynx cancer, myeloma cells, bladder cancer, cholangiocellular carcinoma, clear cell renal carcinoma, neuroendocrine tumor, oncogenic osteomalacia, sarcoma, CUP (carcinoma of unknown primary), thymus carcinoma, desmoid tumors, glioma, astrocytoma, cervix carcinoma and prostate cancer.
15. A kit comprising or consisting of the compound of any of items 1 to 12 or the pharmaceutical composition of item 13 and instructions for the diagnosis of a disease.

## Claims

1. A compound having the structure 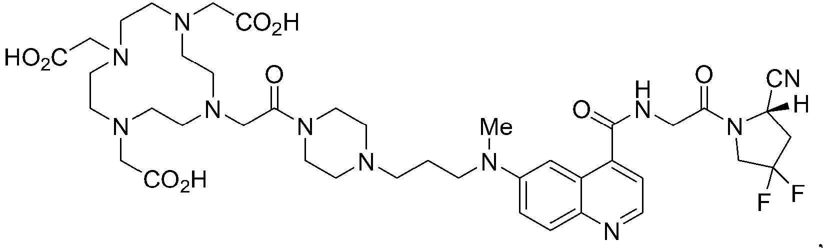 or a pharmaceutically acceptable salt thereof.

2. The compound of claim 1, which is labeled with Ga-68 or Y-90.

3. The compound of claim 1, which is labeled with Ga-68.

4. A pharmaceutical composition comprising or consisting of at least one compound according to any of claims 1 to 3; and, optionally, a pharmaceutically acceptable carrier and/or excipient.

5. The compound of any of claims 1 to 3 or the pharmaceutical composition of claim 4 for use in the diagnosis or treatment of a disease **characterized by** overexpression of fibroblast activation protein (FAP) in an animal or a human subject.

6. The compound or the pharmaceutical composition for use of claim 5, wherein the disease **characterized by** overexpression of fibroblast activation protein (FAP) is selected from the group consisting of cancer, chronic inflammation, atherosclerosis, fibrosis, tissue remodeling and keloid disorder.

7. The compound or the pharmaceutical composition for use of claim 6, wherein the cancer is selected from the group consisting of breast cancer, pancreatic cancer, small intestine cancer, colon cancer, rectal cancer, lung cancer, head and neck cancer, ovarian cancer, hepatocellular carcinoma, esophageal cancer, hypopharynx cancer, nasopharynx cancer, larynx cancer, myeloma cells, bladder cancer, cholangiocellular carcinoma, clear cell renal carcinoma, neuroendocrine tumor, oncogenic osteomalacia, sarcoma, CUP (carcinoma of unknown primary), thymus carcinoma, desmoid tumors, glioma, astrocytoma, cervix carcinoma and prostate cancer.

8. A kit comprising or consisting of the compound of any of claims 1 to 3 or the pharmaceutical composition of claim 4 and instructions for the diagnosis or treatment of a disease.

9. The kit of claim 8, wherein the disease is **characterized by** overexpression of fibroblast activation protein (FAP) in an animal or a human subject.

10. The kit of claim 9, wherein the disease **characterized by** overexpression of fibroblast activation protein (FAP) is selected from the group consisting of cancer, chronic inflammation, atherosclerosis, fibrosis, tissue remodeling and keloid disorder.

11. The kit of claim 10, wherein the cancer is selected from the group consisting of breast cancer, pancreatic cancer, small intestine cancer, colon cancer, rectal cancer, lung cancer, head and neck cancer, ovarian cancer, hepatocellular carcinoma, esophageal cancer, hypopharynx cancer, nasopharynx cancer, larynx cancer, myeloma cells, bladder cancer, cholangiocellular carcinoma, clear cell renal carcinoma, neuroendocrine tumor, oncogenic osteomalacia, sarcoma, CUP (carcinoma of unknown primary), thymus carcinoma, desmoid tumors, glioma, astrocytoma, cervix carcinoma and prostate cancer.
